(19) Europäisches Patentamt
European Patent Office
Office européen des brevets

(11) EP 1 491 190 A1

(12) EUROPEAN PATENT APPLICATION
published in accordance with Art. 158(3) EPC

(43) Date of publication:
29.12.2004 Bulletin 2004/53

(21) Application number: 03712957.4

(22) Date of filing: 26.03.2003

(51) Int Cl.[7]: **A61K 31/198**, A61K 31/381, A61K 31/4025, A61K 31/405, A61K 31/421, A61K 31/4245, A61K 31/426, A61K 31/427, A61P 19/02, A61P 29/00, A61P 43/00 // (C07D209/20, 263:32, 271:06, 277:26, 333:24, 333:34, 409:12, 413:04, 417:04)

(86) International application number:
PCT/JP2003/003673

(87) International publication number:
WO 2003/080042 (02.10.2003 Gazette 2003/40)

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: 27.03.2002 JP 2002087330

(71) Applicant: SHIONOGI & CO., LTD.
Osaka-shi, Osaka 541-0045 (JP)

(72) Inventors:
- GEMBA, Takefumi
  Kawanishi-shi, Hyogo 666-0014 (JP)
- OKAMOTO, Hiroyuki c/o Shionogi & Co., Ltd.
  Osaka-shi, Osaka 553-0002 (JP)
- WATANABE, Fumihiko c/o Shionogi & Co., Ltd.
  Osaka-shi, Osaka 553-0002 (JP)

(74) Representative: VOSSIUS & PARTNER
Siebertstrasse 4
81675 München (DE)

# (54) DECOMPOSITION INHIBITOR FOR EXTRACELLULAR MATRIX OF CARTILAGE

(57) It is provided that a pharmaceutical composition inhibiting cartilage extracellular matrix degradation containing a compound of the formula (I):

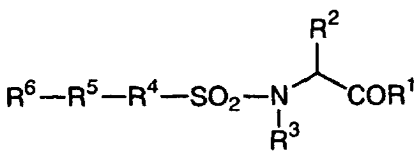

(I)

wherein $R^1$ is hydroxy and the like; $R^2$ is optionally substituted lower alkyl and the like; $R^3$ is hydrogen atom and the like; $R^4$ is optionally substituted arylene and the like; $R^5$ is a bond, or -C≡C- and the like; $R^6$ is optionally substituted aryl and the like,
its optically active substance, its prodrug, their pharmaceutically acceptable salt, or solvate thereof.

EP 1 491 190 A1

## Description

### Technical Field

The present invention relates to sulfonamide derivatives having an inhibitory activity against aggrecanase.

### Background Art

Progression of osteoarthrosis and rheumatoid arthritis relates to rupture of structure and function of cartilago articularis. An extracellular matrix produced by cartilage cell plays an important role in maintenance of structure and function of cartilago articularis. Main compositions of extracellular matrix that constitute cartilago articularis are type II collagen and proteoglycan, and aggrecan occupies about 90 % of proteoglycan in cartilage. Type II collagen is essential for cartilage structure and its tensile strength, and aggrecan is essential for maintenance of water and elasticity. Therefore, those degradation causes progression of osteoarthritis.

Especially, the loss of aggrecan contributes to the progression of osteoarthritis. In osteoarthritis and rheumatoid arthritis, aggrecan is one of the cartilage extracellular matrix components which disappear in early stage of the diseases (Non Patent Document 1). In human arthritis, aggrecan degradation is associated with amino acid cleavage at either the $Glu^{373}$-$Ala^{374}$ or the $Asn^{341}$-$Phe^{342}$ site. Recently, enzyme ADAMTS-4 that cleaves the $Glu^{373}$-$Ala^{374}$ site of aggrecan- has been identified (Non Patent Document 2).

On the other hand, type II collagen constitutes most of fibril of cartilage extracellular matrix. Those collagens consist of tightly coiled triple helix. MMP-1 (collagenase-1), MMP-8 (collagenase-2), and MMP-13 (collagenase-3) are known as matrix metalloproteinases that degrade type II collagen. Especially, MMP-13 (collagenase-3 (Non Patent Document 3)) in the three types is found almost exclusively in cartilage. This enzyme has been shown to significantly degrade type IT collagen and, in addition, increased amounts are present in human osteoarthritic cartilage (Non Patent Document 4).

Compounds having an inhibitory activity against matrix metalloproteinase-13 and an inhibitory activity against aggrecanase are described in Patent Document 1. Selective inhibitors of aggrecanase for treatment of osteoarthropathy are disclosed in Patent Document 2.

Sulfonamide derivatives having an inhibitory activity against MMP are disclosed in the following patent documents 3-14 and the like.

(Patent Document 1)
Japanese Patent Publication (Kokai) 2000-319250 official bulletin
(Patent Document 2)
Japanese Patent Publication (Kokai) 2001-114765 official bulletin
(Patent Document 3)
International Publication 97/27174 pamphlet
(Patent Document 4)
International Publication 99/04780 pamphlet
(Patent Document 5)
International Publication 00/63194 pamphlet
(Patent Document 6)
International Publication 00/15213 pamphlet
(Patent Document 7)
International Publication 01/83431 pamphlet
(Patent Document 8)
International Publication 01/83461 pamphlet
(Patent Document 9)
International Publication 01/83463 pamphlet
(Patent Document 10)
International Publication 01/83464 pamphlet
(Patent Document 11)
International Publication 00/46189 pamphlet
(Patent Document 12)
International Publication 00/58280 pamphlet
(Patent Document 13)
International Publication 02/28844 pamphlet
(Patent Document 14)

International Application PCT/JP02/11046

(Non Patent Document 1)

Mankin (H.J.Mankin) et. al., Journal of Bone and Joint Surgery (J.Bone Joint Surg.) 1970, Vol. 52A, p. 424-434

(Non Patent Document 2)

Tortorella (M. D. Tortorella) et. al., Science (Science) 1999, Vol. 284 p. 1664-1666

(Non Patent Document 3)

Freije (J. M. Freije) et. al., The Journal of Biological Chemistry (J. Biol. Chem.) 1994, Vol. 269, p.16766-16773

(Non Patent Document 4)

Mitchell (Peter G. Mitchell) et. al., The Journal of clinical investigation (J. Clin. Invest.) 1996, Vol. 96, p. 761-768

## Disclosure of Invention

Usually for treatment of osteoarthrosis and rheumatoid arthritis are used non-steroidal anti-inflammatory drugs (NSAIDs). However, NSAIDs, such as acetaminophen, act by inhibiting the synthesis of cytokines such as prostaglandins that cause pain and swelling. Thus, NSAIDs do not directly prevent cartilage destruction.

On the other hand, compounds having inhibitory activities against cartilage extracellular matrix degradation are considered to greatly contribute to improvement of disease (e.g., osteoarthritis and rheumatoid arthritis) caused by or associated with the activities or to prevention of its progression. Thus, it is desired to develop cartilage extracellular matrix degradation inhibitors, especially aggrecanase inhibitors. However, what kind of cartilage extracellular matrix degradation enzyme should be inhibited is dependent on the stage of the diseases. For example, in osteoarthritis, contribution of aggrecanase is the main in early stage, but contribution of MMP-13 increases in progression of the disease as well as aggrecanase. If it is the object to inhibit only the accelerating activities without affecting a recuperation of nomal cartilage, compounds inhibiting aggrecanase selectively are desirable for treatment in the early stage, but compounds having inhibitory activities against not only aggrecanase but also MMP-13 are desirable for treatment in the late stage. Otherwise, preferable are compounds inhibiting only MMP-13 depending on the stage of diseases.

In the above situation, the inventors of the present invention have found that certain sulfonamide derivatives have an inhibitory activity against aggrecanase.

That is, the present invention relates to:

1) A pharmaceutical composition inhibiting aggrecanase which contains as an active ingredient a compound of the formula (I):

$$R^6—R^5—R^4—SO_2—W \quad (I)$$

wherein W group is represented by the formula:

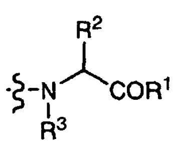 , 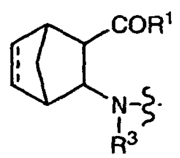 , 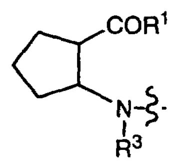 , 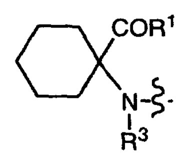 ,

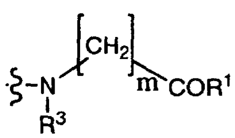 , 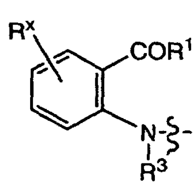 , or 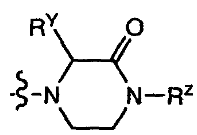

wherein $R^1$ is NHOH, hydroxy, or lower alkyloxy;

$R^2$ is hydrogen atom, optionally substituted lower alkyl, optionally substituted aryl, optionally substituted aralkyl, optionally substituted heteroaryl, or optionally substituted heteroarylalkyl;
$R^3$ is hydrogen atom, optionally substituted lower alkyl, optionally substituted aryl, optionally substituted aralkyl, optionally substituted heteroaryl, or optionally substituted heteroarylalkyl;
$R^x$ is hydrogen atom, lower alkyl, or halogen;
$R^Y$ is hydrogen atom or lower alkyl;
$R^Z$ is hydrogen atom or lower alkyl;
m is an integer from 2 to 6;
a broken line (---) represents the presence or absence of a bond;
$R^4$ is optionally substituted arylene, or optionally substituted heteroarylene;
$R^5$ is a bond, $-(CH_2)_p-$, -CH=CH-, -C≡C-, -CO-, -CO-NH-, -N=N-, $-N(R^A)-$, -NH-CO-NH-, -NH-CO-, -S-, $-SO_2-$, $-SO_2NH-$, $-SO_2-NH-N=CH-$ or a group represented by the formula:

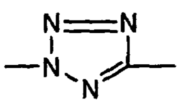 , 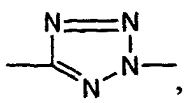 , 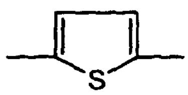 , 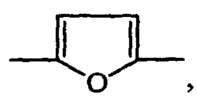 , 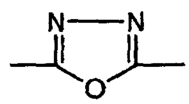

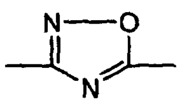 , 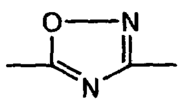 , 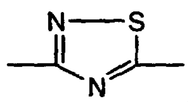 , 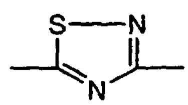 , 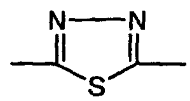

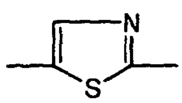 , 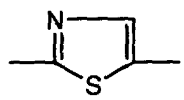 , 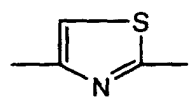 , 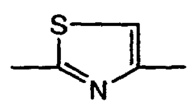

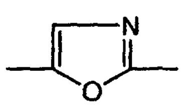 , 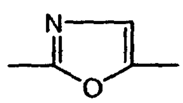 , 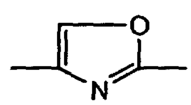 , 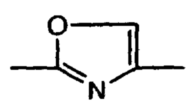 ,

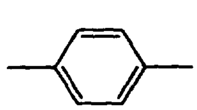 , or 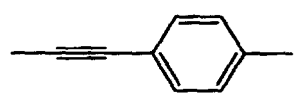

$R^A$ is hydrogen atom or lower alkyl;
p is 1 or 2;
$R^6$ is optionally substituted lower alkyl, optionally substituted cycloalkyl, optionally substituted aryl, optionally substituted aralkyl, optionally substituted heteroaryl, optionally substituted heteroarylalkyl, optionally substituted non-aromatic heterocyclic group; or optionally substituted cycloalkenyl,
its optically active substance, its prodrug, their pharmaceutically acceptable salt, or solvate thereof.

2) A pharmaceutical composition inhibiting aggrecanase which contains as an active ingredient a compound as described in 1), wherein W group is represented by the formula:

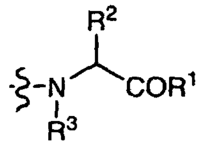 , 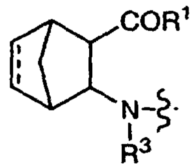 , 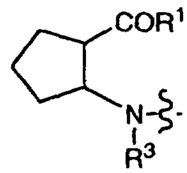 , or 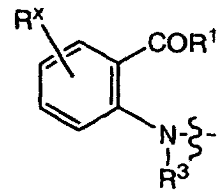

wherein $R^1$, $R^2$, $R^3$ and $R^X$ are as defined in 1) ;
its optically active substance, its prodrug, their pharmaceutically acceptable salt, or solvate thereof.
3) A pharmaceutical composition inhibiting aggrecanase which contains as an active ingredient a compound as described in 2), wherein W group is represented by the formula:

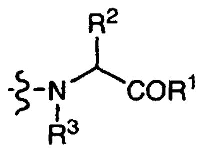

wherein $R^1$, $R^2$, and $R^3$ are as defined in 1) ;
its optically active substance, its prodrug, their pharmaceutically acceptable salt, or solvate thereof.
4) A pharmaceutical composition inhibiting aggrecanase which contains as an active ingredient a compound, as described in 3), wherein $R^2$ is hydrogen atom, optionally substituted lower alkyl wherein the substituent is hydroxy, carboxy, carbamoyl, or lower alkylthio, aryl optionally substituted with hydroxy, optionally substituted aralkyl wherein the substituent is hydroxy or phenyl, heteroaryl, or optionally substituted heteroarylalkyl wherein the substituent is hydroxy or halogen,
its optically active substance, its prodrug, their pharmaceutically acceptable salt, or solvate thereof.
5) A pharmaceutical composition inhibiting aggrecanase which contains as an active ingredient a compound as described in 4), wherein $R^2$ is hydrogen atom, methyl, isopropyl, isobutyl, sec-butyl, carbamoylmethyl, carbamoylethyl, 2-methylthioethyl, hydroxymethyl, carboxymethyl, carboxyethyl, phenyl, 4-hydroxyphenyl, benzyl, 4-hydroxybenzyl, 4-biphenylmethyl, thienyl, indol-3-ylmethyl, (4-hydroxy-indol-3-yl)methyl, (5-hydroxy-indol-3-yl)methyl, (6-hydroxy-indol-3-yl)methyl, (7-hydroxy-indol-3-yl)methyl or (5-fluoro-indol-3-yl)methyl,
its optically active substance, its prodrug, their pharmaceutically acceptable salt, or solvate thereof.
6) A pharmaceutical composition inhibiting aggrecanase which contains as an active ingredient a compound, its optically active substance, its prodrug, their pharmaceutically acceptable salt, or solvate thereof as described in any one of 1) to 5), wherein $R^1$ is hydroxy.
7) A pharmaceutical composition inhibiting aggrecanase which contains as an active ingredient a compound, its optically active substance, its prodrug, their pharmaceutically acceptable salt, or solvate thereof as described in any one of 1) to 6), wherein $R^3$ is hydrogen atom.
8) A pharmaceutical composition inhibiting aggrecanase which contains as an active ingredient a compound, its optically active substance, its prodrug, their pharmaceutically acceptable salt, or solvate thereof as described in any one of 1) to 7), wherein $R^4$ is 1,4-phenylene or 2,5-thiophen-diyl.
9) A pharmaceutical composition inhibiting aggrecanase which contains as an active ingredient a compound, its optically active substance, its prodrug, their pharmaceutically acceptable salt, or solvate thereof as described in any one of 1) to 8), wherein $R^5$ is a bond, -C≡C-, or a group represented by the formula:

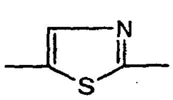 , 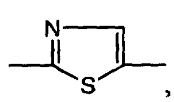 , 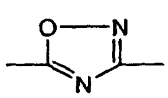 , 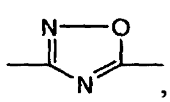 , 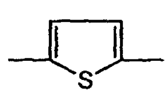

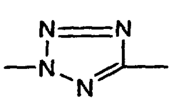 , 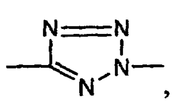 , 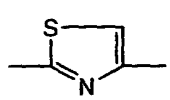 , 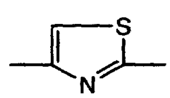 , or 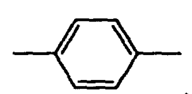 .

10) A pharmaceutical composition inhibiting aggrecanase which contains as an active ingredient a compound as described in 2), wherein W is a group represented by the formula:

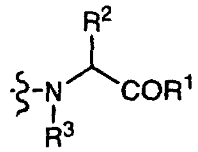 , 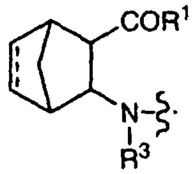 , 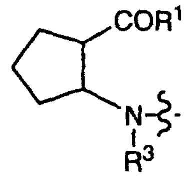 , or 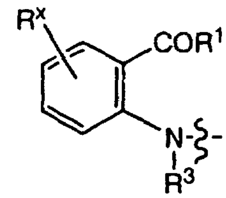

wherein $R^1$ is hydroxy;

$R^2$ is hydrogen atom, methyl, isopropyl, isobutyl, sec-butyl, carbamoylmethyl, carbamoylethyl, 2-methylthioethyl, hydroxymethyl, carboxymethyl, carboxyethyl, phenyl, 4-hydroxyphenyl, benzyl, 4-hydroxybenzyl, 4-biphenylmethyl, thienyl, indol-3-ylmethyl, (4-hydroxy-indol-3-yl)methyl, (5-hydroxy-indol-3-yl)methyl, (6-hydroxy-indol-3-yl)methyl, (7-hydroxy-indol-3-yl)methyl, or (5-fluoro-indol-3-yl)methyl;
$R^3$ is hydrogen atom;
$R^x$ is hydrogen atom, lower alkyl, or halogen;
a broken line (---) represents the presence or absence of a bond;
$R^4$ is 1,4-phenylene or 2,5-thiophen-diyl;
$R^5$ is a bond, -C≡C-, or a group represented by the formula:

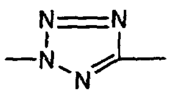 , 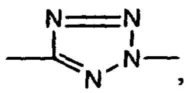 , 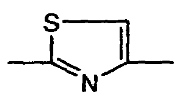 , 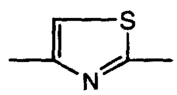 , or 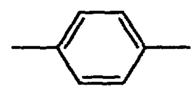

$R^6$ is optionally substituted phenyl, naphtyl, isoxazole, pyrrole, pyrrolidine or cyclohexenyl,
its optically active substance, its prodrug, their pharmaceutically acceptable salt, or solvate thereof.

11) A pharmaceutical composition inhibiting aggrecanase which contains as an active ingredient a compound as described in 10),
wherein $R^4$ is 2,5-thiophen-diyl;

$R^5$ is a bond or -C≡C-;
$R^6$ is optionally substituted phenyl, naphtyl, or cyclohexenyl,
its optically active substance, its prodrug, their pharmaceutically acceptable salt, or solvate thereof.

12) A pharmaceutical composition inhibiting aggrecanase which contains as an active ingredient a compound as described in 3),

$R^1$ is hydroxy;
$R^2$ is hydrogen atom, methyl, isopropyl, isobutyl, sec-butyl, carbamoylmethyl, carbamoylethyl, 2-methylthioethyl, hydroxymethyl, carboxymethyl, carboxyethyl, phenyl, 4-hydroxyphenyl, benzyl, 4-hydroxybenzyl, 4-biphenylmethyl, thienyl, indol-3-ylmethyl, (4-hydroxy-indol-3-yl)methyl, (5-hydroxy-indol-3-yl)methyl, (6-hydroxy-indol-3-yl)methyl, (7-hydroxy-indol-3-yl)methyl, or (5-fluoro-indol-3-yl)methyl;
$R^3$ is hydrogen atom;
$R^4$ is 1,4-phenylene or 2,5-thiophen-diyl;
$R^5$ is a bond, -C≡C-, or a group represented by the formula:

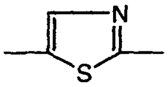 , 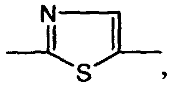 , 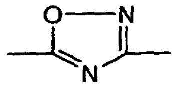 , 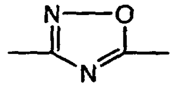 , or 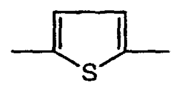

$R^6$ is optionally substituted phenyl, optionally substituted naphtyl, or optionally substituted heteroaryl, its optically active substance, its prodrug, their pharmaceutically acceptable salt, or solvate thereof.

13) A pharmaceutical composition inhibiting aggrecanase which contains as an active ingredient a compound as described in 12), wherein

$R^5$ is -C≡C-, or a group represented by the formula:

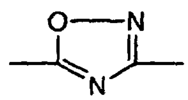

or

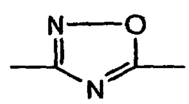

$R^6$ is naphtyl or optionally substituted phenyl, its optically active substance, its prodrug, their pharmaceutically acceptable salt, or solvate thereof.

14) A pharmaceutical composition inhibiting aggrecanase as described in any one of 1) to 13), wherein the composition has an activity of inhibiting matrix metalloproteinase-13.
15) A composition for treating or preventing osteoarthritis as described in any one of 1) to 14).
16) A composition for treating or preventing rheumatoid arthritis as described in any one of 1) to 14).
17) Use of a compound as described in any one of 1) to 13) for the preparation of a composition for treating osteoarthrosis.
18) Use of a compound as described in any one of 1) to 13) for the preparation of a composition for treating rheumatoid arthritis.
19) A method of treatment of a mammal, including a human, to alleviate the pathological effects of osteoarthrosis, which comprises administering to said mammal a therapeutically effective amount of a compound as described in any one of 1) to 13).
20) A method of treatment of a mammal, including a human, to alleviate the pathological effects of rheumatoid arthritis, which comprises administering to said mammal a therapeutically effective amount of a compound as described in any one of 1) to 13).

In the present specification, the term "lower alkyl" employed alone or in combination with other terms means a straight- or branched chain monovalent hydrocarbon group having 1 to 8 carbon atom(s). Examples of the alkyl include methyl, ethyl, n-propyl, isopropyl, n-butyl, isobutyl, sec-butyl, tert-butyl, n-pentyl, isopentyl, neo-pentyl, n-hexyl, isohexyl, n-heptyl, n-octyl and the like. C1 to C6 alkyl is preferred. C1 to C3 alkyl is more preferred.
The term "cycloalkyl" used in the present specification includes cycloalkyl group having 3 to 8 carbon atoms. Examples of cycloalkyl group include cyclopropyl, cyclobutyl, cyclopentyl, cyclohexyl, cycloheptyl, cyclooctyl and the like. C3 to C6 cycloalkyl is preferred.
The term "cycloalkenyl" used in the present specification includes cycloalkenyl group having 3 to 8 carbon atoms and at least one double bond. Examples of cycloalkenyl group include cyclopropenyl, cyclobutenyl, cyclopentenyl, cyclopentadienyl, cyclohexenyl, cyclohexadienyl, cycloheptenyl, cyclooctenyl and the like. C5 to C6 cycloalkenyl is preferred.
Examples as "cycloalkenyl" for $R^6$ include cyclohexenyl.
In the present specification, the term "aryl" employed alone or in combination with other terms includes monocyclic or condensed ring aromatic hydrocarbons. Examples include phenyl, 1-naphtyl, 2-naphtyl, anthryl, and the like.
Examples as "aryl" for $R^2$ include phenyl, and the like.
Examples as "aryl" for $R^3$ include phenyl, and the like.
Examples as "aryl" for $R^6$ include phenyl, 1-naphtyl, 2-naphtyl, and the like.
In the present specification, the term "naphtyl" includes 1-naphtyl and 2-naphtyl.
The term "aralkyl" herein used means the above-mentioned "lower alkyl" substituted with one or more above-mentioned "aryl" at any possible position. Examples of the aralkyl are benzyl, phenylethyl (e.g., 2-phenethyl and the like), phenylpropyl (e.g., 3-phenylpropyl and the like), naphthylmethyl (e.g., 1-naphthylmethyl and 2-naphthylmethyl and the like), anthrylmethyl (e.g., 9-anthrylmethyl and the like), biphenylmethyl (e.g., 2-biphenylmethyl, 3- biphenylmethyl, 4- biphenylmethyl, and the like) and the like.
Examples as "aralkyl" for $R^2$, $R^3$, and $R^6$ include benzyl, phenylethyl, 4- biphenylmethyl, and the like.
In the present specification, the term "heteroaryl" employed alone or in combination with other terms includes a 5 to 6 membered aromatic heterocyclic group which contains one or more hetero atoms selected from the group

consisting of oxygen, sulfur, and nitrogen atoms in the ring and may be fused with cycloalkyl, aryl, non-aromatic heterocyclic group, and other heteroaryl at any possible position. Examples of the heteroaryl are pyrrolyl (e.g., 1-pyrrolyl, 2-pyrrolyl, 3-pyrrolyl), furyl (e.g., 2-furyl, 3-furyl), thienyl (e.g., 2-thienyl 3-thienyl), imidazolyl (e.g., 2- imidazolyl, 4-imidazolyl), pyrazolyl (e.g., 1-pyrazolyl, 3-pyrazolyl), isothiazolyl (e.g., 3-isothiazolyl), isoxazolyl (e.g., 3-isoxazolyl), oxazolyl (e.g., 2-oxazolyl), thiazolyl (e.g., 2-thiazolyl), pyridyl (e.g., 2-pyridyl, 3-pyridyl, 4-pyridyl), pyrazinyl (e.g., 2-pyrazinyl), pyrimidinyl (e.g., 2-pyrimidinyl, 4-pyrimidinyl), pyridazinyl (e.g., 3-pyridazinyl), tetrazolyl (e.g., 1H-tetrazolyl), oxadiazolyl (e.g., 1,3,4-oxadiazolyl), thiadiazolyl (e.g., 1,3,4- thiadiazolyl), indolizinyl (e.g., 2-indolizinyl, 6-indolizinyl), isoindolyl (2-isoindolyl), indolyl (e.g., 1-indolyl, 2-indolyl, 3-indolyl), indazolyl (e.g., 3-indazolyl), puriyl (e.g., 8-puriyl), quinolizinyl ( e.g., 2-quinolizinyl), isoquinolyl (e.g., 3-isoquinolyl), quinolyl (e.g., 2-quinolyl, 5-quinolyl), phthalazinyl (e.g., 1-phthalazinyl), naphthyridinyl (e.g., 2-naphthyridinyl), quinoxanyl (2-quinoxanyl), quinazolinyl (e.g., 2-quinazolinyl), cinnolinyl (e.g., 3-cinnolinyl), pteridinyl (e.g., 2- pteridinyl), carbazolyl (e.g., 2-carbazolyl, 3-carbazolyl), phenanthridinyl (e.g., 2-phenanthridinyl, 3-phenanthridinyl), acridinyl (e.g., 1-acridinyl, 2-acridinyl), dibenzofuranyl (e.g., 1-dibenzofuranyl, 2-dibenzofuranyl), benzimidazolyl (e.g., 2-benzimidazolyl), benzisoxazolyl (e.g., 3-benzisoxazolyl), benzoxazolyl (e.g., 2-benzoxazolyl), benzoxadiazolyl (e.g., 4- benzoxadiazolyl), benzisothiazolyl (e.g., 3-benzisothiazolyl), benzothiazolyl (e.g., 2-benzothiazolyl), benzofuryl (e.g., 3-benzofuryl), benzothienyl (e.g., 2-benzothienyl), benzotriazolyl (e.g., 1-benzotriazolyl), and the like.

Examples as "heteroaryl" for $R^2$ include indolyl, imidazolyl, thienyl, and the like.

Examples as "heteroaryl" for $R^3$ include pyridyl, thienyl, furyl, indolyl, imidazolyl, and the like.

Examples as "heteroaryl" for $R^6$ include pyridyl, thienyl, furyl, indolyl, imidazolyl, benzothienyl, benzofuranyl, isoxazolyl, pyrrolyl, and the like.

The term "heteroarylalkyl" herein used includes the above-mentioned "lower alkyl" substituted with at least one above-mentioned "heteroaryl" at any possible position. Examples of the heteroarylalkyl are thiazolylmethyl (e.g., 4-thiazolylmethyl), thiazolylethyl (e.g., 5-thiazolyl-2-ethyl), benzothiazolylmethyl (e.g., (benzothiazol-2-yl)methyl), indolylmethyl (e.g., (indol-3-yl)methyl), imidazolylmethyl (e.g., 4-imidazolylmethyl), benzothiazolylmethyl (e.g., 2-benzothiazolylmethyl), indazolylmethyl (e.g., 1-indazolylmethyl), benzotriazolylmethyl (e.g., 1-benzotriazolylmethyl), benzoquinolylmethyl (e.g., 2-benzoquinolylmethyl), benzimidazolylmethyl (e.g., 2-benzimidazolylmethyl), pyridylmethyl (e.g., 2-pyridylmethyl, 3-pyridylmethyl, 4-pyridylmethyl), and the like.

Examples as "heteroarylalkyl" for $R^2$ include indolylmethyl (e.g., indol-3-ylmethyl, (4-hydroxy-indol-3-yl)methyl, (5-hydroxy-indol-3-yl)methyl, (6-hydroxy-indol-3-yl)methyl, (7-hydroxy-indol-3-yl)methyl, (5-fluoro-indol-3-ylmethyl,) and imidazolylmethyl (imidazol-5-ylmethyl) and the like.

Examples as "heteroarylalkyl" for $R^3$ include indolylmethyl (e.g., indol-3-ylmethyl), imidazolylmethyl (imidazol-5-ylmethyl), pyridylmethyl (e.g., 2-pyridylmethyl, 3-pyridylmethyl, 4-pyridylmethyl), and the like.

Examples as "heteroarylalkyl" for $R^6$ include indolylmethyl (e.g., indol-3-ylmethyl), imidazolylmethyl (imidazol-5-ylmethyl), pyridylmethyl (e.g., 2-pyridylmethyl, 3-pyridylmethyl, 4-pyridylmethyl), furylmethyl (e.g., 2-furylmethyl, 3-furylmethyl), thienylmethyl (e.g., 2-thienylmethyl, 3-thienylmethyl), and the like.

The term "non-aromatic heterocyclic group" includes a 5 to 7 membered non-aromatic ring which contains one or more hetero atoms selected from the group consisting of oxygen, sulfur, and nitrogen atoms in the ring and a condensed ring which are formed with two or more of the non-aromatic ring. Examples of the non-aromatic heterocyclic group are pyrrolidinyl (e.g., 1-pyrrolidinyl, 2-pyrrolidinyl), pyrrolinyl (e.g., 3-pyrrolinyl), imidazolidinyl (e.g., 2-imidazolidinyl), imidazolinyl (e.g., imidazolinyl), pyrazolidinyl (e.g., 1-pyrazolidinyl, 2-pyrazolidinyl), pyrazolinyl (e.g., pyrazolinyl), piperidyl (piperidino, 2-piperidyl), piperazinyl (e.g., 1-piperazinyl), indolynyl (e.g., 1-indolynyl), isoindolinyl (e.g., isoindolinyl), morpholinyl (e.g., morpholino, 3-morpholinyl), 4H-[1, 2, 4]oxaziazole-5-one, 1,2,3,4-tetrahydro-[1, 8] naphtylidine, 1,3-benzodioxolyl and the like.

Examples as "non-aromatic heterocyclic group" for $R^6$ include pyrrolidinyl, piperidyl, morpholinyl, imidazolinyl, and the like.

The term "arylene" herein used means a divalent group of the above-mentioned "aryl". Examples of the arylene are phenylene, naphthylene, and the like. Mentioned in more detail, it is exemplified by 1,2-phenylene, 1,3-phenylen, 1,4-phenylene, and the like. Preferable is 1,4-phenylene.

The term "heteroarylene" herein used means a divalent group of the above-mentioned "heteroaryl". Examples of the heteroarylene are thiophene-diyl, furan-diyl, pyridine-diyl, and the like. Preferable is 2,5-thionphene-diyl, 2,5-furan-diyl, and the like.

The term "halogen" herein used means fluoro, chloro, bromo, and iodo. Fluoro, chloro, and bromo are preferred.

The term "lower alkyloxy" herein used means methyloxy, ethyloxy, n-propyloxy, isopropyloxy, n-butyloxy, isobutyloxy, sec-butyloxy, tert-butyloxy, and the like. Methyloxy, ethyloxy, n-propyloxy, isopropyloxy and n-butyloxy are preferred.

The term "lower alkylthio" herein used means methylthio, ethylthio, and the like.

The substituents of "optionally substituted lower alkyl" means cycloalkyl, hydroxy, lower alkyloxy, mercapto,

lower alkylthio, halogen, nitro, cyano, carboxy, lower alkyloxycarbonyl, halo(lower)alkyl, halo(lower)alkyloxy, optionally substituted amino, optionally substituted aminocarbonyl (e.g.,carbamoyl), acyl, acyloxy, optionally substituted non-aromatic heterocyclic group, aryloxy (e.g., phenyloxy), aralkyloxy (e.g., benzyloxy), lower alkylsulfonyl, lower alkylsulfonyloxy, guanidino, azo group, optionally substituted ureide and the like. These substituents are able to locate at one or more of any possible positions.

The term "lower alkyloxycarbonyl" herein used means methyloxy-carbonyl, ethyloxycarbonyl, n-propyloxycarbonyl, isopropyloxycarbonyl, and the like.

In the present specification, the term "halo(lower)alkyl" employed alone or in combination with other terms includes the above-mentioned "lower alkyl" which is substituted with the above mentioned "halogen" at 1 to 8 positions, preferably, at 1 to 5. Examples of the halo(lower)alkyl are trifluoromethyl, trichloromethyl, difluoroethyl, trifluoroethyl, dichloroethyl, trichloroethyl, and the like. Preferable is trifluoromethyl.

Examples of the term "halo(lower)alkyloxy" herein used are trifluoromethyloxy and the like.

In the present specification, the term "optionally substituted amino" includes unsubstituted amino or amino substituted with one or two of the above mentioned "lower alkyl", "aralkyl", "heteroarylalkyl" or "acyl" mentioned later. Examples of the optionally substituted amino are amino, methylamino, dimethylamino, ethylmethylamino, diethylamino, benzylamino, acetylamino, benzoylamino and the like. Preferable are amino, methylamino, dimethylamino, ethylmethylamino, diethylamino and acetylamino.

Examples of the term "optionally substituted aminocarbonyl" herein used are aminocarbonyl (carbamoyl), methylaminocarbonyl, dimethylaminocarbonyl, ethylmethylaminocarbonyl, diethylaminocarbonyl, and the like. Preferable is aminocarbonyl, diethylaminocarbonyl.

In the present specification, the term "acyl" employed alone or in combination with other terms includes alkylcarbonyl in which alkyl group is the above-mentioned "lower alkyl" and arylcarbonyl in which aryl group is the above-mentioned "aryl". Examples of the acyl are acetyl, propionyl, benzoyl, and the like. "Lower alkyl" and "aryl" may be substituted respectively with substituents mentioned below.

Examples of the term "acyloxy" herein used means acetyloxy, propionyloxy, benzoyloxy and the like.

Examples of the term "lower alkylsulfonyl" herein used are methylsulfonyl, ethylsulfonyl, propylsulfonyl and the like.

Examples of the term "lower alkylsulfonyloxy" herein used are methanesulfonyloxy, ethanesulfonyloxy, propanesulfonyl, and the like.

Preferable are hydroxy, lower alkyloxy, lower alkylthio, carboxy, or carbamoyl as substituents of "optionally substituted lower alkyl" for $R^2$.

Preferable are hydroxy, lower alkyloxy, optionally substituted non-aromatic heterocyclic group as substituents of "optionally substituted lower alkyl" for $R^3$.

Preferable are hydroxy, lower alkyloxy, lower alkylthio, carboxy, carbamoyl, optionally substituted non-aromatic heterocyclic group as substituents of "optionally substituted lower alkyl" for $R^6$.

The substituents of "optionally substituted arylene", "optionally substituted heteroarylene", "optionally substituted cycloalkyl", "optionally substituted cycloalkenyl", "optionally substituted aryl", "optionally substituted heteroaryl", "optionally substituted non-aromatic heterocyclic group", "optionally substituted aralkyl", "optionally substituted heteroarylalkyl", and "optionally substituted ureide" means optionally substituted lower alkyl, cycloalkyl, lower alkenyl, lower alkynyl optionally substituted with aryl, hydroxy, lower alkyloxy, mercapto, lower alkylthio, halogen, nitro, cyano, carboxy, lower alkyloxycarbonyl, halo(lower)alkyl, halo(lower)alkyloxy, optionally substituted amino, optionally substituted aminocarbonyl, acyl, acyloxy, optionally substituted aryl, optionally substituted heteroaryl, optionally substituted non-aromatic heterocyclic group, optionally substituted aralkyl, lower alkylsulfonyl, guanidino group, azo group, or optionally substituted ureide, and the like. These substituents are able to locate at one or more of any possible positions.

In the present specification, the term "lower alkenyl" employed alone or in combination with other terms means a straight- or branched chain monovalent hydrocarbon group having 2 to 8 carbon atoms and at least one double bond. Examples of the alkenyl include vinyl, allyl, propenyl, crotonyl, isopentenyl, a variety of butenyl isomers and the like. C2 to C6 alkenyl is preferred. C2 to C4 alkenyl is more preferred.

The term "lower alkynyl" used in the present specification means a straight- or branched chain monovalent hydrocarbon group having 2 to 8 carbon atoms and at least one triple bond. The alkynyl may contain (a) double bond (s). Examples of the alkynyl include ethynyl, 2-propynyl, 3-butynyl, 4-pentynyl, 5-hexynyl, 6-heptynyl, 7-octynyl and the like. C2 to C6 alkynyl is preferred. C2 to C4 alkynyl is more preferred.

Substituents of "optionally substituted arylene" and "optionally substituted heteroarylene" for $R^4$ herein used are halogen, nitro, cyano, lower alkyloxy, and the like. Preferable are unsubstituted "arylene" and unsubstituted "heteroarylene".

Substituents of "optionally substituted cycloalkyl" for $R^6$ are optionally substituted lower alkyl, lower alkenyl, lower alkynyl, hydroxy, lower alkyloxy, mercapto, lower alkylthio, halogen, nitro, cyano, carboxy, lower alkyloxycarbonyl, halo(lower)alkyl, halo(lower)alkyloxy, optionally substituted amino, optionally substituted aminocarbonyl, acyl, acyloxy,

and the like. And as another substituent is included $-O-CH_2-O-$, or -S-C(=O)-NH-, which joins to two positions of cycloalkyl to form a ring. Lower alkyl, lower alkyloxy, halogen, or lower alkylthio are preferred.

Substituents of "optionally substituted cycloalkenyl" for $R^6$ are optionally substituted lower alkyl, lower alkenyl, lower alkynyl, hydroxy, lower alkyloxy, mercapto, lower alkylthio, halogen, nitro, cyano, carboxy, lower alkyloxycarbonyl, halo(lower)alkyl, halo(lower)alkyloxy, optionally substituted amino, optionally substituted aminocarbonyl, acyl, acyloxy, and the like. Unsubstituted cycloalkenyl is preferred.

Preferable are optionally substituted lower alkyl, lower alkenyl, lower alkynyl optionally substituted with aryl, hydroxy, lower alkyloxy, mercapto, lower alkylthio, halogen, nitro, cyano, carboxy, lower alkyloxycarbonyl, halo(lower) alkyl, halo(lower)alkyloxy, optionally substituted amino, optionally substituted aminocarbonyl, acyl, acyloxy, heteroaryl, non-aromatic heterocyclic group, and the like as the substituents of "optionally substituted aryl" or "optionally substituted heteroaryl" for $R^2$, $R^3$, or $R^6$. As another substituent is included $-O-CH_2-O-$, or -S-C(=O)-NH-, which joins to two positions of aryl or heteroaryl to form a ring.

Substituents of "optionally substituted aryl" for $R^2$ are hydroxy, or halogen. Preferable is hydroxy.

Preferable are hydroxy, lower alkyloxy, halogen, or halo(lower)alkyl as substituents of "optionally substituted aryl" for $R^3$.

Substituents of "optionally substituted aryl" for $R^6$ are optionally substituted lower alkyl (the substituents are amino optionally substituted with lower alkyl or acyl, or lower alkylsulfonyloxy), lower alkyloxy, halogen, lower alkylthio, halo(lower)alkyl, halo(lower)alkyloxy, amino optionally substituted with lower alkyl, acetyl, vinyl, ethynyl optionally substituted with aryl, pyrrolidinyl, piperidinyl, morpholinyl, isoxazolyl, benzofuryl, benzothienyl, pyrrolyl, ring formation joining two positions of the ring with $-O-CH_2-O-$, or -S-C(=O)-NH- and the like. Preferable are lower alkyl, lower alkyloxy, halogen, lower alkylthio, or another preferable substituent is $-O-CH_2-O-$, or -S-C(=O)-NH-, which joins to two positions of aryl to form a ring.

Preferable are hydroxy, or halogen as substituents of "optionally substituted heteroaryl" for $R^2$.

Preferable are hydroxy, lower alkyloxy, halogen, or halo(lower)alkyl as substituents of "optionally substituted heteroaryl" for $R^3$.

Preferable are lower alkyl, lower alkyloxy, halogen, or lower alkylthio as substituents of "optionally substituted heteroaryl" for $R^6$. Another preferable substituent is $-O-CH_2-O-$, or -S-C(=O)-NH-, which joins to two positions of heteroaryl to form a ring.

Preferable are optionally substituted lower alkyl, lower alkenyl, lower alkynyl, hydroxy, lower alkyloxy, mercapto, lower alkylthio, halogen, nitro, cyano, carboxy, lower alkyloxycarbonyl, halo(lower)alkyl, halo(lower)alkyloxy, optionally substituted amino, optionally substituted aminocarbonyl, acyl, acyloxy, phenyl, and the like as the substituents of "optionally substituted aralkyl" or "optionally substituted heteroarylalkyl" for $R^2$, $R^3$, or $R^6$. Another included substituent is $-O-CH_2-O-$, or -S-C(=O)-NH-, which joins to two positions of aralkyl or heteroarylalkyl to form a ring.

Preferable are hydroxy, or phenyl as substituents of "optionally substituted aralkyl" for $R^2$.

Preferable are hydroxy, lower alkyloxy, halogen, or halo(lower)alkyl as substituents of "optionally substituted aralkyl" for $R^3$.

Preferable are lower alkyl, lower alkyloxy, halogen, or lower alkylthio as substituents of "optionally substituted aralkyl" for $R^6$. Another preferable substituent is $-O-CH_2-O-$, or -S-C(=O)-NH-, which joins to two positions of aralkyl to form a ring.

Preferable are halogen, or hydroxy as substituents of "optionally substituted heteroarylalkyl" for $R^2$.

Preferable are hydroxy, lower alkyloxy, halogen, or halo(lower)alkyl as substituents of "optionally substituted heteroarylalkyl" for $R^3$.

Preferable are lower alkyl, lower alkyloxy, halogen, or lower alkylthio as substituents of "optionally substituted heteroarylalkyl" for $R^6$. Another preferable substituent is $-O-CH_2-O-$, or -S-C(=O)-NH-, which joins to two positions of heteroarylalkyl to form a ring.

Substituents of "optionally substituted non-aromatic heterocyclic group" for $R^6$ are optionally substituted lower alkyl, lower alkenyl, lower alkynyl, hydroxy, lower alkyloxy, mercapto, lower alkylthio, halogen, nitro, cyano, carboxy, lower alkyloxycarbonyl, halo(lower)alkyl, halo(lower)alkyloxy, optionally substituted amino, optionally substituted aminocarbonyl, acyl, acyloxy, and the like. Non-substituent or lower alkyl, lower alkyloxy, halogen, or lower alkylthio are preferred.

The term "aggrecanase" used in the present specification means an enzyme, which cleaves a bond of $Glu^{373}$-$Ala^{374}$ of aggrecan. Compounds used in the present invention have an inhibitory activity against aggrecanase, a suppression activity against GAG release, and an inhibitory activity against cartilage extracellular matrix degradation. Therefore, they are useful for a composition for treatment or prevention of osteoarthrosis, rheumatoid arthritis, osteoarthropathy, joint injury, reactive arthritis, acute pyrophosphate arthritis, and psoriatic arthritis.

The term "osteoarthrosis" used in the present specification means arthritis characterized by sore of cartilago articularis. The disorder causes malacia, fraying, and thinning of cartilago articularis, which are accompanied with eburnation of subchondral bone and formation of marginal osteophyte, and produces ache and dysfunction.

The term "rheumatoid arthritis" used in the present specification means a systemic disease, including arthritis as main the clinical symptom. The arthritis causes hypertrophy in articular soft tissue of many joints, especially joints of hands and feet, where synovial tissues cover cartilago articularis to erodes cartilage. The disorder is more observed in women, and the progress is of variety, which is chronic and progressive deformation of joint and to be cripple in some cases.

## Brief Description of Drawings

Figure 1 shows western blot analysis of a culture supernatant using the ARGSV specific antibody. It means that a test compound (XXI) inhibits the production of aggrecanase-generated new N-terminus (ARGSV).
Figure 2 shows western blot analysis of a culture supernatant using the ARGSV specific antibody. It means that the test compounds inhibit the production of aggrecanase-generated new N-terminus (ARGSV).

## Best Mode for Carrying Out the Invention

Compounds used in the present invention are able to be synthesized in accordance with the procedure described in WO97/2717 (method A ~ method F) and in accordance with the procedure described in WO99/04780, WO00/63194, WO01/83431, WO01/83461, WO01/83463, WO01/83464, WO00/46189, WO00/58280, WO02/28844, PCT/JP02/11046 and the like.
The following is described in detail.
And it is possible to produce the compounds used in the present invention in the following manner (method A) ~ (method C)

(Method A)

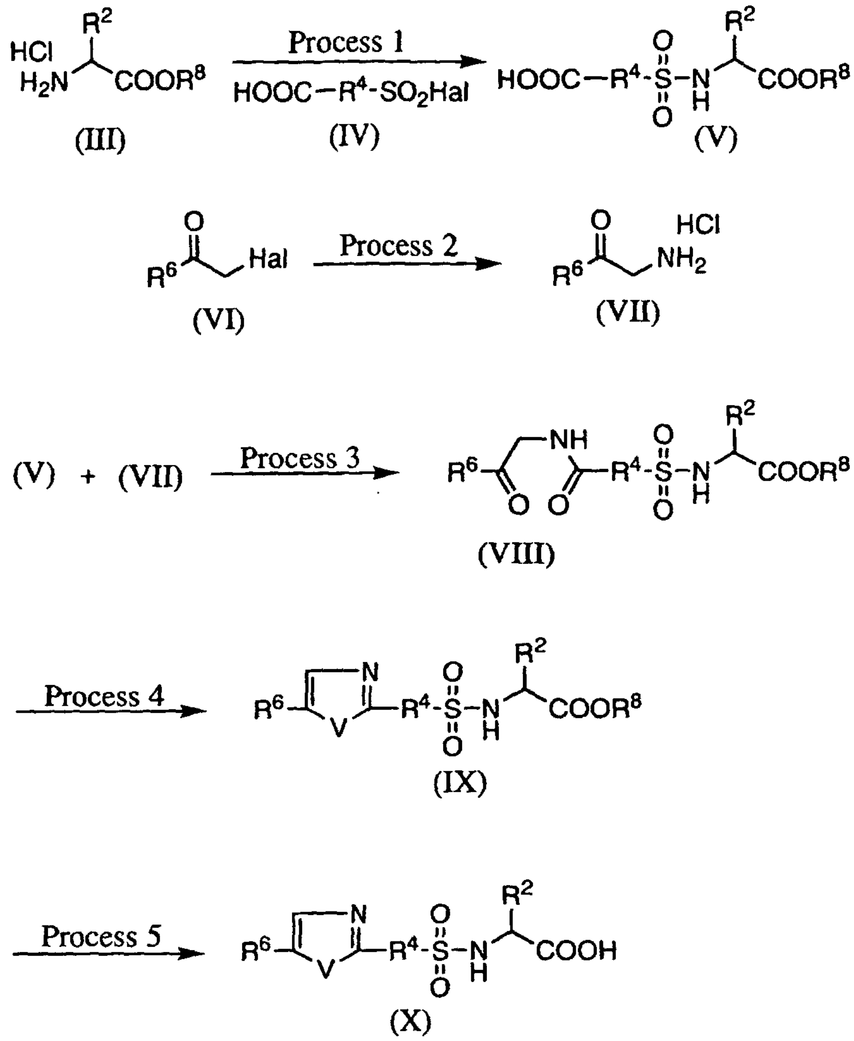


wherein $R^2$, $R^4$ and $R^6$ are as defined above, $R^8$ is a protecting group of carboxy (e.g. lower alkyl), Hal is independently halogen, V is oxygen atom or sulfur atom.

(Process 1)

This process can be accomplished in accordance with the process 1 in the method A described in WO97/27174.

(Process 2)

Compound (VI) is dissolved in a solvent such as chloroform, dichloromethane. An amine such as hexamethylenetetramine is added. The mixture is stirred at 0 °C to 60 °C, preferably 10 °C to 40 °C for 1 to 48 h, preferably 10 to 30 h. The obtained quaternary ammonium salt is suspended in a solvent such as methanol, ethanol. Concentrated hydrochloric acid is added thereto. The mixture is stirred at 0 °C to 60 °C, preferably 10 °C to 40 °C for 1 to 48 h, preferably 10 to 30 h to give a primary ammonium hydrochloride (VII).

(Process 3)

Compound (V) and dimethylformamide are suspended in a solvent such as dichloromethane. A halogenating agent such as oxalyl chloride is added thereto at -30 °C to 20 °C, preferably at -10 °C to 5 °C. The suspension is stirred for 10 min to 3 h, preferably for 30 min to 2 h to give the acid halide of compound (V). Compound (VII) and pyridine or N-methylmorpholine are suspended in a solvent such as dichloromethane. The above obtained solution containing the acid halide is added to the suspension at -30 °C to 30 °C, preferably at -10 °C to 10 °C. The mixture is stirred at 0 °C to 50 °C, preferably at 10 °C to 40 °C for 1 h to 24 h, preferably for 2 to 5 h to give compound (VIII).

(Process 4)

In a case of preparing a compound in which V is oxygen atom, compound (VIII) is suspended in phosphorus oxychloride. The mixture is stirred at 70 °C to 150 °C, preferably at 90 °C to 120 °C for 1 h to 5 h, preferably for 1 h to 2 h to give compound (IX).

In a case of preparing a compound in which V is sulfur atom, compound (VIII) is dissolved in a solvent such as tetrahydrofuran and the like. Lawesson's reagent is added. The mixture is stirred at 40 °C to 100 °C, preferably at 60 °C to 90 °C for 1 h to 5 h, preferably for 1 h to 3 h to give compound (IX).

(Process 5)

This process can be accomplished in accordance with the process 1 in the method A described in WO97/27174.

In accordance with the method described in WO97/27174, the obtained compound (X) can be converted to a compound, which has a hydroxamic acid group or is substituted with $R^3$ at the nitrogen atom.

(Method B)

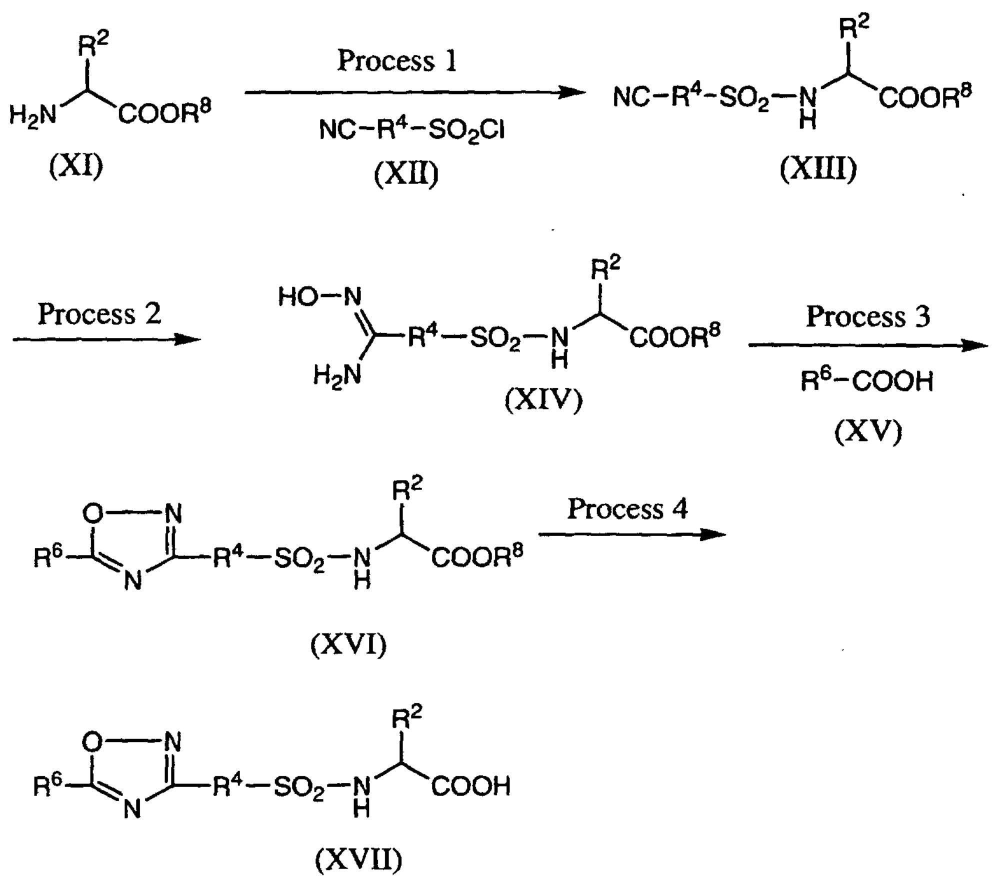

(XI)
(XII)
(XIII)
(XIV)
(XV)
(XVI)
(XVII)

wherein $R^2$, $R^4$, $R^6$, $R^8$ are as defined above.

(Process 1)

This is a process of obtaining sulfonamide derivatives (XIII) by the reaction of compound (XI) having an amino group and compound (XII) having a cyano group. The process may be carried out in accordance with the same procedure as (Method A-Process 1) in WO97/27174.

(Process 2)

The present process is a process of obtaining compound (XIV) by the reaction of compound (XIII) having cyano group and hydroxylamine. To a solution of compound (XIII) and a hydroxyl amine derivative such as hydroxylamine hydrochloride in ethanol and the like is added a base (e.g., triethylamine) and the mixture is reacted under 10°C to reflux to obtain the target compound (XIV).

(Process 3)

The present process is a process of constructing of an oxadiazole ring by the reaction of compound (XIV) and compound (XV) having a carboxy group. This process may be carried out in the same manner as that described in Gui-Bai Liang, Danqing D.Feng, Tetrahedron Letters, 37, 6627 (1996). Another method for the process of constructing the oxadiazole ring by acid chloride is reported (Cuy D. Diana, et al., J. Med. Chem., 1994, 37, 2421-2436).

(Process 4)

The present step is a process of yielding the compound (XVII) by hydrolysis of the compound (XVI). This step may be carried out in accordance with a usual method as described in "Protective Groups in Organic Synthesis, Theodora W Green (John Wiley & Sons)" and the like.

(Method C)

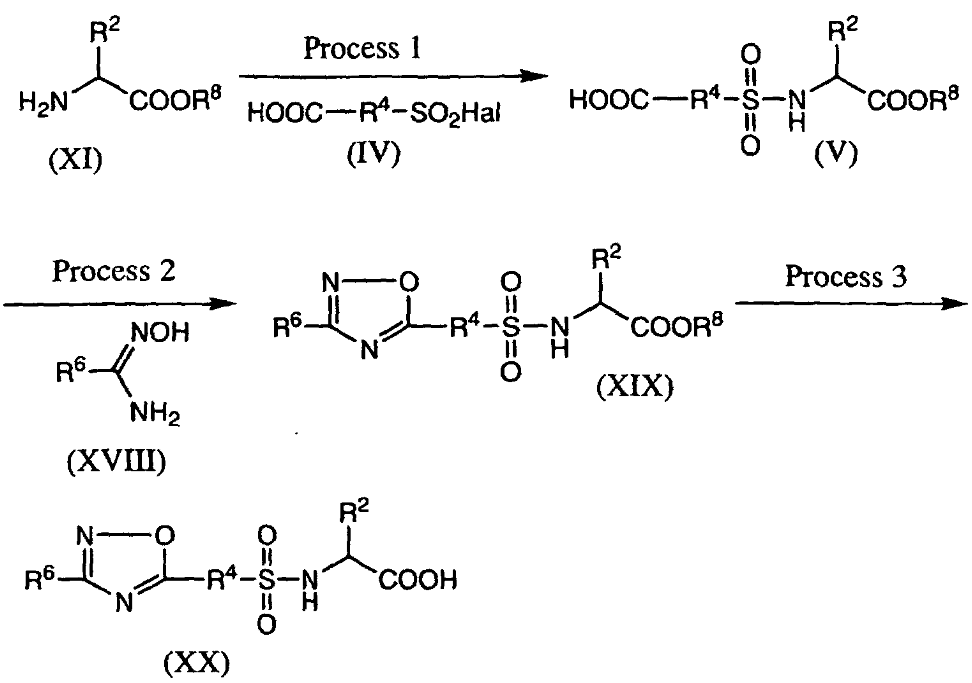


wherein $R^2$, $R^4$, $R^6$ and $R^8$ are as defined above, Hal is halogen.

(Process 1)

The process may be carried out in accordance with the same procedure as (Method A-Process 1) in WO97/27174.

(Process 2)

This process is a process of constructing an oxadiazole ring by the reaction of a compound (V) and a compound (XVIII).

A compound (V) is dissolved in diglyme and toluene, etc., and then to the reaction mixture are added oxalyl chloride and dimethylformamide at 0 °C to 30 °C, preferably 0 °C to 20 °C, and then the reaction mixture is stirred preferably for 60 to 120 min. To a solution of a compound (XVIII) and pyridine in diglyme and toluene is added the solution of acyl chloride prepared above under ice-cooling, and then the reaction mixture is stirred at 0 °C to 110 °C for 2 h to 18 h, preferably 2 h to 3 h. A compound (XIX) is obtained by a usual post-treatment.

(Process 3)

This process is a process of obtaining a compound (XX) by removing the protecting group of carboxyl of a compound (XIX). It may be carried out in accordance with a usual method as described in "Protective Groups in Organic Synthesis, Theodora W Green (John Wiley & Sons)" and the like.

The term "compound of the present invention" herein used is not restricted to any particular isomers but includes all possible isomers and racemic modifications. Furthermore, a pharmaceutically acceptable salt or its solvate is included.

Prodrug is a derivative of the compound having a group which can be decomposed chemically or metabolically, and such prodrug is a compound according to the present invention which becomes pharmaceutically active by means of solvolysis or by placing the compound in vivo under a physiological condition. The method of both selection and manufacture of appropriate prodrug derivatives is described in, for example. Design of Prodrugs, Elsevier, Amsterdam, 1985. For instance, prodrugs such as an ester derivative, optionally substituted alkyloxycarbonyl, which is prepared by reacting a basal acid compound with a suitable alcohol, or an amide derivative, optionally substituted alkylaminocarbonyl, which is prepared by reacting a basal acid compound with a suitable amine are exemplified when the compounds according to present invention have a carboxylic group (e.g., $R^1$ is OH). Particularly preferred esters as prodrugs are methyl ester, ethyl ester, n-propyl ester, isopropyl ester, n-butyl ester, isobutyl ester, tert-butyl ester, morpholinoethyl ester, and N,N-diethylglycolamido ester, and the like. For instance, prodrugs such as an acyloxy derivative which is prepared by reacting a basal hydroxy compound with a suitable acyl halide or a suitable acid anhydride are exemplified when the compounds according to present invention have a hydroxy group. Particularly preferred acyloxy derivatives as prodrugs $-OCOC_2H_5$, -OCO(t-Bu), $-OCOC_{15}H_{31}$, -OCO(m-COONa-Ph), $-OCOCH_2CH_2COONa$, $-OCOCH(NH_2)CH_3$, $-OCOCH_2N(CH_3)_2$, and the like. For instance, prodrugs such as an amide derivative which is prepared by reacting a basal amino compound with a suitable acid halide or a suitable acid anhydride are exemplified when the compounds according to present invention have an amino group. Particularly preferred amide as prodrugs are $-NHCO(CH_2)_{20}CH_3$, $-NHCOCH(NH_2)CH_3$, and the like.

The term "pharmaceutically acceptable salt " herein used includes a salt with an alkali metal (e.g., lithium, sodium, and potassium), an alkaline earth metal (e.g., magnesium and calcium), an ammonium, an organic base, an amino acid, a mineral acid (e.g., hydrochloric acid, hydrobromic acid, phosphoric acid, and sulfuric acid), or an organic acid (e.g., acetic acid, citric acid, maleic acid, fumaric acid, benzenesulfonic acid, and p-toluenesulfonic acid). These salts can be formed by usual methods.

The term "solvate" in the present invention herein used includes a solvate with an organic solvent(s), a hydrate and the like. These hydrates can coordinate with any water molecules.

The present invention compound shows an excellent inhibitory activity against aggrecanase as shown in test examples described later. Some compounds have not only an inhibitory activity against aggrecanase but also inhibitory activity against matrix metalloproteinase. Concretely, the present invention compounds can be used as a composition for treatment or prevention of osteoarthritis or rheumatoid arthritis.

Aggrecanase inhibition activity test is performed by detection of aggrecanase activity or aggrecanase-cleavable endogenous substrates in the presence or absence of a test compound.

Aggrecanase activity assay with an enzyme can be conducted by using a mammalian cartilage tissue or its culture as the enzyme source, in combination with various chromatographies. Aggrecanase activity can be detected by monitoring aggrecan cleavage-sites upon addition of aggrecan as substrate to an obtained enzyme. The detection of these sites can be performed by SDS-PAGE method or Immunoblotting using a substrate-specific antibodies, to be more exact, immunoblotting using antibodies against a neoepitope formed by cleavage of aggrecan by aggrecanase.

The detection of aggrecanase-cleavable endogenous substrates (aggrecan, brevican and so on) in the mammalian tissues or its culture supernatant can be performed by SDS-PAGE method or Immunoblotting using a substrate-specific antibodies, to be more exact, immunoblotting using antibodies against a neoepitope formed by cleavage of aggrecan by aggrecanase.

The enzymatic activity on MMP-13 can be analyzed by the method described in "C. Graham Knight, Frances Willenbrock and Gillian Murphy : A novel coumarin-labelled peptide for sensitive continuous assays of the matrix metalloproteinases : FEBS LETT., 296, (1992), 263-266."

When the compound of the present invention is administered to a person for the treatment of the above diseases, it can be administered orally as powder, granules, tablets, capsules, pilulae, and liquid medicines, or parenterally as injections, suppositories, percutaneous formulations, insufflation, or the like. An effective dose of the compound is formulated by being mixed with appropriate medicinal admixtures such as excipient, binder, penetrant, disintegrators, lubricant, and the like if necessary. Parenteral injections are prepared by sterilizing the compound together with an appropriate carrier. Oral agent, injections (intraarticular injection, intravenous injection) or transdermal patch are more preferable.

The dosage varies with the conditions of the patients, administration route, their age, and body weight. In the case of oral administration, the dosage can generally be between 0.1 to 100 mg/kg/day, and preferably 0.1 to 20 mg/kg/day for adult.

The following examples and test examples are provided to further illustrate the present invention and are not to be constructed as limiting the scope thereof.

Abbreviations described below are used in the following examples.

Me : methyl
Et : ethyl
iPr : isopropyl
nBu : n-butyl
iBu : isobutyl
sBu : sec-butyl
tBu : tert-butyl
Ph : phenyl
Bn : benzyl
Ac : acetyl
Ms : methanesulfonyl
4-Biphenylmethyl : 4 -Biphenylmethyl
4-Hydroxybenzene : 4-Hydroxybenzene
(Indol-3-yl)methyl : (Indol-3-yl)methyl
(4-Hydroxy-indol-3-yl)methyl : (4-Hydroxy-indol-3-yl)methyl
(6-Hydroxy-indol-3-yl)methyl : (6-Hydroxy-indol-3-yl)methyl
(7-Hydroxy-indol-3-yl)methyl : (7-Hydroxy-indol-3-yl)methyl
2-Thienyl : 2-Thienyl

Example 1 Preparation of compound C-1

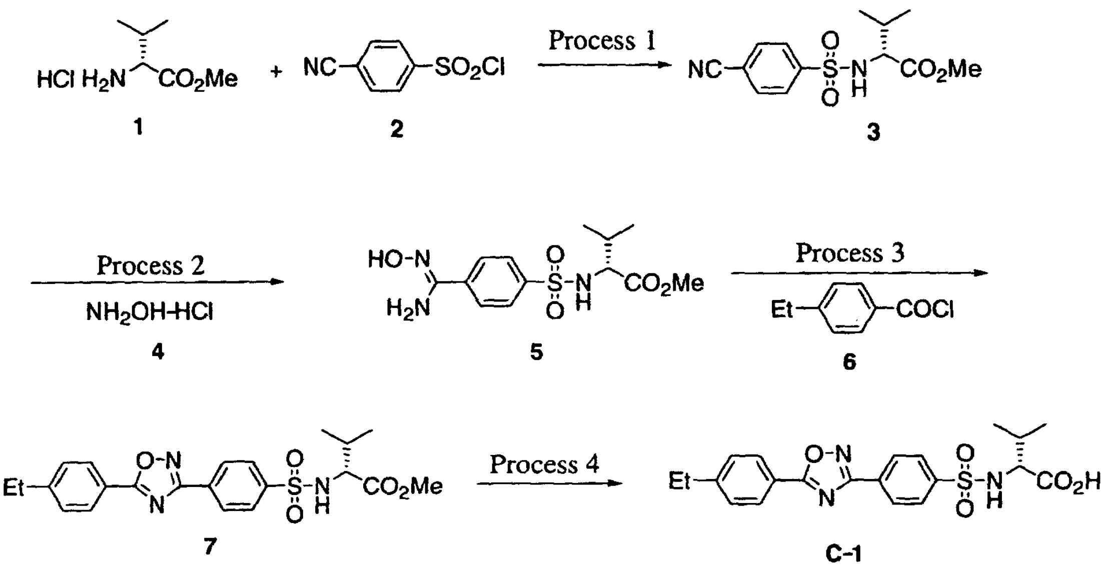


1 2 3 4 5 6 7 C-1

Process 1

To a suspension of D-valine methyl ester hydrochloric acid salt (1) (2.2 g, 13.1 mmol) and 4-cyanobenzenesulfonyl chloride (2) (2.4 g, 11.9 mmol) in tetrahydrofuran (40 mL) was added in ice *N*-methylmorpholine (3.9 mL, 35.5 mmol). The reaction mixture was stirred at room temperature for 1.5 hours, poured into ice-2 mol/L hydrochloric acid and was extracted with ethyl acetate. The organic layer was washed with saturated sodium hydrogen carbonate aqueous solution and water successively, dried over anhydrous sodium sulfate and concentrated under reduced pressure. The residue was recrystallized from ethyl ether / hexane to give a desired product (3) (3.12 g, yield 88.4%). Melting point 54-57 °C.
IR (KBr, ν max $cm^{-1}$) 3292, 2974, 2231, 1732, 1709, 1469, 1446, 1348, 1173, 833 $^1H$ NMR ($CDCl_3$, δ ppm): 0.87 (d, J = 6.8 Hz, 3H), 0.97 (d, J = 6.8 Hz, 3H), 2.09 (m, 1H), 3.51 (s, 3H), 3.80 (dd, J = 5.0, 10.2 Hz, 1H), 5.25 (d, J = 10.2 Hz, 1H), 7.80 (d, J= 8.4 Hz, 2H), 7.96 (d, J=8.8 Hz, 2H)
$[\alpha]_D$ + 4.7 ± 0.9° (*c* = 0.506, DMSO, 23 °C)
Elemental analysis ($C_{13}H_{16}N_2O_4S$)
Calcd.: C;52.69, H;5.44, N;9.45, S;10.82
Found: C;52.80, H;5.34, N;9.52, S;10.55

Process 2

To a suspension of compound (3) (3.24 g, 10.9 mmol) and hydroxylamine hydrochloride (0.91 g, 13.1 mmol) in ethanol (100 mL) was added at room temperature triethylamine (1.83 mL, 13.1 mmol) and the reaction mixture was refluxed for 1.5 hours. Ethanol was evaporated under reduced pressure and to the residue was added water (50 mL) and the mixture was extracted with ethyl acetate (50 mL × 3). The organic layer was washed with water (50 mL × 1), dried over anhydrous sodium sulfate and concentrated under reduced pressure. The residue was recrystallized from ethyl ether /n-hexane to give a desired product (5) (3.4 g, yield 94.4%). Melting point 130-131 °C.
IR (KBr, ν max $cm^{-1}$) 3485, 2960, 1722, 1651, 1335, 1165, 1090, 606
$^1H$ NMR ($CDCl_3$, δ ppm): 0.87 (d, J = 7.0 Hz, 3H), 0.95 (d, J = 7.0 Hz, 3H), 2.04 (m, 1H), 3.46 (s, 3H), 3.77 (dd, J = 5.0, 10.2 Hz, 1H), 5.04 (br s, 2H), 5.44 (d, J = 10.2 Hz, 1H), 7.76 (d, J= 8.8 Hz, 2H), 7.85 (d, J= 8.2 Hz, 2H)
$[\alpha]_D$ + 8.8 ± 1.0° (*c* = 0.503, DMSO, 23 °C)
Elemental analysis ($C_{13}H_{19}N_3O_5S$)
Calcd.: C;47.40, H;5.81, N;12.76, S;9.73
Found: C;47.60, H;5.76, N;12.47, S;9.76

Process 3

To a solution of a compound (5) (5.0 g, 15.2 mmol) in ethylene glycol dimethyl ether (100mL) were added in ice pyridine (3.7mL, 45.7mmol) and 4-ethylbenzoyl chloride (6) (2.3mL, 15.2mmol) successively. The mixture was warmed to room temperature, stirred at same temperature for 45 minutes, stirred at 110 °C for 20 hours and it was poured into water. The precipitated crystal was filtrated, suspended in ice-2 mol/L hydrochloric acid and extracted with ethyl acetate / tetrahydrofuran. The organic layer was washed with saturated sodium hydrogencarbonate aqueous solution, water successively, dried over anhydrous sodium sulfate and concentrated under reduced pressure. The residue was subjected to silica gel column chromatography and the fraction eluted with chloroform / ethyl acetate = 10 /1 was collected, recrystallized from acetone / n-hexane to give a desired product (7) (3.69g, yield 54.8%). Melting point 174-175°C.
$^1$H NMR ($CDCl_3$-$d_6$, δ ppm): 0.89 (d, J = 6.9 Hz, 3H), 0.98 (d, J = 6.6 Hz, 3H), 1.30 (t, J= 7.5 Hz, 3H), 2.07 (m, 1H), 2.76 (q, J= 7.5 Hz, 2H), 3.46 (s, 3H), 3.81 (dd, J= 5.1, 10.2 Hz, 1H), 5.20 (d, J= 10.2 Hz, 1H), 7.40 (d, J= 8.4 Hz, 2H), 7.97 (d, J= 8.7 Hz, 2H), 8.13 (d, J= 8.4 Hz, 2H), 8.31 (d, J = 8.7 Hz, 2H)
Elemental analysis ($C_{22}H_{25}N_3O_5S$)
Calcd.: C;59.58, H;5.68, N;9.48, S;7.23
Found: C;59.34, H;5.50, N;9.62, S;7.47

Process 4

To a solution of a compound (7) (357mg, 0.8 mmol) in dimethyl sulfoxide (10 mL) was added at room temperature 1 mol/L sodium hydroxide aqueous solution (2.4 mL,2.4mmol). The reaction mixture was stirred at room temperature for 4.5 hours, poured into ice-2 mol/L hydrochloric acid and extracted with ethyl acetate. The organic layer was washed with water, dried over anhydrous sodium sulfate and concentrated under reduced pressure. The residue was recrystallized from acetone / n-hexane to give a desired product (C-1) (283mg, yield 81.8%). Melting point 181-183 °C.
IR(KBr, ν max $cm^{-1}$) 3286, 2968, 1714, 1616, 1408, 1350, 1167, 1136, 752
$^1$H NMR (DMSO-$d_6$, δ ppm): 0.81 (d, J = 6.6 Hz, 3H), 0.85 (d, J = 7.0 Hz, 3H), 1.24 (t, J= 7.8 Hz, 3H), 1.97 (m, 1H), 2.75 (q, J= 7.4 Hz, 2H), 3.59 (m, 1H), 7.53 (d, J= 8.4 Hz, 2H), 7.99 (d, J= 8.4 Hz, 2H), 8.13 (d, J= 8.0 Hz, 2H), 8.26 (d, J = 8.8 Hz, 2H), 12.64 (br s, 1H)
$[\alpha]_D$ - 10.0 ± 1.0° (*c* = 0.508, DMSO, 25 °C)
Elemental analysis ($C_{21}H_{23}N_3O_5S$-$0.3H_2O$)
Calcd.: C;58.00, H;5.47, N;9.66, S;7.37
Found: C;58.03, H;5.45, N;9.63, S;7.44

Example 2 Preparation of compound F-1

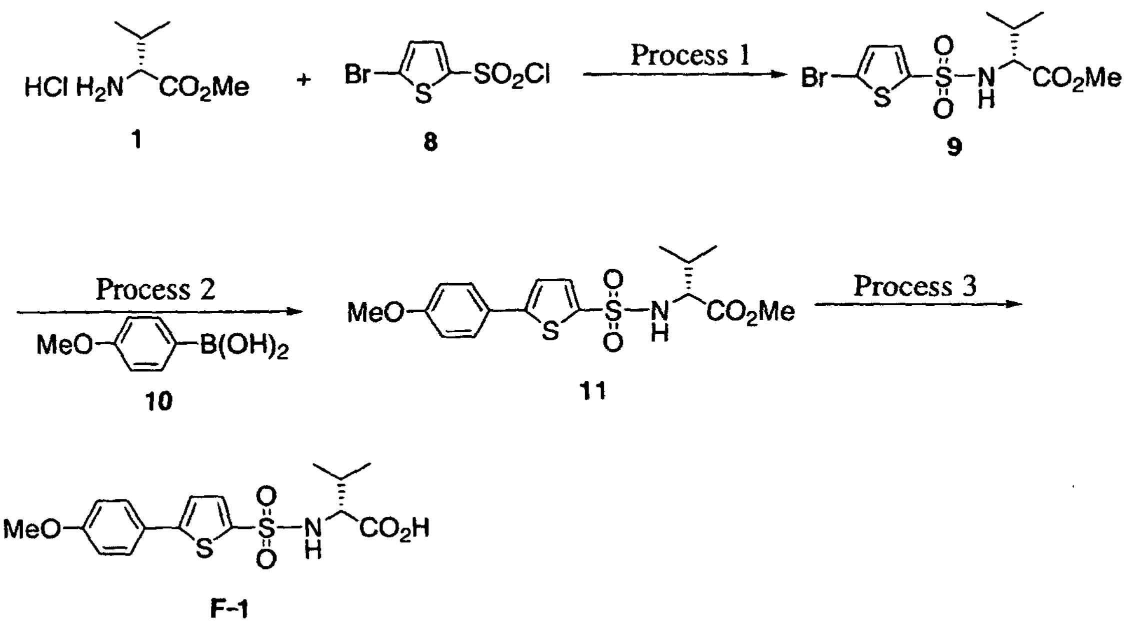


1 8 9 10 11 F-1

Process 1

To a solution of D-valine methyl ester hydrochloride (1) (755 mg, 4.5 mmol) in dichloromethane (12 mL) was added N-methylmorpholine (1.49 ml, 3 x 4.5 mmol) and 5-bromo-2-thiophensulfonyl chloride (8) (1.24 g, 1.05 x 4.5 mmol) was added under ice-cooling. After being stirred for 15 h at room temperature, the reaction mixture was washed with 2 mol/L hydrochloric acid, 5% sodium hydrogencarbonate aqueous solution, and water successively. The organic layer was dried over sodium sulfate, and concentrated in vacuo. The residue was subjected to silica gel column chromatography and the fractions eluting with ethyl acetate / hexane = 1/3 were collected and washed with n-hexane to give 1.32 g (yield 82%) of the desired compound (9) with a melting point of 109-110°C.
Elemental analysis $C_{10}H_{14}BrNO_4S_2$
Calcd. : C; 33.71 H; 3.96 Br; 22.43 N; 3.93 S;18.00
Found : C; 33.75 H; 3.89 Br; 22.43 N; 3.96 S; 17.86
$[\alpha]_D$ : -34.5±0.7(c=1.012 $CHCl_3$ 25°C)
IR($CHCl_3$, ν max $cm^{-1}$) 1737,1356,1164,1138
NMR($CDCl_3$, δ ppm): 0.89(d,J=6.8Hz,3H), 1.00(d,J=6.8Hz,3H), 2.00 (m,1H), 3.60(s,3H), 3.83(dd,J=5.2,10.0Hz,1H), 5.20(d,J=10.0Hz,1H), 7.04(d,J=4.1Hz, 1H), 7.32(d,J=4.1Hz,1H)

Process 2

To a solution of compound (9) (500 mg, 1.4 mmol) obtained by Process 1 in dry tetrahydrofuran (12 mL) were added powdery potassium carbonate (387 mg, 2 x 1.4 mmol), 4-methoxyphenylboronic acid (10) (319 mg, 1.5 x 1.4 mmol) and tetrakis(triphenylphosphine)palladium (81 mg, 0.05 x 1.4 mmol). The resulting mixture was stirred under argon atmosphere for 48 h at 75°C. The reaction mixture was diluted with ethyl acetate. The organic layer was washed with 1mol/L hydrochloric acid, 5% sodium hydrogencarbonate aqueous solution, and water successively, dried over sodium sulfate, and concentrated in vacuo. The residue was column chromatographed on silica gel. The fractions eluting with n-hexane / ethyl acetate = 3 / 1 were collected and recrystallized from n-hexane to give the desired compound (11) (447 mg, yield 83 %) with a melting point of 122-123°C.
Elemental analysis $C_{17}H_{21}NO_5S_2$
Calcd. : C; 53.25 H; 5.52 N; 3.65 S; 16.72
Found : C; 53.26 H; 5.50 N; 3.69 S; 16.63
$[\alpha]_D$-21.7±0.6(c=1.000 DMSO 25 °C)
IR(KBr, ν max $cm^{-1}$)1735,1605,1505,1350,1167,1136

NMR($CDCl_3$, δ ppm):0.90(d,J=7.0Hz,3H), 1.00(d,J=6.6Hz,3H), 2.10(m, 1H), 3.54(s,3H), 3.85(s,3H), 3.87(dd, J=5.0,10.2Hz,1H), 5.20(d,J=10.2Hz,1H), 6.94 (d,J=9.0Hz,2H),7.52(d,J=9.0Hz,2H),7.11(d,J=4.0Hz,1H),7.49(d, J=4.0Hz,1H)

Process 3

To a solution of compound (11) (390 mg, 1.01 mmol) in tetrahydrofuran (8mL) and methanol (8mL) was added 1 mol/L sodium hydroxide aqueous solution (5.1 ml), and the resulting mixture was stirred at 60°C for 6 h. The reaction mixture was concentrated in vacuo to remove an organic solvent. The resulting residue was diluted with ethyl acetate. The mixture was acidified with aqueous solution of citric acid and extracted with ethyl acetate. The organic layer was washed with brine, dried over sodium sulfate, and concentrated in vacuo to give 373 mg (yield 100%) of compound (F-1).
mp. : 174 - 176°C
IR(KBr, ν max $cm^{-1}$) : 1735, 1503, 1343, 1163.

Example 3 Preparation of compound N-7

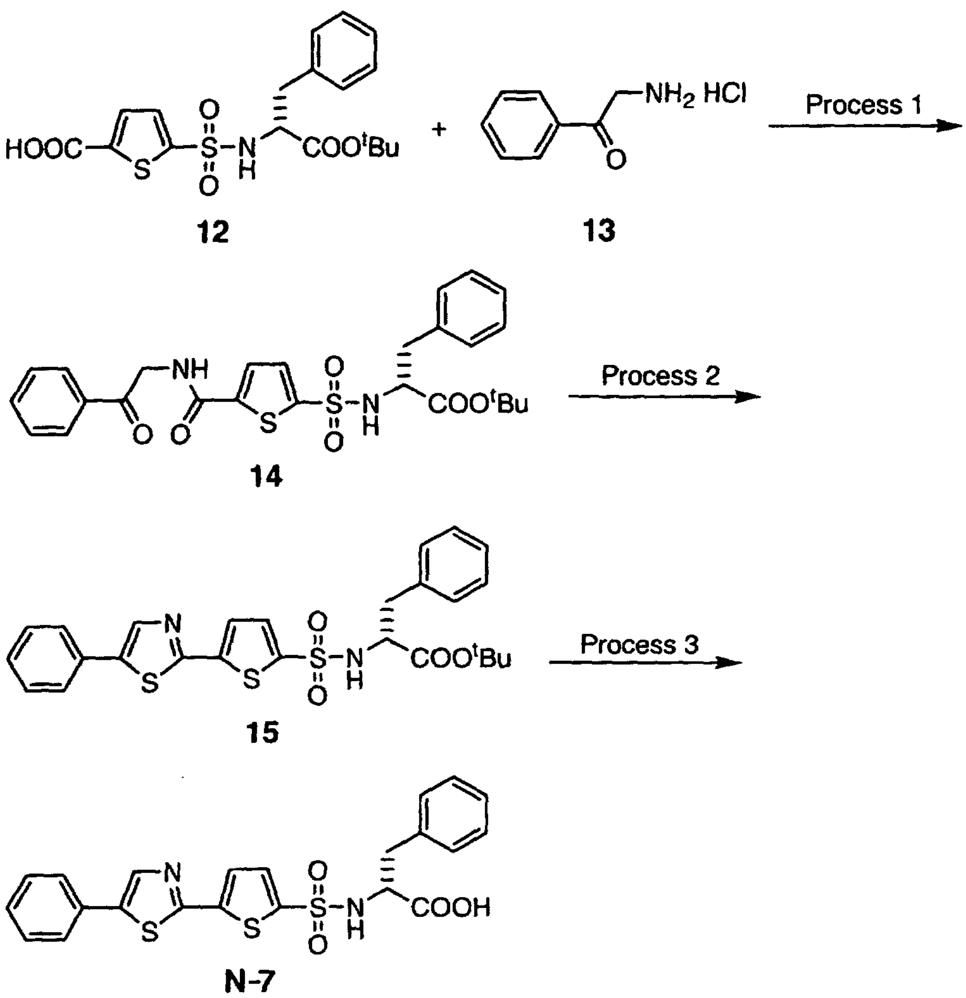


12 13 14 15 N-7

(Process 1)

Oxalyl chloride (0.25 ml, 2.87 mmol) was added to a solution of compound (12) (1.00 g, 2.43 mmol) prepared in accordance with a method described in Patent Document 13 and dimethylformamide (0.05 ml) in tetrahydrofuran (20 mL). The mixture was stirred at a room temperature for 6 h. Compound (13) (0.501 g, 2.92 mmol) and then *N*-methylmorpholine (0.80 ml, 7.28 mmol) were added to the reaction mixture. The mixture was stirred at a room temperature for 20 h. Water was added and then 2 mol/L hydrochloric acid was added to acidify. The mixture was extracted with ethyl acetate. The organic phase was washed with 5% aqueous sodium hydrogencarbonate solution and water successively, dried over anhydrous sodium sulfate and concentrated under a reduced pressure. The residue was washed with hexane / ethyl acetate =2 / 1 to give a desired product (14, 947 mg, yield 73.7%).
$^1$H NMR ($CDCl_3$, δ ppm): 1.30 (s, 9H), 3.08 (d, J = 5.8 Hz, 2H), 4.23 (m, 1H), 4.93 (d, J = 3.7 Hz, 2H), 5.33 (d, J = 9.2 Hz, 1H), 7.15-7.28 (m, 6H), 7.46-7.57 (m, 4H), 7.68 (m, 1H), 8.02 (d, J = 8.5 Hz, 1H)

(Process 2)

A solution of compound (14) (470 mg, 0.889 mmol) and Lawesson's reagent (360 mg, 0.890 mmol) in tetrahydrofuran (5 mL) was stirred at 70 °C for 4 h. Lawesson's reagent (180 mg, 0.445 mmol) was added to the mixture, which was stirred further 4 h. Water was added to the mixture, which was acidified with 2mol/L hydrochloric acid and extracted with ethyl acetate. The organic phase was washed with 5% aqueous sodium hydrogencarbonate solution and water successively, dried over anhydrous sodium sulfate and concentrated under a reduced pressure. The residue was chromatographed on silica gel in chloroform / ethyl acetate=10 / 1 and crystallized from acetone / hexane to give a desired product (15, 337 mg, yield 71.9%) with a melting point of 163-165 °C.
IR(KBr, ν max $cm^{-1}$) 3446, 3292, 2979, 1712, 1427, 1408, 1367, 1348, 1288, 1157, 1084, 1020, 897
$^1$H NMR ($CDCl_3$, δ ppm): 1.27 (s, 9H), 3.08 (d, J = 5.8 Hz, 2H), 4.24 (m, 1H), 5.28 (d, J = 9.1 Hz, 1H), 7.16-7.30 (m, 6H), 7.36-7.47 (m, 4H), 7.58 (d, J = 6.9 Hz, 2H), 7.97 (s, 1H)
$[\alpha]_D$ - 16.0±1.1 (*c* = 0.506, DMSO, 27°C)

(Process 3)

Trifluoroacetic acid (0.823 ml, 10.7 mmol) was added to a solution of compound (15) (281 mg, 0.534 mmol) in dichloromethane (7 ml). The mixture was stirred for 20 h and concentrated under a reduced pressure. Toluene was added to the residue and the mixture was concentrated under a reduced pressure again. The residue was crystallized from acetone / hexane to give a desired product (N-7, 204 mg, yield 81.1%) with a melting point of 233-234 °C. IR(KBr, ν max $cm^{-1}$) 3340, 3087, 1736, 1701, 1377, 1352, 1165, 1105, 1018, 812 $^1$H NMR (DMSO-$d_6$, δ ppm): 2.76 (dd, J = 9.9, 13.7 Hz, 1H), 3.01 (dd, J = 4.7, 13.5 Hz, 1H), 4.00 (m, 1H), 7.13-7.18 (m, 5H), 7.31 (d, J = 3.8 Hz, 1H), 7.39-7.55 (m, 4H), 7.74 (d, J = 7.1 Hz, 2H), 8.33 (s, 1H), 8.76 (d, J = 6.9 Hz, 1H)
$[\alpha]_D$ - 13.9±1.1 (c = 0.504, DMSO, 27°C)

According to examples, the methods described by the reference, and (Method A) ~ (Method C), the following compound (A-1) ~ compound (A-27), compound (B-1) ~ compound (B-5), compound (C-1) ~ compound (C-10), compound (D-1) ~ compound (D-6), compound (E-1) ~ compound (E-30), compound (F-1) ~ compound (F-26), compound (G-1) ~ compound (G-15), compound (H-1) ~ compound (H-14), compound (I-1) ~ compound (I-5), compound (J-1) ~ compound (J-4), compound (K-1) ~ compound (K-6), compound (L-1) ~ compound (L-5), compound (M-1) ~ compound (M-5), compound (N-1) ~ compound (N-8), compound (O-1) ~ compound (O-10) were synthesized. Their structures and the results of their physical data were shown in Table 1 to 36.

The mark * shows an asymmetric carbon. In regard of the mark **, the melting points instead of their NMR data were shown.

Table 1

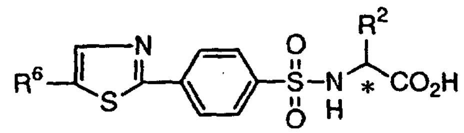

| Example No. | $R^2$ | $R^6$ | * | $^1$H-NMR (DMSO-$d_6$) |
|---|---|---|---|---|
| A-1 | sBu | 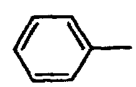 | S | 0.75-0.83 (m, 6H), 1.12 (m, 1H), 1.37 (m, 1H), 1.70 (m, 1H), 3.61 (m, 1H), 7.39-7.53 (m, 3H), 7.77 (d, J=7.2 Hz, 2H), 7.90 (d, J=8.1 Hz, 2H), 8.14 (d, J=8.1 Hz, 2H), 8.21 (d, J=9.6 Hz, 1H), 8.43 (s, 1H), 12.64 (br s, 1H) |
| A-2 | sBu | 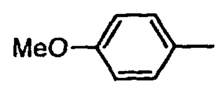 | S | 0.77 (t, J=7.2 Hz, 3H), 0.82 (d, J=6.9 Hz, 3H), 1.12 (m, 1H), 1.37 (m, 1H), 1.69 (m, 1H), 3.60 (m, 1H), 3.82 (s, 3H), 7.06 (d, J=9.0 Hz, 2H), 7.70 (d, J=9.0 Hz, 2H), 7.89 (d, J=8.4 Hz, 2H), 8.11 (d, J=8.7 Hz, 2H), 8.22 (d, J=9.3 Hz, 1H), 8.31 (s, 1H), 12.65 (br s, 1H) |
| A-3 | MeS-$CH_2$-$CH_2$- | 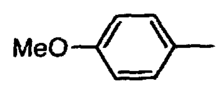 | R | 1.65-1.95 (m, 2H), 1.94 (s, 3H), 2.26-2.50 (m, 2H), 3.82 (s, 3H), 3.91 (m, 1H), 7.06 (d, J=9.0 Hz, 2H), 7.70 (d, J=9.0 Hz, 2H), 7.89 (d, J=8.4 Hz, 2H), 8.12 (d, J=8.4 Hz, 2H), 8.31 (s, 1H), 8.36 (d, J=9.0 Hz, 1H), 12.79 (br s, 1H) |
| A-4 | HOOC-$CH_2$-$CH_2$- | 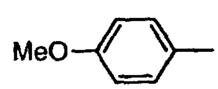 | R | 1.68 (m, 1H), 1.88 (m, 1H), 2.24 (t, J=6.9 Hz, 2H), 3.82 (s, 3H), 3.83 (m, 1H), 7.06 (d, J=8.7 Hz, 2H), 7.70 (d, J=9.0 Hz, 2H), 7.85 (d, J=8.7 Hz, 2H), 8.11 (d, J=8.7 Hz, 2H), 8.30 (s, 2H), 12.42 (br s, 2H) |
| A-5 | iPr | 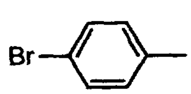 | S | 0.81 (d, J=6.6 Hz, 3H), 0.85 (d, J=6.9 Hz, 3H), 1.97 (m, 1H), 3.57 (m, 1H), 7.67-7.74 (m, 4H), 7.90 (d, J=8.4 Hz, 2H), 8.13 (d, J=8.4 Hz, 2H), 8.20 (d, J=8.4 Hz, 2H), 8.47 (s, 1H), 12.66 (br s, 1H) |
| A-6 | Bn | 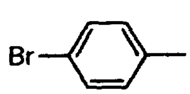 | R | 2.74 (dd, J=9.6, 13.8 Hz, 1H), 2.97 (dd, J=5.7, 13.8 Hz, 1H), 3.94 (m, 1H), 7.10-7.22 (m, 5H), 7.62-7.78 (m, 6H), 7.98 (d, J=8.7 Hz, 2H), 8.46 (d, J=7.5 Hz, 1H), 8.47 (s, 1H), 12.80 (br s, 1H) |

Table 2

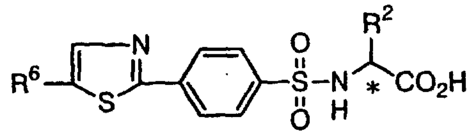

| Example No. | $R^2$ | $R^6$ | * | $^1$H-NMR (DMSO-$d_6$) |
|---|---|---|---|---|
| A-7 | Ph | 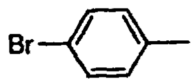 | R | 4.96 (d, J=9.0 Hz, 1H), 7.19-7.33 (m, 5H), 7.66-7.75 (m, 4H), 7.84 (d, J=8.4 Hz, 2H), 8.03 (d, J=8.4 Hz, 2H), 8.45 (s, 1H), 8.89 (d, J=9.0 Hz, 1H) |
| A-8 | MeS-$CH_2$-$CH_2$- | 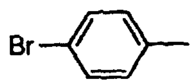 | R | 1.65-1.95 (m, 2H), 1.94 (s, 3H), 2.25-2.50 (m, 2H), 3.91 (m, 1H), 7.67-7.76 (m, 4H), 7.90 (d, J=8.1 Hz, 2H), 8.15 (d, J=8.7 Hz, 2H), 8.38 (d, J=9.3 Hz, 1H), 8.48 (s, 1H), 12.79 (br s, 1H) |
| A-9 | iBu | 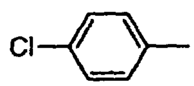 | S | 0.73 (d, J=6.3 Hz, 3H), 0.83 (d, J=6.3 Hz, 3H), 1.38-1.46 (m, 2H), 1.61 (m, 1H), 3.71 (m, 1H), 7.56 (d, J=8.4 Hz, 2H), 7.80 (d, J=8.7 Hz, 2H), 7.89 (d, J=8.7 Hz, 2H), 8.14 (d, J=8.4 Hz, 2H), 8.33 (m, 1H), 8.46 (s, 1H), 12.65 (br s, 1H) |
| A-10 | Ph | 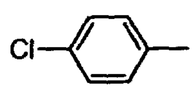 | S | 4.97 (d, J=8.7 Hz, 1H), 7.18-7.33 (m, 5H), 7.56 (d, J=8.4 Hz, 2H), 7.79 (d, J=8.4 Hz, 2H), 7.84 (d, J=8.4 Hz, 2H), 8.03 (d, J=8.4 Hz, 2H), 8.45 (s, 1H), 8.90 (d, J=8.7 Hz, 1H), 12.98 (br s, 1H) |
| A-11 | iBu | 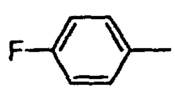 | S | 0.73 (d, J=6.3 Hz, 3H), 0.83 (d, J=6.6 Hz, 3H), 1.38-1.45 (m, 2H), 1.60 (m, 1H), 3.70 (m, 1H), 7.35 (t, J=9.0 Hz, 2H), 7.82 (dd, J=5.4, 9.0 Hz, 2H), 7.89 (d, J=8.7 Hz, 2H), 8.13 (d, J=8.4 Hz, 2H), 8.32 (d, J=7.5 Hz, 1H), 8.40 (s, 1H), 12.68 (br s, 1H) |
| A-12 | Me | 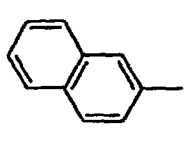 | R | 1.21 (d, J=7.5 Hz, 3H), 3.86 (m, 1H), 7.53-7.63 (m, 2H), 7.90-8.07(m, 6H), 8.18 (d, J=8.1 Hz, 2H), 8.31 (d, J=1.2 Hz, 1H), 8.33 (d, J=8.7 Hz, 1H), 8.57 (s, 1H), 12.69 (br s, 1H) |

Table 3

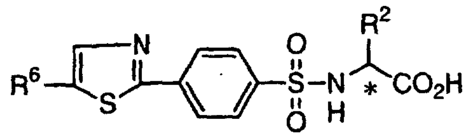

| Example No. | $R^2$ | $R^6$ | * | $^1$H-NMR (DMSO-$d_6$) |
|---|---|---|---|---|
| A-13 | Me | 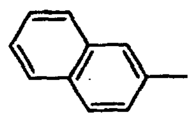 | S | 1.21 (d, J=7.5 Hz, 3H), 3.86 (m, 1H), 7.53-7.63 (m, 2H), 7.91-8.07(m, 6H), 8.18 (d, J=8.1 Hz, 2H), 8.31 (s, 1H), 8.33 (d, J=8.7 Hz, 1H), 8.57 (s, 1H), 12.69 (br s, 1H) |
| A-14 | (Indol-3-yl)-methyl | 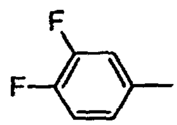 | R | 2.87 (dd, J=8.7, 14.7 Hz, 1H), 3.08 (dd, J=5.7, 14.7 Hz, 1H), 3.94(m, 1H), 6.84-6.99 (m, 2H), 7.08 (d, J=2.4 Hz, 1H), 7.29 (d, J=8.4 Hz, 1H), 7.32 (d, J=8.4 Hz, 1H), 7.54-7.66 (m, 4H), 7.85 (d, J=8.7 Hz, 2H), 7.96 (m, 1H), 8.40 (m, 1H), 8.45 (s, 1H), 10.72 (d, J=2.4 Hz, 1H) |
| A-15 | $CH_3S$-$CH_2$-$CH_2$- | 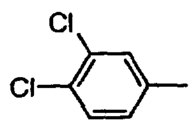 | R | 1.68-1.92 (m, 2H), 1.94 (s, 3H), 2.26-2.46 (m, 2H), 3.90 (m, 1H), 7.73 (m, 1H), 7.77 (m, 1H), 7.90 (d, J=8.4 Hz, 2H), 8.10 (m, 1H), 8.15 (d, J=8.4 Hz, 2H), 8.37 (d, J=9.0 Hz, 1H), 8.56 (s, 1H), 12.80 (br s, 1H) |
| A-16 | HOOC-$CH_2$- | 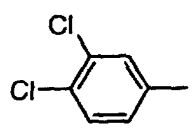 | R | 2.46 (dd, J=6.6, 16.5 Hz, 1H), 2.62 (dd, J=6.6, 16.5 Hz, 1H), 4.11 (m, 1H), 7.72 (m, 1H), 7.76 (m, 1H), 7.92 (d, J=8.4 Hz, 2H), 8.12 (m, 1H), 8.14 (d, J=8.4 Hz, 2H), 8.41 (br s, 1H), 8.56 (s, 1H), 12.64 (br, 2H) |
| A-17 | Me | 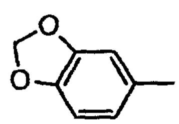 | R | 1.18 (d, J=7.5 Hz, 3H), 3.82 (m, 1H), 6.10 (s, 2H), 7.03 (d, J=8.1 Hz, 1H), 7.22 (dd, J=1.8, 8.1 Hz, 1H), 7.42 (d, J=1.8 Hz, 1H), 7.90 (d, J=8.7 Hz, 2H), 8.11 (d, J=8.7 Hz, 2H), 8.28 (br, 1H), 8.31 (s, 1H), 12.63 (br s, 1H) |

Table 4

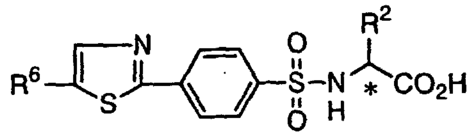

| Example No. | $R^2$ | $R^6$ | * | $^1$H-NMR (DMSO-$d_6$) |
|---|---|---|---|---|
| A-18 | iPr | 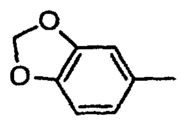 | R | 0.81 (d, J=6.6 Hz, 3H), 0.84 (d, J=6.6 Hz, 3H), 1.96 (m, 1H), 3.56 (m, 1H), 6.10 (s, 2H), 7.02 (d, J=8.4 Hz, 1H), 7.22 (dd, J=1.8, 8.4 Hz, 1H), 7.41 (d, J=1.8 Hz, 1H), 7.88 (d, J=8.4 Hz, 2H), 8.09 (d, J=8.4 Hz, 2H), 8.19 (d, J=9.0 Hz, 1H), 8.31 (s, 1H), 12.61 (br s, 1H) |
| A-19 | MeS-$CH_2$-$CH_2$- | 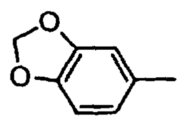 | R | 1.67-1.90 (m, 2H), 1.93 (s, 3H), 2.26-2.46 (m, 2H), 3.90 (m, 1H), 6.10 (s, 2H), 7.02 (d, J=8.1 Hz, 1H), 7.22 (dd, 1.8, 8.1 Hz, 1H), 7.41 (d, J=1.8 Hz, 1H), 7.88 (d, J=8.4 Hz, 2H), 8.11 (d, J=8.4 Hz, 2H), 8.31 (s, 1H), 8.35 (d, J=8.7 Hz, 1H), 12.78 (br, 1H) |
| A-20 | HOOC-$CH_2$ | 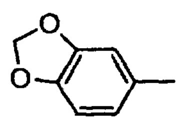 | R | 2.64 (dd, J=6.6, 14.7 Hz, 1H), 2.62 (dd, J=6.6, 14.7 Hz, 1H), 4.11 (m, 1H), 6.10 (s, 2H), 7.03 (d, J=8.4 Hz, 1H), 7.22 (dd, J=1.8, 8.4 Hz, 1H), 7.41 (d, J=1.8 Hz, 1H), 7.90 (d, J=8.1 Hz, 2H), 8.08 (d, J=8.1 Hz, 2H), 8.32 (s, 1H), 8.36 (m, 1H), 12.59 (br, 2H) |
| A-21 | iBu | 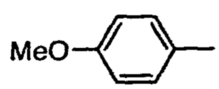 | S | 0.73 (d, J=6.6 Hz, 3H), 0.83 (d, J=6.9 Hz, 3H), 1.38-1.44 (m, 2H), 1.61 (m, 1H), 3.71 (m, 1H), 3.82 (s, 3H), 7.06 (d, J=9.0 Hz, 2H), 7.69 (d, J=8.7 Hz, 2H), 7.88 (d, J=8.7Hz, 2H), 8.11 (d, J=8.7 Hz, 2H), 8.30 (s, 2H), 12.60 (br s, 1H) |
| A-22 | HOOC-CH2-CH2- | 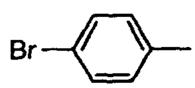 | R | 1.69 (m, 1H), 1.89 (m, 1H), 2.24 (t, J=6.9 Hz, 2H), 3.83 (m, 1H), 7.67-7.76 (m, 4H), 7.86-7.92 (m, 2H), 8.11-8.17 (m, 2H), 8.34 (br s, 1H), 8.47 (s, 1H), 12.42 (br s, 2H) |

Table 5

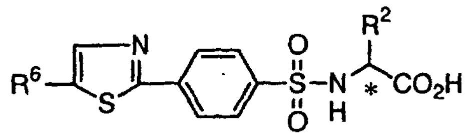

| Example No. | $R^2$ | $R^6$ | * | $^1$H-NMR (DMSO-$d_6$) |
|---|---|---|---|---|
| A-23 | iPr | 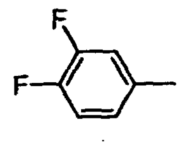 | R | 0.81 (d, J=7.2 Hz, 3H), 0.85 (d, J=6.9 Hz, 3H), 1.96 (m, 1H), 3.57 (m, 1H), 7.60 (d, J=6.6 Hz, 2H), 7.91 (d, J=8.4 Hz, 2H), 7.98 (m, 1H), 8.13 (d, J=8.7 Hz, 2H), 8.21 (d, J=9.6 Hz, 1H), 8.47 (s, 1H), 12.7 (br s, 1H) |
| A-24 | iBu | 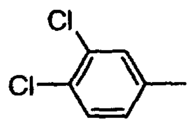 | R | 0.73 (d, J=6.3 Hz, 3H), 0.83 (d, J=6.9 Hz, 3H), 1.36-1.48 (m, 2H), 1.61 (m, 1H), 3.72 (m, 1H), 7.72-7.80 (m, 2H), 7.90 (d, J=8.1 Hz, 2H), 8.12-8.20 (m, 3H), 8.33 (d, J=9.0 Hz, 1H), 8.56 (s, 1H), 12.60 (br s, 1H) |
| A-25 | iPr | 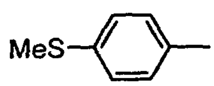 | R | 0.81 (d, J=6.9 Hz, 3H), 0.85 (d, J=6.9 Hz, 3H), 1.97 (m, 1H), 2.53 (s, 3H), 3.57 (dd, J=6.0, 8.7 Hz, 1H), 7.33-7.39 (m, 2H), 7.66-7.73 (m, 2H), 7.87-7.92 (m, 2H), 8.08-8.15 (m, 2H), 8.18 (d, J=8.7 Hz, 1H), 8.40 (s, 1H), 12.62 (br s, 1H) |
| A-26 | iPr | 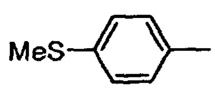 | S | 0.81 (d, J=6.9 Hz, 3H), 0.85 (d, J=6.6 Hz, 3H), 1.97 (m, 1H), 2.53 (s, 3H), 3.57 (dd, J=6.6, 9.0 Hz, 1H), 7.33-7.40 (m, 2H), 7.66-7.73 (m, 2H), 7.87-7.93 (m, 2H), 8.09-8.15 (m, 2H), 8.18 (d, J=9.0 Hz, 1H), 8.39 (s, 1H), 12.63 (br s, 1H) |
| A-27 | Me | 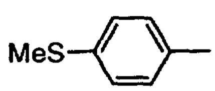 | S | 1.19 (d, J=7.2 Hz, 3H), 2.53 (s, 3H), 3.83 (m, 1H), 7.33-7.39 (m, 2H), 7.66-7.73 (m, 2H), 7.88-7.93 (m, 2H), 8.11-8.16 (m, 2H), 8.31 (d, J=8.7 Hz, 1H), 8.40 (s, 1H), 12.67 (br s, 1H) |

Table 6

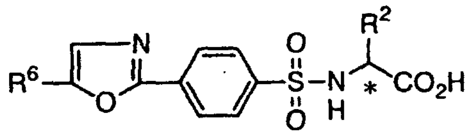

| Example No. | $R^2$ | $R^6$ | * | $^1H$-NMR (DMSO-$d_6$) |
|---|---|---|---|---|
| B-1 | HOOC-$CH_2$-$CH_2$- | 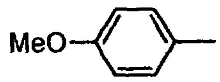 | R | 1.67 (m, 1H), 1.88 (m, 1H), 2.23 (t, J=7.8 Hz, 2H), 3.83 (s, 4H), 7.09 (d, J=9.0 Hz, 2H), 7.79 (s, 1H), 7.82 (d, J=8.7 Hz, 2H), 7.91 (d, J=8.7 Hz, 2H), 8.22 (d, J=8.4 Hz, 2H), 8.34 (br s, 1H), 12.44 (br s, 2H) |
| B-2 | Me | 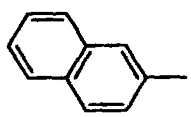 | S | 1.21 (d, J=7.5Hz, 3H), 3.87 (m, 1H), 7.54-7.65 (m, 2H), 7.95-8.09 (m, 7H), 8.29-8.41 (m, 3H), 7.94 (s, 1H), 7.96 (d, J=8.1Hz, 2H), 8.47 (s, 1H), 12.68 (br s, 1H) |
| B-3 | Bn | 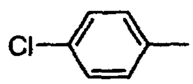 | R | 2.74 (dd, J=9.3, 13.8 Hz, 1H), 2.94 (dd, J=5.4, 13,8 Hz, 1H), 3.94 (m, 1H), 7.10-7.25 (m, 5H), 7.61 (d, J=8.7 Hz, 2H), 7.72 (d, J=8.7 Hz, 2H), 7.92 (d, J=8.7 Hz, 2H), 7.98 (s, 1H), 8.12 (d, J=8.7 Hz, 2H), 8.49 (d, J=9.9 Hz, 1H) |
| B-4 | iBu | 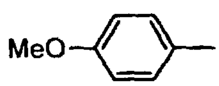 | S | 0.73 (d, J=6.6 Hz, 3H), 0.83 (d, J=6.6 Hz, 3H), 1.36-1.47 (m, 2H), 1.60 (m, 1H), 3.71 (m, 1H), 3.83 (s, 3H), 7.09 (d, J=9.0 Hz, 2H), 7.79 (s, 1H), 7.83 (d, J=9.0 Hz, 2H), 7.91 (d, J=8.7 Hz, 2H), 8.23 (d, J=8.4 Hz, 2H), 8.32 (d, J=8.1 Hz, 1H), 12.60 (br s, 1H) |
| B-5 | Bn | 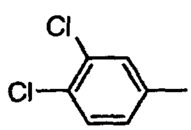 | R | 2.74 (dd, J=9.6, 12.3 Hz, 1H), 2.97 (dd, J=5.4, 13,2 Hz, 1H), 3.94 (m, 1H), 7.10-7.24 (m, 5H), 7.72 (d, J=8.7 Hz, 2H), 7.80 (d, J=8.7 Hz, 1H), 7.86-7.93 (m, 1H), 8.09 (s, 1H), 8.17 (d, J=8.4 Hz, 2H), 8.23 (s, 1H), 8.52 (br s, 1H) |

Table 7

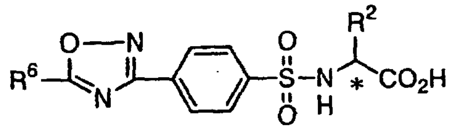

| Example No. | $R^2$ | $R^6$ | * | $^1$H-NMR (DMSO-$d_6$) |
|---|---|---|---|---|
| C-1 | iPr | 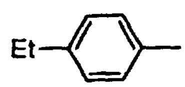 | R | 0.83(t, J=6.8 Hz, 6H), 1.24(t, J=7.8 Hz, 3H), 1.97(m, 1H), 2.75(q, J=7.4 Hz, 2H), 3.59(m, 1H), 7.53(d, J=8.4 Hz, 2H), 7.99(d, J=8.4Hz, 2H), 8.13 (d, J=8.0Hz, 2H), 8.26(d, J=8.8Hz, 2 H), 8.20-8.30(1H), 12.64(br s, 1H) |
| C-2 | Bn | 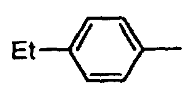 | R | 1.25 (t, J=7.5 Hz, 3H), 2.69-2.81 (m, 3H), 2.98 (dd, J=5.4, 13.5 Hz, 1H), 3.96 (m, 1H), 7.10-7.23 (m, 5H), 7.53 (d, J=8.1 Hz, 2H), 7.76 (d, J=8.7 Hz, 2H), 8.09-8.16 (m, 4H), 8.51 (d, J=8.7 Hz, 1H), 12.78 (br s, 1H) |
| C-3 | Me | 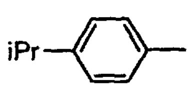 | R | 1.20 (d, J=7.2 Hz, 3H), 1.27 (d, J=7.2 Hz, 6H), 3.04 (m, 1H), 3.86 (m, 1H), 7.56 (d, J=8.4 Hz, 2H), 7.99-8.01 (m, 2H), 8.14 (d, J=8.4 Hz, 2H), 8.26-8.29 (m, 2H), 8.39 (d, J=8.1 Hz, 1H), 12.72 (br s, 1H) |
| C-4 | iPr | 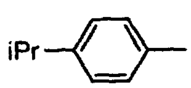 | R | 0.83 (dd, J=6.6, 10.8 Hz, 6H), 1.27 (d, J=6.9 Hz, 6H), 1.97 (m, 1H), 3.04 (m, 1H), 3.58 (s, 1H), 7.56 (d, J=8.4 Hz, 2H), 7.99 (d, J=8.7 Hz, 2H), 8.14 (d, J=8.4 Hz, 2H), 8.26 (d, J=8.7 Hz, 3H), 12.86 (br s, 1H), |
| C-5 | Bn | 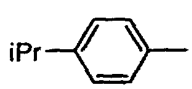 | R | 1.27 (d, J=6.9 Hz, 6H), 2.75 (dd, J=9.6, 13.8 Hz, 1H), 2.95-3.09 (m, 2H), 7.11-7.21 (m, 5H), 7.57 (d, J=8.4 Hz, 2H), 7.76 (d, J=8.4 Hz, 2H), 8.13 (t, J=7.2 Hz, 4H), 8.53 (d, J=9.3 Hz, 1H), 12.82 (br s, 1H) |
| C-6 | MeS-$CH_2$-$CH_2$- | 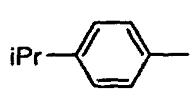 | R | 1.27 (d, J= 6.9 Hz, 6H), 1.74-1.92 (m, 2H), 1.95 (s, 3H), 2.29-2.46 (m,2 H), 3.04 (m, 1H), 3.92 (m, 1H), 7.56 (d, J=8.4 Hz, 2H), 7.98-8.01 (m, 2H), 8.14 (d, J=8.4 Hz, 2H), 8.26-8.29 (m, 2H), 8.42 (d, J=8.1 Hz, 1H), 12.80 (br s, 1H) |
| C-7 | HOOC-$CH_2$- | 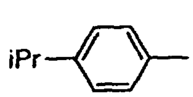 | R | 1.27 (d, J= 6.9 Hz, 6H), 2.48 (m, 1H), 2.64 (dd, J=6.3, 16.5 Hz, 1H), 3.04 (m, 1H), 4.14 (t, J=6.0 Hz, 1H), 7.56 (d, J=8.4 Hz, 2H), 8.00 (d, J=8.7 Hz, 2H), , 8.14 (d, J=8.4 Hz, 2H), 8.26 (d, J=8.7 Hz, 2H), 8.46 (br s, 1H), 12.62 (br s, 2H) |

Table 8

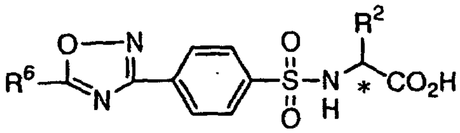

| Example No. | $R^2$ | $R^6$ | * | $^1$H-NMR (DMSO-$d_6$) |
|---|---|---|---|---|
| C-8 | Bn | 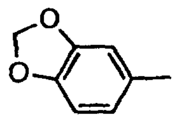 | S | 2.74 (dd, J=9.6, 13.8 Hz, 1H), 2.98 (d, J=5.4, 13.8 Hz, 1H), 3.95 (dt, J=5.1, 8.4 Hz, 1H), 6.22 (s, 2H), 7.10-7.23 (m, 6H), 7.67 (d, J=1.5 Hz, 1H), 7.75(d, J=8. 4 Hz, 2H), 7.81 (dd, J=1.5, 8.4 Hz, 1H), 8.06 (d, J=8.4 Hz, 2H), 8.51 (d, J=9.0 Hz, 1H), 12.81 (br s, 1H) |
| C-9 | Bn | 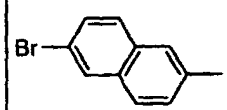 | S | 2.76 (dd, J=9.6,13.5Hz, 1H), 3.00 (d, J=5.4,13.5Hz, 1H), 3.98 (m, 1H), 7.10-7.25 (m, 5H), 7.76-7.85 (m, 3H), 8.12-8.30 (m, 5H), 8.40 (s, 1H), 8.53 (d, J=8.4Hz, 1H), 8.94 (s, 1H), 12.83 (br s, 1H) |
| C-10 | Bn | 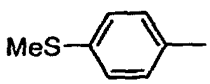 | R | 2.59 (s, 3H), 2.74 (dd, J=9.6, 13.8 Hz, 1H), 2.98 (dd, J=5.4, 13.8 Hz, 1H), 3.95 (m, 1H), 7.10-7.23 (m, 5H), 7.52 (d, J=8.4 Hz, 2H), 7.75 (d, J=8.4 Hz, 2H), 8.08-8.15 (m, 4H), 8.52 (d, J=9.0 Hz, 1H), 12.80 (br s, 1H) |

Table 9

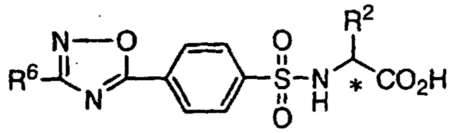

| Example No. | $R^2$ | $R^6$ | * | $^1$H-NMR (DMSO-$d_6$) |
|---|---|---|---|---|
| D-1 | iPr | 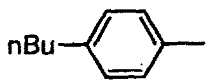 | R | 0.75-1.10 (m, 9H), 1.20-1.45 (m, 2H), 1.50-1.75 (m, 2H), 1.98 (m, 1H), 2.60-2.75 (m, 2H), 3.61 (m, 1H), 7.44 (d, J=8.2 Hz, 2H), 8.02 (d, J=8.4 Hz, 2H), 8.04 (d, J=8.4 Hz, 2H), 8.35 (d, J=8.4 Hz, 2H), 8.31(m, 1H) |
| D-2 | Bn | 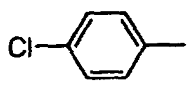 | R | 2.74 (dd, J=9.1, 13.8 Hz, 1H), 2.99 (dd, J=5.1, 13.8 Hz, 1H), 3.95 (brs, 1H), 7.09-7.26 (m, 5H), 7.71 (d, J=8.7 Hz, 2H), 7.79 (d, J=8.7 Hz, 2H), 8.14 (d, J=8.7 Hz, 2H), 8.21 (d, J=8.7 Hz, 2H), 8.58 (brs, 1H), 12.77 (brs, 1H) |
| D-3 | Bn | 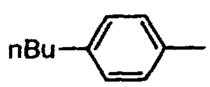 | R | 0.92 (t, J=7.4 Hz, 3H), 1.25-1.45 (m, 2H), 1.50-1.70 (m, 2H), 2.60-2.85 (m, 3H), 2.99 (dd, J=4.8, 13.6 Hz, 1H), 3.97 (m, 1H), 7.10-7.25 (m, 5H), 7.44 (d, J=8.4 Hz, 2H), 7.79 (d, J=8.4 Hz, 2H), 8.03 (d, J=8.0 Hz, 2H), 8.20 (d, J=8.4 Hz, 2H), 8.60 (m, 1H) |
| D-4 | Bn | 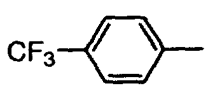 | R | 2.75 (dd, J=9.6, 13.8 Hz, 1H), 3.00 (dd, J=5.1, 13.8 Hz, 1H), 3.98 (m, 1H), 7.10-7.25 (m, 5H), 7.80 (d, J=8.7 Hz, 2H), 8.01 (d, J=8.1 Hz, 2H), 8.23 (d, J=8.7 Hz, 2H), 8.34 (d, J=8.4 Hz, 2H), 8.63 (d, J=9.3 Hz, 1H), 12.84 (br s, 1H) |
| D-5 | Bn | 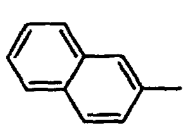 | R | 2.76 (dd, J=9.9,13.8Hz, 1H), 3.00 (d, J=5.1,13.8Hz, 1H), 3.99 (m, 1H), 7.10-7.22 (m, 5H), 7.62-7.71 (m, 2H), 7.82 (d, J=8.4Hz, 2H), 8.05 (m, 1H), 8.12-8.22 (m, 3H), 8.25 (d, J=8.4Hz, 2H), 8.61 (d, J=8.4Hz, 1H), 8.77 (s, 1H), 12.84 (br s, 1H) |

Table 10

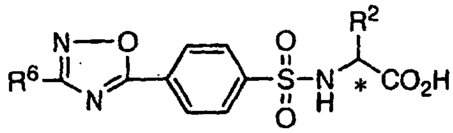

| Example No. | $R^2$ | $R^6$ | * | $^1$H-NMR (DMSO-$d_6$) |
|---|---|---|---|---|
| D-6 | Bn | 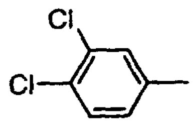 | R | 2.74 (dd, J=9.6, 13.5 Hz, 1H), 2.99 (dd, J=5.1, 14.1 Hz, 1H), 3.97 (m, 1H), 7.08-7.22 (m, 5H), 7.79 (d, J=8.4 Hz, 2H), 7.91 (d, J=8.4 Hz, 1H), 8.09 (dd, J=1.8, 8.4 Hz, 1H), 8.21 (d, J=8.7 Hz, 2H), 8.28 (d, J=1.8 Hz, 1H), 8.63 (d, J=8.1 Hz, 1H), 12.87 (br s, 1H) |

Table 11

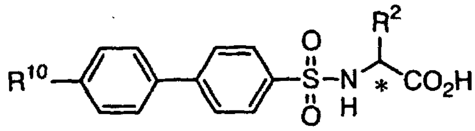

| Example No. | $R^2$ | $R^{10}$ | * | $^1$H-NMR (DMSO-$d_6$) |
|---|---|---|---|---|
| E-1 | iPr | H | R | **m.p.=155-157°C |
| E-2 | (Indol-3-yl)-methyl | Me | R | **m.p.=149-152°C |
| E-3 | iPr | Cl | S | 0.81 (d, J=6.6 Hz, 3H), 0.84 (d, J=6.9 Hz, 3H), 1.96 (m, 1H), 3.56 (dd, J=6.0, 9.0 Hz, 1H), 7.54-7.60 (m, 2H), 7.75-7.81 (m, 2H), 7.86 (s, 4H), 8.09 (d, J=9.0 Hz, 1H), 12.61 (br s, 1H) |
| E-4 | Me | MeS- | S | 1.18 (d, J=7.2 Hz, 3H), 2.53 (s, 3H), 3.80 (m, 1H), 7.38 (d, J=8.4 Hz, 2H), 7.81-7.88 (m, 4H), 7.70 (d, J=8.4 Hz, 2H), 8.18 (d, J=7.5 Hz, 1H), 12.60 (br s, 1H) |
| E-5 | sBu | MeS- | S | 0.76 (t, J=7.2 Hz, 3H), 0.81 (d, J=6.6 Hz, 3H), 1.11 (m, 1H), 1.37 (m, 1H), 1.68 (m, 1H), 2.53 (s, 3H), 3.59 (m, 1H), 7.38 (d, J=8.4 Hz, 2H), 7.70 (d, J=8.4 Hz, 2H), 7.78-7.85 (m, 4H), 8.09 (d, J=9.6 Hz, 1H), 12.61 (br s, 1H) |
| E-6 | Bn | MeS- | S | 2.53 (s, 3H), 2.73 (dd, J=9.3, 13.8 Hz, 1H), 2.95 (dd, J=5.7, 13.8 Hz, 1H), 3.90 (m, 1H), 7.08-7.22 (m, 5H), 7.38 (d, J=8.4 Hz, 2H), 7.59 (d, J=8.4 Hz, 2H), 7.64-7.72 (m, 4H), 8,32 (d, J=9.0 Hz, 1H), 12.73 (br s, 1H) |
| E-7 | MeS-$CH_2$-$CH_2$- | MeS- | R | 1.65-1.86 (m, 2H), 1.91 (s, 3H), 2.25-2.46 (m, 2H), 2.53 (s, 3H), 3.88 (m, 1H), 7.38 (d, J=8.7 Hz, 2H), 7.70 (d, J=8.7 Hz, 2H), 7.79-7.89 (m, 4H), 8.23 (d, J=8.4 Hz, 1H), 12.77 (br s, 1H) |
| E-8 | MeS-$CH_2$-$CH_2$- | MeS- | S | 1.66-1.90 (m, 2H), 1.91 (s, 3H), 2.25-2.47 (m, 2H), 2.53 (s, 3H), 3.89 (m, 1H), 7.38 (d, J=8.7 Hz, 2H), 7.70 (d, J=8.7 Hz, 2H), 7.80-7.89 (m, 4H), 8.24 (d, J=9.0 Hz, 1H), 12.76 (br s, 1H) |

Table 12

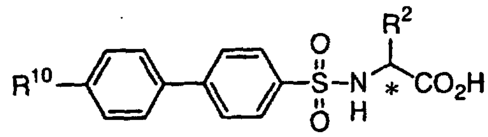

| Example No. | $R^2$ | $R^{10}$ | * | $^1$H-NMR (DMSO-$d_6$) |
|---|---|---|---|---|
| E-9 | HOOC-$CH_2$- | MeS- | R | 2.45 (dd, J=6.9, 16.5 Hz, 1H), 2.53 (s, 3H), 2.63 (dd, J=6.9, 16.5 Hz, 1H), 4.10 (m, 1H), 7.38 (d, J=8.4 Hz, 2H), 7.71 (d, J=8.4 Hz, 2H), 7.80-7.89 (m, 4H), 8.24 (br s, 1H), 12.62 (br s, 2H) |
| E-10 | HOOC-$CH_2$-$CH_2$- | MeS- | R | 1.69 (m, 1H), 1.86 (m, 1H), 2.22 (t, J=7.2 Hz, 2H), 2.53 (s, 3H), 3.81 (m, 1H), 7.35-7.41 (m, 2H), 7.67-7.73 (m, 2H), 7.78-7.87 (m, 4H), 8.19 (d, J=8.1 Hz, 1H), 12.42 (br s, 2H) |
| E-11 | HOOC-$CH_2$-$CH_2$- | MeS- | S | 1.68 (m, 1H), 1.87 (m, 1H), 2.22 (t, J=7.5 Hz, 2H), 2.53 (s, 3H), 3.81 (m, 1H), 7.35-7.41 (m, 2H), 7.67-7.73 (m, 2H), 7.78-7.87 (m, 4H), 8.19 (br s, 1H), 12.42 (br s, 2H) |
| E-12 | HO-$CH_2$- | MeS- | R | 2.53 (s, 3H), 3.47-3.58 (m, 2H), 3.79 (m, 1H), 5.20 (br s, 1H), 7.38 (d, J=8.1 Hz, 2H), 7.71 (d, J=8.1 Hz, 2H), 7.85 (s, 4H), 8.06 (d, J=8.4 Hz, 1H), 12.50 (br s, 1H) |
| E-13 | 2-Thienyl- | MeS- | R | 2.51 (s, 3H), 5.17 (d, J=8.7 Hz, 1H), 6.89 (dd, J=3.6, 5.4Hz, 1H), 6.98 (d, J=5.4 Hz, 1H), 7.38 (d, J=8.7 Hz, 2H), 7.42 (d, J=3.6 Hz, 1H), 7.68 (d, J=8.7 Hz, 2H), 7.75-7.85 (m, 4H), 8.90 (d, J=9.0 Hz, 1H), 13.22 (br s, 1H) |
| E-14 | Me | EtS- | R | 1.18 (d, J=7.2 Hz, 3H), 1.27 (t, J=7.2 Hz, 3H), 3.05 (q, J=7.2 Hz, 2H), 3.80 (m, 1H), 7.42 (d, J=8.4 Hz, 2H), 7.70 (d, J=8.4 Hz, 2H), 7.81-7.89 (m, 4H), 8.18 (d, J=8.1 Hz, 1H), 12.64 (br s, 1H) |

Table 13

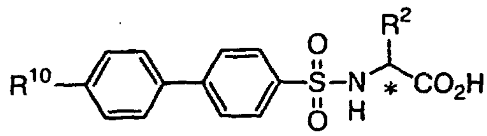

| Example No. | $R^2$ | $R^{10}$ | * | $^1$H-NMR (DMSO-$d_6$) |
|---|---|---|---|---|
| E-15 | iPr | EtS- | R | 0.81 (d, J=6.6 Hz, 3H), 0.84 (d, J=6.6 Hz, 3H), 1.27 (t, J=7.2 Hz, 3H), 1.95 (m, 1H), 3.05 (q, J=7.2 Hz, 2H), 3.55 (dd, J=6.0, 9.0 Hz, 1H), 7.42 (d, J=8.4 Hz, 2H), 7.70 (d, J=8.4 Hz, 2H), 7.80-7.88 (m, 4H), 8.07 (d, J=9.0 Hz, 1H), 12.62 (br s, 1H) |
| E-16 | Bn | EtS- | R | 1.28 (t, J=7.2 Hz, 3H), 2.72 (dd, J=9.0, 13.5 Hz, 1H), 2.96 (dd, J=5.4, 13.5 Hz, 1H), 3.05 (q, J=7.2 Hz, 2H), 3.90 (m, 1H), 7.09-7.22 (m, 5H), 7.42 (d, J=8.4 Hz, 2H), 7.60 (d, J=8.4 Hz, 2H), 7.64-7.72 (m, 4H), 8.32 (d, J=8.7 Hz, 1H), 12.76 (br s, 1H) |
| E-17 | sBu | EtS- | S | 0.77 (t, J=7.2 Hz, 3H), 0.81 (d, J=6.6 Hz, 3H), 1.11 (m, 1H), 1.28 (t, J=7.2 Hz, 3H), 1.37 (m, 1H), 1.68 (m, 1H), 3.05 (q, J=7.2 Hz, 2H), 3.59 (dd, J=6.0, 9.0 Hz, 1H), 7.42 (d, J=8.4 Hz, 2H), 7.70 (d, J=8.4 Hz, 2H), 7.80-7.88 (m, 4H), 8.09 (d, J=9.0 Hz, 1H), 12.60 (br s, 1H) |
| E-18 | iPr | Et | R | 0.81 (d, J=6.9 Hz, 3H), 0.85 (d, J=7.2 Hz, 3H), 1.22 (t, J=7.2 Hz, 3H), 1.96 (m, 1H), 2.67 (q, J=7.2 Hz, 2H), 3.56 (dd, J=5.4, 9.0 Hz, 1H), 7.35 (d, J=7.8 Hz, 2H), 7.66 (d, J=7.8 Hz, 2H), 7.83 (s, 4H), 8.06 (d, J=9.0 Hz, 1H), 12.62 (br s, 1H) |
| E-19 | iPr | EtO- | R | 0.81 (d, J=6.9 Hz, 3H), 0.84 (d, J=6.9 Hz, 3H), 1.35 (t, J=6.9 Hz, 3H), 1.95 (m, 1H), 3.54 (dd, J=6.0, 9.3 Hz, 1H), 4.08 (q, J=6.9 Hz, 2H), 7.04 (d, J=8.7 Hz, 2H), 7.68 (d, J=8.7 Hz, 2H), 7.80 (s, 4H), 8.03 (d, J=9.0 Hz, 1H), 12.60 (br s, 1H) |

Table 14

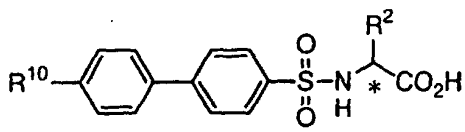

| Example No. | $R^2$ | $R^{10}$ | * | $^1$H-NMR (DMSO-$d_6$) |
|---|---|---|---|---|
| E-20 | iPr | $CF_3O$- | R | 0.81 (d, J=6.9 Hz, 3H), 0.84 (d, J=6.9 Hz, 3H), 1.96 (m, 1H), 3.56 (dd, J=6.0, 9.0 Hz, 1H), 7.50 (d, J=8.4 Hz, 2H), 7.83-7.91 (m, 6H), 8.11 (d, J=9.0 Hz, 1H), 12.62 (br s, 1H) |
| E-21 | iPr | 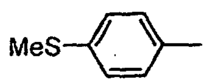 | R | 0.82 (d, J=6.8 Hz, 3H), 0.85 (d, J=6.8 Hz, 3H), 1.96 (m, 1H), 2.53 (s, 3H), 3.57 (dd, J=6.0, 9.3 Hz, 1H), 7.37 (d, J=8.4 Hz, 2H), 7.71 (d, J=8.4 Hz, 2H), 7.81 (d, J=8.4 Hz, 2H), 7.84 (d, J=8.4 Hz, 2H), 7.86 (d, J=8.4 Hz, 2H), 7.92 (d, J=8.4 Hz, 2H), 8.11 (d, J=9.3 Hz, 1H), 12.65 (br s, 1H) |
| E-22 | iPr | 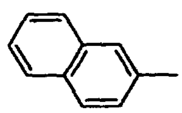 | R | 0.84 (dd, J=9.6, 16.5 Hz, 6H), 1.97 (m, 1H), 3.57 (m, 1H), 7.52-7.60 (m, 2H), 7.86-8.06 (m, 13H), 8.33 (s, 1H), 12.66 (br s, 1H) |
| E-23 | 4-Biphenyl-methyl | H | R | 2.77 (dd, J=9.6, 13.6 Hz, 1H), 3.03 (dd, J=4.8, 13.6 Hz, 1H), 3.93 (m, 1H), 7.10-7.68 (m, 18H), 8.38 (d, J=8.8 Hz, 1H), 12.60 (br s, 1H) |
| E-24 | iPr | 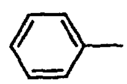 | R | 0.82 (d, J=6.9Hz, 3H), 0.85 (d, J=6.9Hz, 3H), 1.97 (m, 1H), 3.57 (dd, J=6.0, 9.6Hz, 1H), 7.37-7.58 (m, 3H), 7.72-8.00 (m, 9H), 8.06-8.15 (m, 2H) |
| E-25 | Bn | t-Bu | R | 1.33 (s, 9H), 2.73 (dd, J=9.2, 13.6Hz, 1H), 2.96 (dd, J=5.6, 13.6Hz, 1H), 3.91 (td, J=6.0, 9.4Hz, 1H), 7.08-7.22(m,5H), 7.53 (d, J=8.2Hz, 2H), 7.59 (d, J=8.4Hz, 2H), 7.64 (d, J=8.2Hz, 2H), 7.68 (d,J=8.4Hz, 2H), 8.33 (d, J=9.2Hz, 1H), 12.77 (br, 1H |
| E-26 | Bn | F | R | 2.73 (dd, J=9.8, 12.8 Hz, 1H), 2.97 (dd, J=5.6, 14.6 Hz, 1H), 3.91 (m, 1H), 7.15-7.19 (m, 5H), 7.30-7.39 (m, 2H), 7.58-7.79 (m, 6H), 8.34 (d, J=7.0 Hz, 1H) |

Table 15

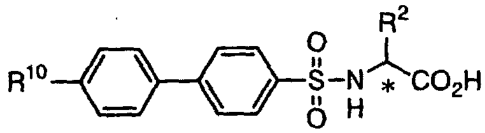

| Example No. | $R^2$ | $R^{10}$ | * | $^1$H-NMR (DMSO-$d_6$) |
|---|---|---|---|---|
| E-27 | (Indol-3-yl)methyl | F | R | 2.88 (dd, J=8.2, 14.6 Hz, 1H), 3.09 (dd, J=6.2, 14.0 Hz, 1H), 3.97 (m, 1H), 6.99-7.38 (m, 7H), 7.61-7.98 (m, 6H), 8.70 (dd, J=8.6 Hz, 1H) |
| E-28 | Me | 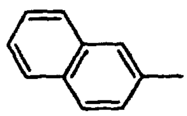 | R | 1.20 (d, J=7.2 Hz, 3H), 3.83 (m, 1H), 7.50-7.62 (m, 2H), 7.86-8.07 (m, 12H), 8.21 (d, J=7.5 Hz, 1H), 8.33 (s, 1H), 12.76 (br s, 1H) |
| E-29 | Bn | 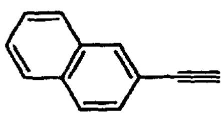 | R | 2.75 (dd, J=9.0, 12.0 Hz, 3H), 2.97 (dd, J=9.0, 12.0 Hz, 1H), 3.92 (m, 1H), 7.12-7.24 (m, 5H), 7.56-7.68 (m, 5H), 7.71-7.85 (m, 6H), 7.94-8.04 (m, 3H), 8.36 (d, J=8.7 Hz, 1H), 12.76 (br s, 1H) |
| E-30 | Bn | MeS | S | 2.53 (s, 3H), 2.73 (dd, 9.3, 13.8 Hz, 1H), 2.95 (dd, J=5.7, 13.8 Hz, 1H), 3.90 (m, 1H), 7.08-7.22 (m, 5H), 7.38 (d, J=8.4 Hz, 2H), 7.59 (d, J=8.4 Hz, 2H), 7.64-7.72 (m, 4H), 8.32 (d, J=9.0 Hz, 1H), 12.73 (br s, 1H) |

Table 16

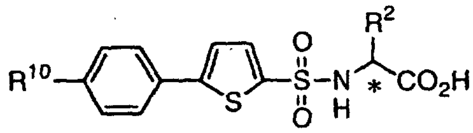

| Example No. | $R^2$ | $R^{10}$ | * | $^1$H-NMR (DMSO-$d_6$) |
|---|---|---|---|---|
| F-1 | iPr | MeO- | R | **m.p.=174-176°C |
| F-2 | iPr | MeO- | S | 0.82 (d, J=6.8 Hz, 3H), 0.87 (d, J=6.8 Hz, 3H), 1.98 (m, 1H), 3.60 (dd, J=9.0, 6.0 Hz, 1H), 3.81 (s, 3H), 7.02 (d, J=8.6 Hz, 2H), 7.41 (d, J=4.0 Hz, 1H), 7.49 (d, J=4.0 Hz, 1H), 7.65 (d, J=8.6 Hz, 2H), 8.29 (d, J=9.4 Hz, 1H), 12.6 (br, 1H) |
| F-3 | MeS-$CH_2$-$CH_2$- | MeS- | R | 1.70-1.95 (m, 2H), 1.95 (s, 3H), 2.35-2.50 (m, 2H), 2.52 (s, 3H), 3.94 (m, 1H), 7.33 (d, J=8.7 Hz, 2H), 7.51 (d, J=4.2 Hz, 1H), 7.53 (d, J=4.2 Hz, 1H), 7.65 (d, J=8.7 Hz, 2H), 8.50 (m, 1H), 12.86 (br, 1H) |
| F-4 | iBu | MeS- | R | 0.75 (d, J=6.6 Hz, 3H), 0.84 (d, J=6.6 Hz, 3H), 1.35-1.52 (m, 2H), 1.63 (m, 1H), 2.52 (s, 3H), 3.75 (m, 1H), 7.33 (d, J=8.7 Hz, 2H), 7.50-7.52 (m, 2H), 7.65 (d, J=8.4 Hz, 2H), 8.44 (d, J=8.7 Hz, 1H), 12.70 (br s, 1H) |
| F-5 | iBu | EtO- | R | 0.75 (d, J=6.7 Hz, 3H), 0.85 (d, J=6.7 Hz, 3H), 1.36 (t, J=7.0 Hz, 3H), 1.40-1.47 (m, 2H), 1.68 (m, 1H), 3.75 (m, 1H), 4.09 (q, J=7.0 Hz, 2H), 6.99 (d, J=8.7 Hz, 2H), 7.40 (d, J=4.0 Hz, 1H), 7.49 (d, J=4.0 Hz, 1H), 7.69 (d, J=8.7 Hz, 2H), 8.38 (d, J=9.0 Hz, 1H) |
| F-6 | MeS-$CH_2$-$CH_2$- | MeO- | R | 1.70-1.92 (m, 2H), 1.95 (s, 3H), 2.30-2.50 (m, 2H), 3.80 (s, 3H), 3.93 (m, 1H), 7.02 (d, J=9.0 Hz, 2H), 7.41 (d, J=3.9 Hz, 1H), 7.50 (d, J=3.9 Hz, 1H), 7.65 (d, J=9.0 Hz, 2H), 8.45 (d, J=9.0 Hz, 1H), 12.85 (br s, 1H) |

Table 17

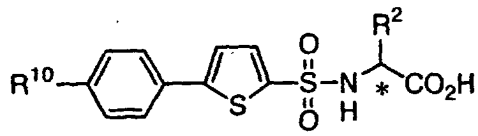

| Example No. | $R^2$ | $R^{10}$ | * | $^1$H-NMR (DMSO-$d_6$) |
|---|---|---|---|---|
| F-7 | (Indol-3-yl)-methyl- | 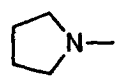 | S | 1.78-2.06 (m, 4H), 2.92 (dd, J=7.5, 14.1 Hz, 1H), 3.09 (dd, J=6.6, 14.7 Hz, 1H), 3.16 -3.44 (m, 4H), 3.99 (m, 1H), 6.58 (d, J=8.7 Hz, 2H), 6.91 (t, J=7.8 Hz, 1H), 7.02 (t, J=7.2 Hz, 1H), 7.26-7.28 (m, 2H), 7.38 (d, J=7.8 Hz, 1H), 7.44 (d, J=8.7 Hz, 2H), 8.39 (d, J=8.4 Hz, 1H), 10.82 (s, 1H), 12.78 (br s, 1H) |
| F-8 | Bn | Br | R | 2.74 (dd, J=9.6, 13.8 Hz, 1H), 2.99 (dd, J=4.5, 13.5 Hz, 1H), 3.95 (m, 1H), 7.08-7.21 (m, 5H), 7.28 (d, J=3.0 Hz, 1H), 7.43 (d, J=3.0 Hz, 1H), 7.59-7.68 (m, 4H), 8.65 (d, J=8.1 Hz, 1H), 12.89 (br s, 1H) |
| F-9 | (Indol-3-yl)-methyl- | MeO | R | 2.91 (dd, J=7.6, 14.6 Hz, 1H), 3.11 (dd, J=6.4, 14.8 Hz, 1H), 3.81 (s, 3H), 4.00 (m, 1H), 6.91-7.11 (m, 4H), 7.20-7.40 (m, 4H), 7.54-7.63 (m, 3H), 8.50 (d, J=8.6 Hz, 1H) |
| F-10 | (Indol-3-yl)-methyl- | Me | R | 2.34 (s, 3H), 2.91 (dd, J=8.4, 14.2 Hz, 1H), 3.11 (dd, J=6.4, 14.6 Hz, 1H), 4.01 (m, 1H), 6.91-7.00 (m, 3H), 7.12-7.53 (m, 4H), 7.49-7.63 (m, 4H), 8.53 (d, J=8.2 Hz, 1H) |
| F-11 | (Indol-3-yl)-methyl- | $Me_2N$ | R | .91 (m, 1H), 2.97 (s, 6H), 3.10 (dd, J=7.2, 14.7Hz, 1H), 4.00 (m, 1H), 6.75 (d, J=8.4Hz, 2H), 6.91 (t, J=7.8Hz, 1H), 6.99 (t, J=8.1Hz, 1H), 7.10-7.15 (m, 2H), 7.26-7.30 (m, 2H), 7.38 (d, J=7.5Hz, 1H), 7.45 (d, J=8.4Hz, 2H), 8.41 (d, J=8.7Hz, 1H), 10.82 (s, 1H), 12.80 (br, 1H) |

Table 18

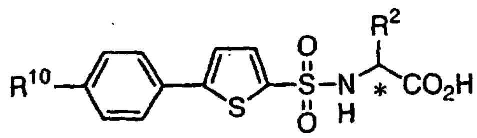

| Example No. | $R^2$ | $R^{10}$ | * | $^1$H-NMR (DMSO-$d_6$) |
|---|---|---|---|---|
| F-12 | Bn | MeNH | R | 2.65-2.85 (m, 4H), 2.97 (dd, J=5.0, 13.2 Hz, 1H), 3.92 (m, 1H), 6.15 (m, 1H), 6.58 (d, J=9.0 Hz, 2H), 7.05-7.30 (m, 7H), 7.39 (d, J=8.6 Hz, 2H), 8.44 (d, J=9.0 Hz, 1H), 12.79 (br s, 1H) |
| F-13 | H | 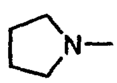 | - | 1.71-1.81 (m, 2H), 1.91-2.02 (m, 2H), 3.22-3.33 (m, 2H), 3.56-3.65 (m, 2H), 3.65 (d, J=5.4 Hz, 2H), 6.58 (d, J=8.7 Hz, 2H), 7.28 (d, J=3.6 Hz, 1H), 7.48 (d, J=3.6 Hz, 1H), 7.51 (d, J=8.7 Hz, 2H), 8.19 (br t, J=5.4 Hz, 1H), 12.76 (br s, 1H) |
| F-14 | Bn | 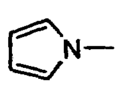 | R | 2.76 (dd, J=9.3, 13.5 Hz, 1H), 3.00 (dd, J=5.1, 13.5 Hz, 1H), 3.96 (brs, 1H), 6.31 (t, J=2.1 Hz, 2H), 7.09-7.23 (m, 5H), 7.30 (d, J=3.9 Hz, 1H), 7.43 (d, J=3.9 Hz, 1H), 7.47 (t, J=2.1 Hz, 2H), 7.69 (d, J=9.0 Hz, 2H), 7.73 (d, J=9.0 Hz, 2H), 8.61 (d, J=8.7 Hz, 1H), 12.89 (brs, 1H) |
| F-15 | Me | 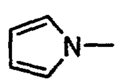 | R | 1.23 (d, J=7.2 Hz, 3H), 3.88 (t, J=7.2 Hz, 1H), 6.30 (t, J=2.1 Hz, 2H), 7.47 (t, J=2.1 Hz, 2H), 7.57 (d, J=3.9 Hz, 1H), 7.58 (d, J=3.9 Hz, 1H), 7.69 (d, J=8.7 Hz, 2H), 7.80 (d, J=8.7 Hz, 2H), 8.46 (d, J=8.1 Hz, 1H), 12.73 (brs, 1H) |
| F-16 | iBu | 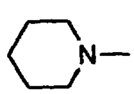 | R | 0.75 (d, J=6.3 Hz, 3H), 0.83 (d, J=6.3 Hz, 3H), 1.39-1.45 (m, 2H), 1.58 (brs, 7H), 3.24 (br, 4H), 3.71 (brs, 1H), 6.97 (d, J=9.0 Hz, 2H), 7.32 (d, J=3.9 Hz, 1H), 7.45 (d, J=3.9 Hz, 1H), 7.51 (d, J=9.0 Hz, 2H), 8.30 (brs, 1H), 12.66 (brs, 1H) |

Table 19

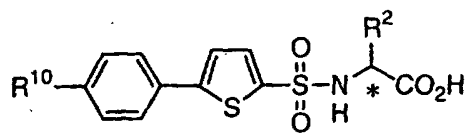

| Example No. | $R^2$ | $R^{10}$ | * | $^1$H-NMR (DMSO-$d_6$) |
|---|---|---|---|---|
| F-17 | 4-Hydroxy-benzene | 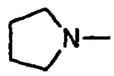 | R | 1.85-2.10 (m, 4H), 3.10-3.50 (m, 4H), 4.80 (d, J=7.8 Hz, 1H), 6.58 (d, J=8.8 Hz, 2H), 6.67 (d, J=8.8 Hz, 2H), 7.12 (d, J=8.4 Hz, 2H), 7.20 (d, J=3.8 Hz, 1H), 7.38 (d, J=4.0 Hz, 1H), 7.47 (d, J=8.8 Hz, 2H), 8.70 (d, J=8.8 Hz, 1H), 9.45 (s, 1H), 12.72 (br s, 1H) |
| F-18 | iPr | 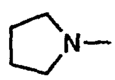 | S | 0.80-0.88 (m, 6H), 1.94-2.00 (m, 5H), 2.25-3.33 (m, 4H), 3.57 (m, 1H), 6.58 (d, J=9.0 Hz, 2H), 7.25 (d, J=3.9 Hz, 1H), 7.42 (d, J=4.2 Hz, 1H), 7.50 (d, J=8.7 Hz, 2H), 8.15 (d, J=7.8 Hz, 1H), 12.72 (br s, 1H) |
| F-19 | (Indol-3-yl)-methyl- | Me | S | 2.34 (s, 3H), 2.94 (dd, J=7.4, 14.6Hz, 1H), 3.12 (dd, J=6.2, 14.8Hz, 1H), 3.98 (m, 1H), 6.86-7.60 (m, 11H), 8.32 (br, 1H), 10.78 (s, 1H) |
| F-20 | iPr | Me | S | 0.82 (d, J=7.0Hz, 3H), 0.87 (d, J=6.6Hz, 3H), 1.98 (m, 1H), 2.34 (s, 3H), 3.58 (d, J=5.8Hz, 1H), 7.27 (d, J=8.2Hz, 2H), 7.38 (m, 1H), 7.47 (d, J=4.2Hz, 1H), 7.51 (d, J=4.2Hz, 1H), 7.60 (d, J=8.2Hz, 2H), 8.20 (br, 1H) |
| F-21 | iPr | MeNH | S | 0.84 (d, J=6.9Hz, 3H), 0.86 (d, J=6.6Hz, 3H), 1.98 (m, 1H), 2.71 (s, 3H), 3.57 (br, 1H), 6.14 (m, 1H), 6.58 (d, J=8.7Hz, 2H), 7.22 (d, J=3.9Hz, 1H), 7.41 (d, J=3.9Hz, 1H), 7.43 (d, J=8.7Hz, 2H), 8.12 (m, 1H), 12.0 (br, 1H) |
| F-22 | Bn | MeNH | S | 2.71 (s, 3H), 2.70-2.80 (m, 1H), 2.97 (dd, J=5.7, 6.9Hz, 1H), 3.92 (m, 1H), 6,12 (br, 1H), 6.58 (d, J=8.7Hz, 2H), 7.1o-7.24 (m, 7H), 7.39 (d, J=8.7Hz, 2H), 8.34 (d, J=9.0Hz, 1H), 12.81 (br, 1H) |

Table 20

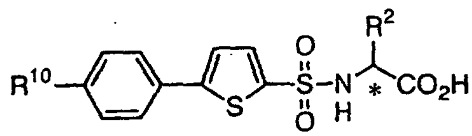

| Example No. | $R^2$ | $R^{10}$ | * | $^1$H-NMR (DMSO-$d_6$) |
|---|---|---|---|---|
| F-23 | MeS-$CH_2CH_2$- | MeS | R | 1.7-2.0 (m, 2H), 1.95 (s, 3H), 2.33-2.50 (m, 2H), 2.54 (s, 3H), 3.92 (m, 1H), 7.33 (d, J=8.4Hz, 2H), 7.51 (d, J=4.2Hz, 1H), 7.52 (d, J=4.2Hz, 1H), 7.65 (d, J=8.4Hz, 2H), 8.22 (br, 1H), 12.80 (br, 1H) |
| F-24 | Bn | MeS | S | 2.51 (s, 3H), 2.75 (dd, J = 5.0, 8.4 Hz, 1H), 2.98 (dd, J = 5.0,8.4 Hz, 1H), 3.90-3.96 (m, 1H), 7.10-7.20 (m, 5H), 7.27 (d, J = 5.0 Hz, 1H), 7.32 (d, J = 8.1 Hz, 2H), 7.35 (d, J = 5.0 Hz, 1H), 7.59 (d, J = 8.1 Hz, 1H), 8.60 (d, J = 8.6 Hz, 1H) |
| F-25 | MeS-$CH_2CH_2$- | Ac | S | 1.80-1.90 (m, 2H), 1.96 (s, 3H), 2.35-2.44 (m, 2H), 2.60 (s, 3H), 3.90-3.96 (m, 1H), 7.60 (d, J=5.0 Hz, 1H), 7.70 (d, J=5.0 Hz, 1H), 7.90 (d, J=8.6 Hz, 2H), 8.09 (d, J=8.6 Hz, 1H), 8.60 (d, J=8.6 Hz, 1H) |
| F-26 | iPr | $CH_2$=CH- | R | 0.82 (d, J=6.6 Hz, 3H), 0.87 (d, J=6.6 Hz, 3H), 1.99 (m, 1H), 3.61 (t-like, J=7 Hz, 1H), 5.33 (d, J=11.4 Hz, 1H), 5.92 (d, J=18.0 Hz, 1H), 6.77 (dd, J=11.1, 18.0 Hz, 2H), 7.52 (d, J=3.9 Hz, 1H), 7.55-7.58 (m, 3H), 7.70 (d, J=8.4 Hz, 2H), 8.33 (d, J=9.0, 1H), 12.72 (brs, 1H) |

Table 21

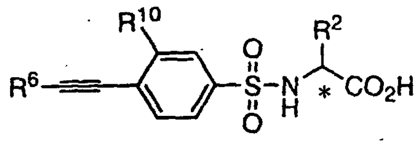

| Example No. | $R^2$ | $R^6$ | $R^{10}$ | * | $^1$H-NMR (DMSO-$d_6$) |
|---|---|---|---|---|---|
| G-1 | (Indol-3-yl)-methyl- | 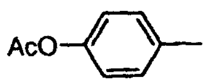 | H | R | 2.30 (s, 3H), 2.88 (dd, J=8.1, 14.4Hz, 1H), 3.08 (dd, J=5.7, 14.4Hz, 1H), 3.92 (m, 1H), 6.92-7.12 (m, 3H), 7.20-7.37 8m, 4H), 7.48-7.68 (m, 6H), 8.37 (m, 1H), 10.81 (s, 1H) |
| G-2 | (Indol-3-yl)-methyl- | 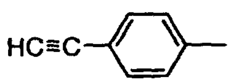 | H | R | 2.88 (dd, J=7.8, 14.4 Hz, 1H), 3.09 (dd, J=6.0, 14.3 Hz, 1H), 3.95 (m, 1H), 4.46 (s, 1H), 6.95-7.11 (m, 3H), 7.29-7.37 (m, 2H), 7.46-7.69 (m, 8H), 8.42 (d, J=8.8 Hz, 1H) |
| G-3 | iPr | 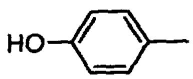 | H | R | 0.77 (d, J=6.6Hz, 3H), 0.80 (d, J=6.6Hz, 3H), 1.93 (m, 1H), 4.24 (d, J=6.0Hz, 1H), 4.39 (s, 1H), 6.87 8d, J=8.6Hz, 2H), 7.41 (d, J=8.6Hz, 2H), 7.71 (d, J=8.6Hz, 2H), 7.94 (d, J=8.6Hz, 2H), 7.99 (d, J=11.0Hz, 1H) |
| G-4 | Bn | 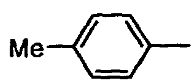 | $NO_2$ | R | 2.38 (s, 3H), 2.70 (dd, J=10.2, 14.1 Hz, 1H), 3.01 (dd, J=4.8, 14.1 Hz, 1H), 4.02 (m, 1H), 7.12 (s, 5H), 7.33 (d, J=7.8 Hz, 2H), 7.54 (d, J=7.8 Hz, 2H), 7.80-7.90 (m, 2H), 8.16 (d, J=0.3 Hz, 1H), 8.76 (d, J=8.4 Hz, 1H), 12.92 (br s, 1H) |
| G-5 | Bn | 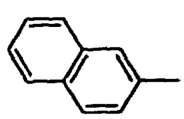 | H | R | 2.74 (dd, J=9.2,13.6Hz, 1H), 2.98 (dd, J=5.2,13.6Hz, 1H), 3.82-4.00 (m, 1H), 7.13-7.28 (m, 5H), 7.55-7.65 (m, 7H), 7.93-8.04 (m, 3H), 8.26 (s, 1H), 8.44 (d, J=9.0Hz, 1H), 12.80 (br, s, 1H) |
| G-6 | (Indol-3-yl)-methyl- | 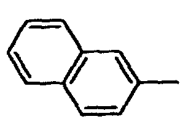 | H | S | 2.89 (dd, J=8.6, 14.2Hz, 1H), 3.09 (dd, J-6.2, 14.2Hz, 1H),3.95 (m, 1H), 6.91-7.14 (m, 3H), 7.30-7.38 (m, 2H), 7.52-7.67 (m, 7H), 7.94-8.06 (m, 3H), 8.25 (s, 1H), 8.37 (m, 1H), 10.82 (s, 1H), 12.71 (br, 1H) |

Table 22

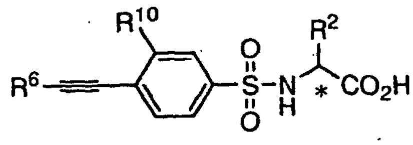

| Example No. | $R^2$ | $R^6$ | $R^{10}$ | * | $^1$H-NMR (DMSO-$d_6$) |
|---|---|---|---|---|---|
| G-7 | MeS-$CH_2CH_2$- | 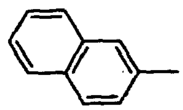 | H | R | 1.72-1.90(m, 2H), 1.96 (s, 3H), 2.28-2.44 (m, 2H), 3.91 ( brs, 1H), 7.60 (dd, J=3.0, 6.0 Hz, 2H), 7.65 (d, J=8.4 Hz, 1H), 7.98 (brs, 3H), 8.25 (s, 1H), 8.33 (d, J=8.1 Hz, 1H), 12.75 (brs, 1H) |
| G-8 | Bn | 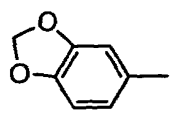 | H | R | 2.73 (dd, J=9.2, 13.8 Hz, 1H), 2.96 (dd, J=6.0, 13.4 Hz, 1H), 3.90 (m, 1H), 6.10 (s, 2H), 7.00 (d, J=8.8 Hz, 1H), 7.05-7.35 (m, 7H), 7.50-7.65 (m, 4H), 8.39 (d, J=9.2 Hz, 1H), 12.75 (br s, 1H) |
| G-9 | Bn | 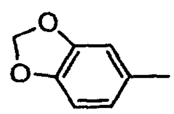 | H | S | 2.73 (dd, J=9.3, 13.8 Hz, 1H), 2.96 (dd, J=5.1, 13.5 Hz, 1H), 3.90 (m, 1H), 6.10 (s, 2H), 7.00 (dd, J=0.3, 7.5 Hz, 1H), 7.10-7.26 (m, 7H), 7.52-7.60 (m, 4H), 8.40 (d, J=9.3 Hz, 1H), 12.77 (br s, 1H) |
| G-10 | Bn | 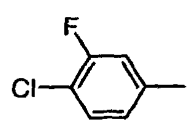 | H | R | 2.73 (dd, J=9.6, 13.8 Hz, 1H), 2.97 (dd, J=5.4, 13.8 Hz, 1H), 3.92 (m, 1H), 7.11-7.26 (m, 5H), 7.49 (dd, J=1.2, 8.1 Hz, 1H), 7.56-7.65 (m, 4H), 7.70 (t, J=8.1 Hz, 1H), 7.74 (dd, J=2.1, 10.2 Hz, 1H), 8.47 (d, J=9.0 Hz, 1H), 12.81 (br s, 1H) |
| G-11 | Bn | 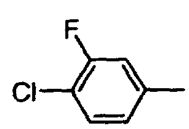 | H | S | 2.73 (dd, J=9.6, 13.8 Hz, 1H), 2.97 (dd, J=5.7, 13.8 Hz, 1H), 3.92 (m, 1H), 7.12-7.26 (m, 5H), 7.49 (dd, J=1.2, 8.4 Hz, 1H), 7.56-7.65 (m, 4H), 7.70 (t, J=8.4 Hz, 1H), 7.74 (dd, J=2.1, 10.2 Hz, 1H), 8.47 (d, J=9.0 Hz, 1H), 12.83 (br s, 1H) |

Table 23

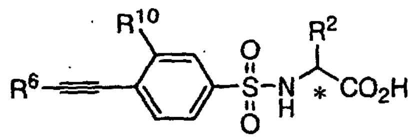

| Example No. | $R^2$ | $R^6$ | $R^{10}$ | * | $^1$H-NMR (DMSO-$d_6$) |
|---|---|---|---|---|---|
| G-12 | $HOCH_2$- | 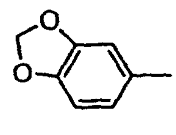 | H | S | 3.49 (dd, J=5.7, 10.8Hz, 1H), 3.54 (dd, J=5.1, 10.8 Hz, 1H), 3.78 (m, 1H), 4.90 (br s, 1H), 6.10 (s, 2H), 7.00 (d, J=7.5 Hz, 1H), 7.11-7.17 (m, 2H), 7.67 (d, J=8.4 Hz, 2H), 7.85 (d, J=8.4 Hz, 2H), 8.16 (d, J=8.7 Hz, 1H) |
| G-13 | Bn | 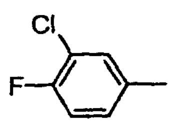 | H | R | 2.73 (dd, J=9.0, 13.5 Hz, 1H), 2.97 (dd, J=5.4, 13.5 Hz, 1H), 3.92 (m, 1H), 7.11-7.25 (m, 5H), 7.52 (t, J=8.7 Hz, 1H), 7.59 (s, 4H), 7.65 (ddd, J=2.1,4.5, 8.7 Hz, 1H), 7.91 (dd, J=1.8, 7.2 Hz, 1H), 8.45 (d, J=9.0 Hz, 1H), 12.80 (br s, 1H) |
| G-14 | Bn | 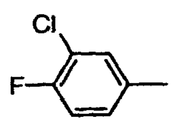 | H | S | 2.73 (dd, J=9.6, 13.8 Hz, 1H), 2.97 (dd, J=5.4, 13.8 Hz, 1H), 3.92 (m, 1H), 7.12-7.26 (m, 5H), 7.52 (t, J=8.7 Hz, 1H), 7.59 (s, 4H), 7.65 (m, 1H), 7.91 (dd, J=1.8, 6.9 Hz, 1H), 8.45 (d, J=9.3 Hz, 1H), 12.81 (br s, 1H) |
| G-15 | iBu | 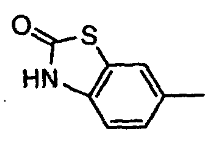 | H | R | 0.72 (d, J=6.3 Hz, 3H), 0.83 (d, J=6.6 Hz, 3H), 1.32-1.48 (m, 2H), 1.58 (m, 1H), 3.68 (m, 1H), 7.17 (d, J=8.1 Hz, 1H), 7.51 (dd, J=1.8, 8.1 Hz, 1H), 7.70 (d, J=8.1 Hz, 2H), 7.79 (d, J=8.1 Hz, 2H), 7.89 (d, J=1.8 Hz, 1H), 8.28 (d, J=9.0 Hz, 1H), 12.16 (s, 1H), 12.60 (br s, 1H) |

Table 24

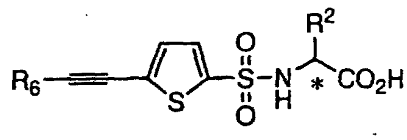

| Example No. | $R^2$ | $R^6$ | * | $^1$H-NMR (DMSO-$d_6$) |
|---|---|---|---|---|
| H-1 | Bn | 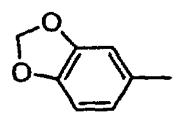 | R | 2.74 (dd, J=9.6, 13.8 Hz, 1H), 3.00 (dd, J=5.1, 13.8 Hz, 1H), 3.96 (m, 1H), 6.11 (s, 2H), 7.00 (d, J=4.8 Hz, 1H), 7.10-7.32 (m, 9H), 8.74 (d, J=8.7 Hz, 1H), 12.91 (br s, 1H) |
| H-2 | Bn | 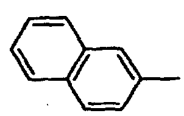 | R | 2.77 (dd, J=9.6, 13.8 Hz, 1H), 3.02 (d, J=5.1, 13.8 Hz, 1H), 3.98 (m, 1H), 7.16-7.27 (m, 5H), 7.28 (d, J=3.9 Hz, 1H), 7.33 (d, J=3.9 Hz, 1H), 7.57-7.66 (m, 3H), 7.95-8.03 (m, 3H), 8.26 (d, J=0.9 Hz, 1H), 8.75 (d, J=8.1 Hz, 1H), 12.91 (br s, 1H) |
| H-3 | Bn | 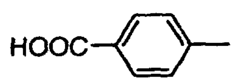 | R | 2.78 (dd, J=9.1, 13.2 Hz, 1H), 3.03 (dd, J=5.4, 13.4 Hz, 1H), 3.96 (m, 1H), 7.00-7.04 (m, 2H), 7.18-7.36 (m, 7H), 7.50-7.56 (m, 2H), 8.70 (br s, 1H) |
| H-4 | (6-Hydroxy-indol-3-yl)methyl | 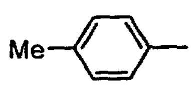 | R | 2.35 (s, 3H), 2.84 (dd, J=8.2, 14.5 Hz, 1H), 3.03 (dd, J=6.1, 14.5 Hz, 1H), 3.98 (m, 1H), 6.50 (dd, J=2.1, 8.5 Hz, 1H), 6.68 (d, J=2.1 Hz, 1H), 6.87 (d, J=2.2 Hz, 1H), 7.15 (d, J=8.5 Hz, 1H), 7.21 (d, J=3.9 Hz, 1H), 7.27 (d, J=3.9 Hz, 1H), 7.28 (d, J=8.1 Hz, 2H), 7.50 (d, J=8.1 Hz, 2H), 8.66 (d, J=7.9 Hz, 1H), 8.86 (s, 1H), 10.44 (d, J=2.2 Hz, 1H), 12.76 (br s, 1H) |
| H-5 | (4-Hydroxy-indol-3-yl)methyl | 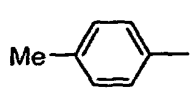 | R | 2.36 (s, 3H), 2.92 (dd, J=9.3, 13.8 Hz, 1H), 3.23 (dd, J=5.7, 13.8 Hz, 1H), 4.28 (brs, 1H), 6.29 (d, J=6.3 Hz, 1H), 6.73-6.83 (m, 2H), 6.87 (d, J=2.1 Hz, 1H), 7.03 (d, J=3.6 Hz, 1H), 7.06 (d, J=3.6 Hz, 1H), 7.28 (d, J=7.8 Hz, 2H), 7.48 (d, J=7.8 Hz, 2H), 8.50 (s, 1H), 9.43 (s, 1H), 10.60 (d, J=2.1 Hz, 1H), 12.54 (brs, 1H) |

Table 25

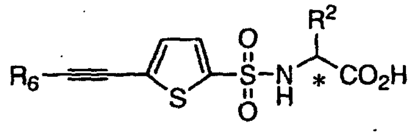

| Example No. | $R^2$ | $R^6$ | * | $^1$H-NMR (DMSO-$d_6$) |
|---|---|---|---|---|
| H-6 | (7-Hydroxy-indol-3-yl)methyl | 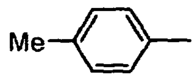 | R | 2.35 (s, 3H), 2.87 (dd, J=7.8, 14.7 Hz, 1H), 3.06 (dd, J=6.3, 14.7 Hz, 1H), 4.00 (m, 1H), 6.48 (d, J=7.5 Hz, 1H), 6.76 (t, J=7.5 Hz, 1H), 6.85 (d, J=7.5 Hz, 1H), 7.00 (d, J=2.4 Hz, 1H), 7.21 (d, J=4.2 Hz, 1H), 7.24-7.28 (m, 3H), 7.49 (d, J=8.1 Hz, 2H), 8.67 (d, J=8.7 Hz, , 1H), 9.44 (s, 1H), 10.64 (s, 1H), 12.75 (brs, 1H) |
| H-7 | Bn | 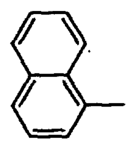 | R | 2.77 (dd, J=9.9,13,8Hz, 1H), 3.03 (dd, J=4.5,13.5Hz, 1H), 3.89 (br, s, 1H), 7.15-7.30 (m,5H), 7.32 (d, J=3.9Hz, 1H), 7.43 (d, J=3.9Hz, 1H), 7.57 (m, 3H), 7.88 (d, J=7.2Hz, 1H), 8.05 (d, J=7.8Hz, 1H), 8.08 (d, J=8.1Hz, 1H), 8,29 (d, J=7.8Hz, 1H), 8.78 (d, J=7.8Hz, 1H), 12.95 (br, s, 1H) |
| H-8 | HOOC-$CH_2CH_2$- | 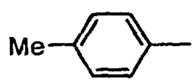 | R | 1.70 (m, 1H), 1.90 (m, 1H), 2.24 (t, J=6.9 Hz, 2H), 2.35 (s, 3H), 3.86 (brs, 1H), 7.27 (d, J=8.1 Hz, 2H), 7.39 (d, J=3.0 Hz, 1H), 7.48-7.50 (m, 3H), 8.64 (brs, 1H), 12.42 (brs, 2H) |
| H-9 | Bn | 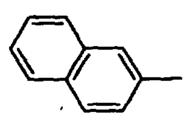 | S | 2.76 (dd, J=9.4, 13.8Hz, 1H), 3.02 (dd, J=4.6, 13.8Hz, 1H), 3.98 (m, 1H), 7.15-7.30 (m, 1H), 7.28 (d, J=3.8Hz, 1H), 7.33 (d, J=3.8Hz, 1H), 7.56-7.67 (m, 3H), 7.94-8.05 (m, 3H), 8.26 (s, 1H), 8.79 (br, 1H), 12.96 (br, 1H) |
| H-10 | iBu | 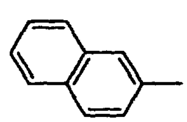 | R | 0.79 (d, J=6.3 Hz, 3H), 0.86 (d, J=6.6 Hz, 3H), 1.37-1.54 (m, 2H), 1.64 (m, 1H), 3.78 (m, 1H), 7.47 (d, J=3.9 Hz, 1H), 7.54 (d, J=3.9 Hz, 1H), 7.57-7.67 (m, 3H), 7.94-8.03 (m, 3H), 8.26 (s, 1H), 8.63 (d, J=9.0 Hz, 1H), 12.79 (br s, 1H) |

Table 26

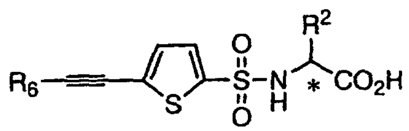

| Example No. | $R^2$ | $R^6$ | * | $^1$H-NMR (DMSO-$d_6$) |
|---|---|---|---|---|
| H-11 | Me | 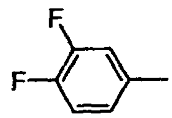 | R | 1.23 (d, J=7.2 Hz, 3H), 3.89 (m, 1H), 7.45 (d, J=3.9 Hz, 1H), 7.46-7.60 (m, 2H), 7.55 (d, J=3.9 Hz, 1H), 7.79 (m, 1H), 8.62 (br s, 1H), 12.79 (br s, 1H) |
| H-12 | Bn | 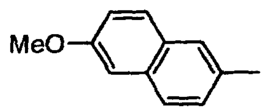 | R | 2.76 (dd, J=9.3, 13.5 Hz, 1H), 3.01 (dd, J=4.8, 13.5 Hz, 1H), 3.90 (s, 3H), 3.98 (m, 1H), 7.16-7.27 (m, 6H), 7.27 (d, J=3.9 Hz, 1H), 7.30 (d, J=3.9 Hz, 1H), 7.40 (d, J=2.4 Hz, 1H), 7.59 (dd, J=1.8, 8.7 Hz, 1H), 7.89 (dd, J=5.1, 8.7 Hz, 2H), 8.17 (s, 1H), 8.77 (d, J=8.4 Hz, 1H), 12.96 (br s, 1H) |
| H-13 | Bn | 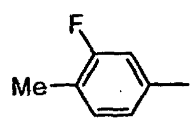 | R | 2.28 (d, J=1.5 Hz, 3H), 2.74 (dd, J=9.6, 13.5 Hz, 1H), 3.00 (dd, J=5.4, 13.5 Hz, 1H), 3.96 (m, 1H), 7.15-7.25 (m, 5H), 7.26 (d, J=3.9 Hz, 1H), 7.29 (d, J=3.9 Hz, 1H), 7.32-7.45 (m, 3H), 8.77 (d, J=8.7 Hz, 1H), 12.93 (br s, 1H) |
| H-14 | Bn | 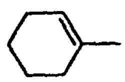 | R | 1.56-1.64 m, 4H), 2.13-2.16 (m, 4H),2.73 (dd, J=9.3, 12.0 Hz, 1H), 2.99 (dd, J=5.4, 13.8 Hz, 1H), 3.93 (m, 1H), 7.10-7.23 (m, 7H), 8.70 (d, J=9.0 Hz, 1H), 12.84 (br s, 1H) |

Table 27

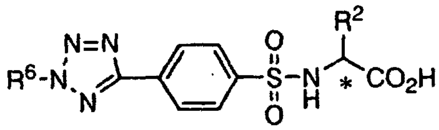

| Example No. | $R^2$ | $R^6$ | * | $^1$H-NMR (DMSO-$d_6$) |
|---|---|---|---|---|
| I-1 | Bn | 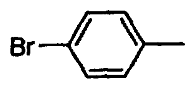 | R | 2.74 (dd, J=9.0, 13.5Hz, 1H), 2.98 (dd, J=5.4, 13.8Hz, 1H), 3.96 (m, 1H), 7.08-7.22 (m, 5H), 7.77 (d, J=8.7Hz, 2H), 7.93 (d, J=8.7Hz, 2H), 8.15 (d, J=9.0Hz, 1H), 8.20 (d, J=8.7Hz, 2H), 8.66 (d, J=9.0Hz, 1H) |
| I-2 | Bn | 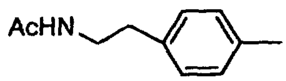 | R | 1.80 (s, 3H), 2.70-3.05 (m, 6H), 3.97 (m, 1H), 7.10-7.30 (m, 5H), 7.54 (d, J=8.6Hz, 2H), 7.77 (d, J=8.6Hz, 2H), 7.88-8.00 (m, 2H), 8.10 (d, J=8.6Hz, 2H), 8.20 (d, J=8.6Hz, 2H), 8.51 (d, J=9.0Hz, 1H), 12.1 (br, 1H) |
| I-3 | Bn | 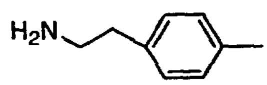 | R | 2.75 (dd, J=9.6, 13.6 Hz, 1H), 2.99 (dd, J=5.4, 13.6 Hz, 1H), 3.00-3.20 (m, 4H), 3.97 (m, 1H), 7.06-7.26 (m, 5H), 7.61 (d, J=8.6 Hz, 2H), 7.77 (d, J=8.6 Hz, 2H), 7.90 (br s, 2H), 8.10-8.26 (m, 4H), 8.51 (d, J=9.0 Hz, 1H) |
| I-4 | Bn | 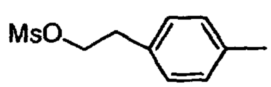 | R | 2.75 (dd, J=9.4, 13.4 Hz, 1H), 2.98 (dd, J=5.6, 13.4 Hz, 1H), 3.10-3.21 (m, 5H), 3.96 (m, 1H), 4.51 (t, J=6.2 Hz, 2H), 7.07-7.27 (m, 5H), 7.64 (d, J=8.4 Hz, 2H), 7.77 (d, J=8.4 Hz, 2H), 8.15 (d, J=8.4 Hz, 2H), 8.20 (d, J=8.4 Hz, 2H), 8.50 (d, J=8.6 Hz, 1H) |
| I-5 | Bn | 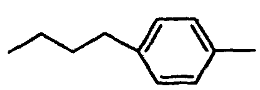 | R | 0.93 (t, J=7.5 Hz, 3H), 1.27-1.42 (m, 2H), 1.56-1.69 (m, 2H), 2.67-2.80 (m, 3H), 2.98 (dd, J=5.4, 13.8 Hz, 1H), 3.96 (m, 1H), 7.09-7.22 (m, 5H), 7.53 (d, J=8.7 Hz, 2H), 7.76 (d, J=8.7 Hz, 2H), 8.08 (d, J=8.4 Hz, 2H), 8.19 (d, J=8.7 Hz, 2H), 8.51 (d, J=9.0 Hz, 1H), 12.80 (br s, 1H) |

Table 28

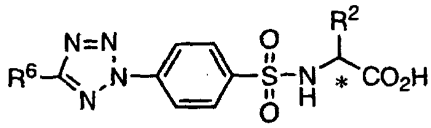

| Example No. | $R^2$ | $R^6$ | * | $^1$H-NMR (DMSO-$d_6$) |
|---|---|---|---|---|
| J-1 | Bn | 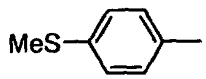 | R | 2.57 (s, 3H), 2.75 (dd, J=9.6, 13.8 Hz, 1H), 3.00 (dd, J=4.8, 13.8 Hz, 1H), 3.97 (m, 1H), 7.10-7.22 (m, 5H), 7.50 (d, J=8.7 Hz, 2H), 7.83 (d, J=8.7 Hz, 2H), 8.12 (d, J=8.7 Hz, 1H), 8.19 (d, J=8.7 Hz, 2H), 8.58 (d, J=9.0 Hz, 1H), 12.82 (br s, 1H) |
| J-2 | Bn | 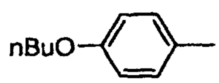 | R | 0.96 (t, J=7.2 Hz, 3H), 1.40-1.54 (m, 2H), 1.69-1.81 (m, 2H), 2.75 (dd, J=9.9, 13.8 Hz, 1H), 2.99 (dd, J=5.1, 13.8 Hz, 1H), 3.97 (m, 1H), 7.08-7.22 (m, 7H), 7.79-7.86 (m, 2H), 8.09-8.15 (m, 2H), 8.15-8.21 (m, 2H), 8.57 (d, J=8.4 Hz, 1H), 12.83 (br s, 1H) |
| J-3 | Bn | 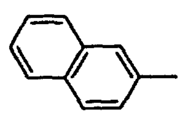 | S | 2.76 (dd, J=9.3,13.5Hz, 1H), 3.00 (dd, J=5.4,13.8Hz, 1H), 3.98 (br, s, 1H), 7.10-7.21 (m,5H), 7.63-7.69 (m, 2H), 7.86 (d, J=9.0Hz, 2H), 8.04-8.07 (m, 1H), 8.16-8.31 (m, 5H), 8.56 (s, 1H), 8.85 (s,1H), 12.68-13.15 (br, s, 1H) |
| J-4 | Bn | 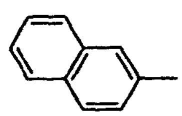 | R | 2.76 (dd, J=9.6,13.5Hz, 1H), 3.00 (dd, J=4.8,13.5Hz, 1H), 3.97 (br, s, 1H), 7.05-7.21 (m, 5H), 7.64-7.67 (m, 2H), 7.86 (d, 8.7Hz, 2H), 8.04-8.07 (m, 1H), 8.17-8.31 (m, 5H), 8.59 (d, J=6.6Hz, 1H), 8.85 (s, 1H), 12.95 (br, s, 1H) |

Table 29

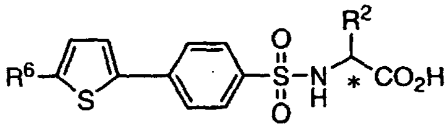

| Example No. | $R^2$ | $R^6$ | * | $^1$H-NMR (DMSO-$d_6$) |
|---|---|---|---|---|
| K-1 | iPr | MeS | R | 0.80 (d, J=6.9 Hz, 3H), 0.83 (d, J=6.9 Hz, 3H), 1.94 (m, 1H), 2.57 (s, 3H), 3.53 (dd, J=5.4, 9.0 Hz, 1H), 7.17 (d, J=3.9 Hz, 1H), 7.59 (d, J=3.9 Hz, 1H), 7.77 (s, 4H), 8.07 (d, J=9.0 Hz, 1H), 12.60 (br s, 1H) |
| K-2 | iPr | 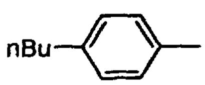 | R | 0.83 (t, J=6.6 Hz, 6H), 0.91 (t, J=6.8 Hz, 3H), 1.26-1.42 (m, 2H), 1.47-1.67 (m, 2H), 1.95 (m, 1H), 2.61 (t, J=7.4Hz, 2H), 3.54 (m, 1H), 7.27 (d, J=8.0 Hz, 2H), 7.55 (d, J=4.0 Hz, 1H), 7.63 (d, J=8.0 Hz, 2H), 7.70 (d, J=4.0 Hz, 1H), 7.75-7.90 (m, 4H), 8.05 (d, J=9.0 Hz, 1H), 12.60 (br s, 1H) |
| K-3 | Me | 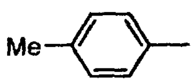 | R | 1.18 (d, J=6.9 Hz, 3H), 2.34 (s, 3H), 3.80 (m, 1H), 7.27 (d, J=8.7 Hz, 2H), 7.55 (d, J=3.9 Hz, 1H), 7.62 (d ,J=8.1 Hz, 2H), 7.70 (d, J=3.9 Hz, 1H), 7.80 (d, J=8.1 Hz, 2H), 7.87 (d, J=8.7 Hz, 2H), 8.17 (d, J=7.8 Hz, 1H), 12.64 (br s, 1H) |
| K-4 | MeS-$CH_2CH_2$- | 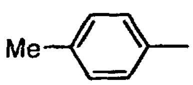 | R | 1.78-1.90 (m, 2H), 1.94 (s, 3H), 2.34 (s, 3H), 2.24-2.50 (m, 2H), 3.85 (m, 1H), 7.26 (d, J=8.1 Hz, 2H), 7.54 (d, J=3.9 Hz, 1H), 7.61 (d, J=8.1 Hz, 2H), 7.70 (d, J=3.9 Hz, 1H), 7.79 (d, J=8.4 Hz, 2H), 7.87 (d, J=8.4 Hz, 2H), 8.21 (d, J=8.7 Hz, 1H), 12.75 (br s, 1H) |
| K-5 | iPr | 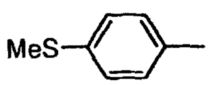 | R | 0.79-0.86 (m, 6H), 1.94 (m, 1H), 3.34 (s, 3H), 3.54 (m, 1H), 7.33 (d, J=8.7 Hz, 2H), 7.58 (d, J=3.6 Hz, 1H), 7.67 (d, J=8.7 Hz, 2H), 7.71 (d, J=3.9 Hz, 2H), 7.79(d, J=8.7 Hz, 2H), 7.86 (d, J=8.7 Hz, 2H), 8.07 (d, J=9.6 Hz, 1H), 12.60 (br s, 1H) |

Table 30

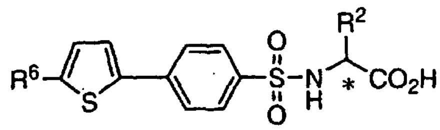

| Example No. | $R^2$ | $R^6$ | * | $^1$H-NMR (DMSO-$d_6$) |
|---|---|---|---|---|
| K-6 | iBu | 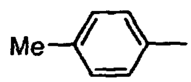 | R | 0.73 (d, J=6.6 Hz, 3H), 0.82 (d, J=6.6 Hz, 3H), 1.34-1.66 (m, 5H), 2.34 (s, 3H), 3.65 (m, 1H), 7.26 (d, J=8.1 Hz, 2H), 7.55 (d, J=3.9 Hz, 1H), 7.61 (d, J=8.1 Hz, 2H), 7.70 (d, J=3.9 Hz, 1H), 7.78 (d, J=8.6 Hz, 2H), 7.86 (d, J=8.6 Hz, 2H), 8.16 (d, J=8.4 Hz, 1H), 12.59 (br.s, 1H) |

Table 31

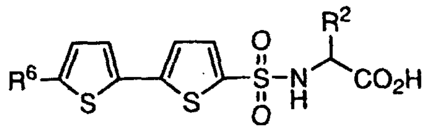

| Example No. | $R^2$ | $R^6$ | * | $^1$H-NMR (DMSO-$d_6$) |
|---|---|---|---|---|
| L-1 | iPr | 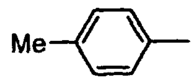 | R | 0.83 (d, J=6.9 Hz, 3H), 0.88 (d, J=6.9 Hz, 3H), 2.00 (m, 1H), 2.33 (s, 3H), 3.61 (s, 1H), 7.26 (d, J=8.4 Hz, 2H), 7.33 (d, J=3.9 Hz, 1H), 7.47-7.51 (m, 3H), 7.59 (d, J=8.1 Hz, 2H), 8.39 (s, 1H), 12.76 (br s, 1H) |
| L-2 | iPr | 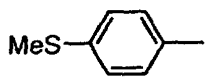 | R | 0.83 (d, J=6.6 Hz, 3H), 0.88 (d, J=6.6 Hz, 3H), 2.00 (m, 1H), 2.51 (s, 3H), 3.61 (s, 1H), 7.31-7.35 (m, 3H), 7.49 (d, J=3.6 Hz, 2H), 7.54 (d, J=3.9 Hz, 1H), 7.64 (d, J=8.4 Hz, 2H), 8.37 (d, J=7.8 Hz, 1H), 12.80 (br s, 1H) |
| L-3 | Bn | 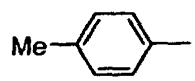 | R | 2.34 (s, 3H), 2.76 (dd, J=9.9, 14.1 Hz, 1H), 3.00 (dd, J=5.4, 14.1 Hz, 1H), 3.96 (m, 1H), 7.10-7.30 (m, 7H), 7.41 (d, J=3.6 Hz, 1H), 7.50 (d, J=3.6 Hz, 1H), 7.59 (d, J=8.1 Hz, 2H), 8.62 (d, J=9.0 Hz, 1H), 12.87 (br s, 1H) |
| L-4 | H | 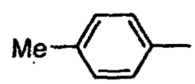 | - | 2.33 (s, 3H), 3.70 (s, 2H), 7.26 (d, J=8.4 Hz, 2H), 7.35 (d, J=3.9 Hz, 1H), 7.48 (d, J=3.9 Hz, 1H), 7.50 (d, J=3.9 Hz, 1H), 7.54 (d, J=3.9 Hz, 1H), 7.59 (d, J=8.4 Hz, 2H), 8.36 (br s, 1H), 12.76 (br s, 1H) |
| L-5 | iPr | 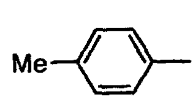 | S | 0.83 (d, J=6.9 Hz, 3H), 0.88 (d, J=6.9 Hz, 3H), 1.99 (m, 1H), 2.33 (s, 3H), 3.61 (s, 1H), 7.26 (d, J=8.1 Hz, 2H), 7.33 (d, J=3.9 Hz, 1H), 7.46-7.53 (m, 3H), 7.59 (d, J=8.4 Hz, 2H), 8.35 (s, 1H), 12.8 (br s, 1H) |

Table 32

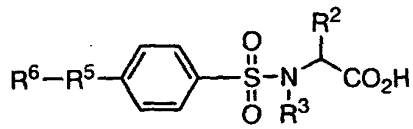

| Example No. | $R^2$ | $R^3$ | $R^6$-$R^5$- | * | $^1$H-NMR (DMSO-$d_6$) |
|---|---|---|---|---|---|
| M-1 | Me | Me | 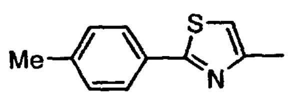 | R | 1.20 (d, J=7.2 Hz, 3H), 2.39 (s, 3H), 2.80 (s, 3H), 4.61 (q, J=7.2 Hz, 1H), 7.37 (d, J=8.1 Hz, 2H), 7.89 (d, J=8.1 Hz, 2H), 7.95 (d, J=8.1 Hz, 2H), 8.26 (d, J=8.1 Hz, 2H), 8.39 (s, 1H), 12.84 (br s, 1H) |
| M-2 | Me | H | 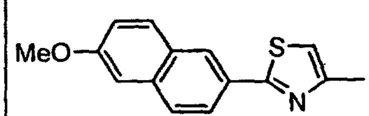 | R | 1.19 (d, J=7.5Hz, 3H), 3.84 (m, 1H), 3.92 (s, 3H), 7.26 (dd, J=2.4, 9.0Hz, 1H), 7.42 (d, J=2.4Hz, 1H), 7.90 (d, J=8.4Hz, 2H), 7.97 (d, J=8.7Hz, 1H), 8.04 (d, J=9.0Hz, 1H), 8.14 (dd, J=2.1, 8.7Hz, 1H), 8.20 (d, J=8.7Hz, 1H), 8.28 (d, J=8.4Hz, 2H), 8.55 (s, 1H), 12.65 (br s, 1H) |
| M-3 | Me | H | 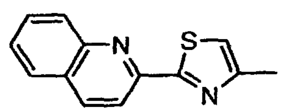 | R | 1.20 (d, J=6.9Hz, 3H), 3.84 (m, 1H), 7.70(m, 1H), 7.86 (m, 1H), 7.92 (d, J=8.7Hz, 2H), 8.06-8.13 (m, 2H), 8.22 (d, J=7.8Hz, 1H), 8.30 (d, J=8.7Hz, 2H), 8.46 (d, J=8.7Hz, 1H), 8.57 (s, 1H), 8.61 (d, J=8.7Hz, 1H), 12.64 (s, 1H) |
| M-5 | MeS-$CH_2CH_2$- | H | 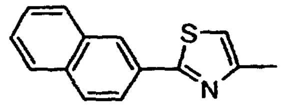 | R | 1.66-1.95 (m, 2H), 1.95 (s, 3H), 2.28-2.50 (m, 2H), 3.93 (m, 1H), 7.59-7.66 (m, 2H), 7.90 (d, J=8.1 Hz, 2H), 8.01 (m, 1H), 8.07-8.17 (m, 4H), 8.21 (d, J=8.7 Hz, 2H), 8.25-8.34 (m, 3H), 8.47 (d, J=0.9 Hz, 1H), 8.65 (s, 1H), 12.79 (br s, 1H) |

Table 33

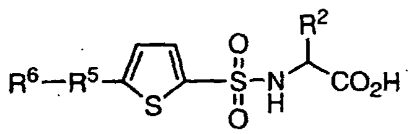

| Example No. | $R^2$ | $R^6$-$R^5$- | * | $^1$H-NMR (DMSO-$d_6$) |
|---|---|---|---|---|
| N-1 | Bn | 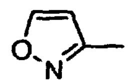 | R | 2.72 (dd, J=10.2, 13.6 Hz, 1H), 3.01 (dd, J=5.0, 13.6 Hz, 1H), 3.99 (m, 1H), 4.65-4.85 (m, 2H), 7.10-7.25 (m, 5H), 7.34 (d, J=4.0 Hz, 1H), 7.64 (m, 1H), 8.94 (d, J=8.0 Hz, 1H), 12.75 (br s, 1H) |
| N-2 | iPr | 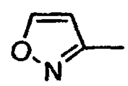 | R | 0.81 (d, J=6.6 Hz, 3H), 0.86 (d, J=7.0 Hz, 3H), 2.01 (m, 1H), 3.66 (m, 1H), 4.65-4.90 (m, 2H), 7.65 (d, J=3.4 Hz, 1H), 7.93 (m, 1H), 8.68 (d, J=9.0 Hz, 1H), 12.78 (br s, 1H) |
| N-3 | Bn | 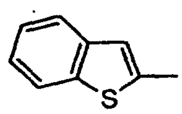 | R | 2.77 (dd, J=9.9, 13.2 Hz, 1H), 3.02 (dd, J=5.1, 13.2 Hz, 1H), 3.98 (m, 1H), 7.08-7.24 (m, 5H), 7.27-7.33 (m, 2H), 7.38-7.47(m, 2H), 7.78 (s, 1H), 7.89 (m, 1H), 8.01 (m, 1H), 8.71 (d, J=7.5 Hz, 1H), 12.93 (br s, 1H) |
| N-4 | Bn | 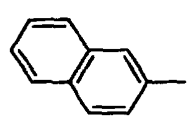 | R | 2.79 (dd, J=9.6, 13.8 Hz, 1H), 3.01 (dd, J=5.4, 13.5 Hz, 1H), 4.00 (m, 1H), 7.07-7.25 (m, 5H), 7.34 (d, J=3.9 Hz, 1H), 7.53-7.62 (m, 2H), 7.58 (d, J=3.9 Hz, 1H), 7.82 (d, J=9.0 Hz, 1H), 7.96(d, J=9.0 Hz, 1H), 8.01 (d, J=8.4 Hz, 2H), 8.23 (s, 1H), 8.62 (d, J=8.4 Hz, 1H), 12.93 (br s, 1H) |

Table 34

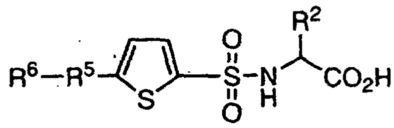

| Example No. | $R^2$ | $R^6$-$R^5$- | * | $^1$H-NMR (DMSO-$d_6$) |
|---|---|---|---|---|
| N-5 | (Indol-3-yl)methyl | 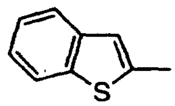 | R | 2.93 (dd, J=9.0, 15.0Hz,1H), 3.14 (dd, J=5.7, 14.1Hz, 1H), 4.1 (m, 1H), 6.88-7.03 (m, 2H), 7.13(d, J=2.4Hz, 1H), 7.16-7.30 (m, 3H), 7.35-7.48 (m, 3H), 7.60 (m, 1H), 7.71 (s, 1H), 7.89 (m, 1H), 7.99 (m, 1H), 8.62 (m, 1H), 10.82 (s, 1H), 12.60 (br, 1H) |
| N-6 | Bn | 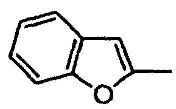 | S | 2.76 (dd, J=9.6, 13.5 Hz, 1H), 3.01 (dd, J=4.9, 14.1 Hz, 1H), 3.98 (m, 1H), 7.12-7.18 (m, 5H), 7.28-7.41 (m, 3H), 7.45 (s, 1H), 7.51 (d, J=3.7 Hz, 1H), 7.68(t, J=9.5 Hz, 2H), 8.73 (d, J=8.5 Hz, 1H) |
| N-7 | Bn | 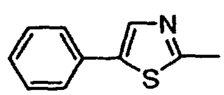 | R | 2.76 (dd, J=9.9, 13.7 Hz, 1H), 3.01 (dd, J=4.7, 13.5 Hz, 1H), 4.00 (m, 1H), 7.13-7.18 (m, 5H), 7.31 (d, J=3.8 Hz, 1H), 7.39-7.55 (m, 4H), 7.74 (d, J=7.1 Hz, 2H), 8.33 (s, 1H), 8.76 (d, J=6.9 Hz, 1H) |
| N-8 | Bn | 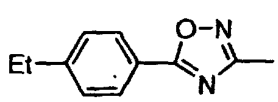 | R | 1.24 (t, J=7.8 Hz, 3H), 2.71-2.81 (m, 3H), 3.03 (dd, J=4.8, 13.5 Hz, 1H), 4.02 (br s, 1H), 7.08-7.22 (m, 5H), 7.40 (d, J=4.2 Hz, 1H), 7.53 (d, J=8.4 Hz, 2H), 7.71 (d, J=3.6 Hz, 1H), 8.10 (d, J=8.1 Hz, 2H), 8.85 (d, J=8.1 Hz, 1H), 8.01 (br s, 1H), 12.91 (br s, 1H) |

Table 35

| Example No. | $R^6$-$R^5$-$R^4$-$SO_2NH$-W | $^1H$-NMR (DMSO-$d_6$) |
|---|---|---|
| O-1 | 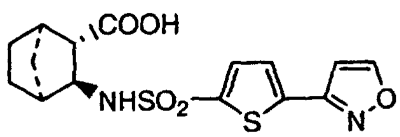 | 1.12-1.64 (m, 6H),2.00 (d, J=3.9 Hz, 1H), 2.18 (s, 1H), 2.27 (s, 1H), 3.60 (m, 1H), 3.78 (m, 1H), 4.75 (s, 1H), 7.64 (s, 1H), 7.92 (s, 1H), 8.45 (s, 1H), 12.13 (br s, 1H) |
| O-2 | 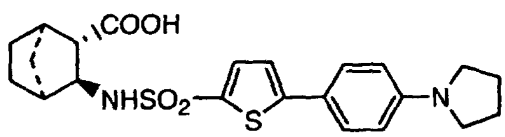 | 1.10-1.70 (m, 6H), 1.93-2.05 (m, 5H), 2.19 (m, 1H), 2.26 (m, 1H), 3.74 (t, J=6.3 Hz, 4H), 3.81 (m, 1H), 6.58 (d, J=8.7 Hz, 2H), 7.24 (d, J=3.9 Hz, 1H), 7.42 (d, J=3.9 Hz, 1H), 7.50 (d, J=8.7 Hz, 2H), 8.00 (d, J=5.7 Hz, 1H), 12.13 (br s, 1H) |
| O-3 | 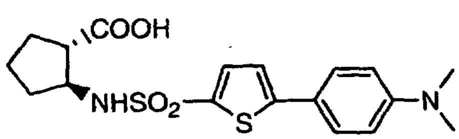 | 1.34-2.00 (m, 6H), 2.57 (m, 1H), 2.96 (s, 6H), 3.84 (m, 1H), 6.76 (d, J=6.0Hz, 2H), 7.29 (d, J=4.2Hz, 1H), 7.45 (d, J=4.2Hz, 1H), 7.52 (d, J=6.0Hz, 2H), 7.95 (m, 1H), 12.0 m, 1H) |
| O-4 | 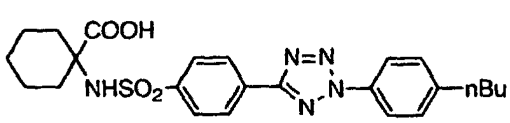 | 0.93 (t, J=7.5 Hz, 3H), 1.22-1.41 (m, 8H), 1.56-1.90 (m, 6H), 2.71 (t, J=7.5 Hz, 2H), 7.53 (d, J=8.4 Hz, 2H), 8.01 (d, J=8.4 Hz, 2H), 8.04-8.11 (m, 3H), 8.34 (d, J=8.4 Hz, 2H), 12.45 (br s, 1H) |
| O-5 | 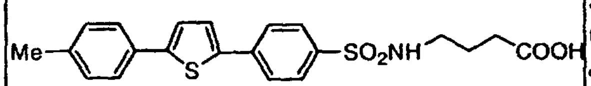 | 1.60 (m, 1H), 2.21 (t, J=7.5 Hz, 1H), 2.34 (s, 3H), 2.78 (brs, 1H), 7.27 (d, J=8.1 Hz, 2H), 7.55 (d, J=3.6 Hz, 1H), 7.62 (d, J=8.1 Hz, 2H), 7.70 (d, J=3.6 Hz, 1H), 7.80 (d, J=8.4 Hz, 2H), 7.89 (d, J=8.4 Hz, 2H) |

Table 36

| Example No. | $R^6$-$R^5$-$R^4$-$SO_2NH$-W | $^1$H-NMR (DMSO-$d_6$) |
|---|---|---|
| O-6 | 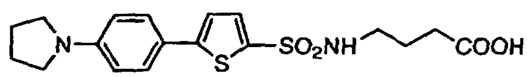 | 1.64 (brs, 2H), 1.97 (brs, 4H), 2.24 (m, 4H), 2.88 (brs, 4H), 6.58 (d, J=8.7 Hz, 2H), 7.28 (d, J=3.6 Hz, 1H), 7.46 (d, J=3.6 Hz, 1H), 7.51 (d, J=8.7 Hz, 2H), 7.76 (brs, 1H), 11.98 (brs, 1H) |
| O-7 | 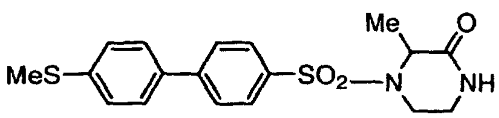 | 1.35 (d, J=6.9 Hz, 3H), 2.53 (s, 3H), 2.94-3.14 (m, 2H), 3.40 (m, 1H), 3.71 (m, 1H), 4.18 (q, J=7.5 Hz, 1H), 7.38 (d, J=8.4 Hz, 2H), 7.72 (d, J=8.4 Hz, 2H), 7.87-7.96 (m, 4H) |
| O-8 | 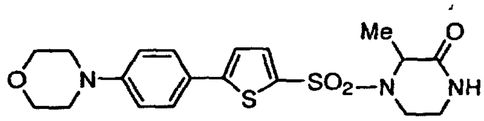 | 1.40 (d, J=7.2 Hz, 3H), 3.06-3.13 (m, 2H), 3.15-3.23 (m, 4H), 3.46 (m, 1H), 3.60-3.80 (m, 5H), 4.14 (q, J=6.9 Hz, 1H), 7.00 (d, J=8.7 Hz, 2H), 7.46 (d, J=3.9 Hz, 1H), 7.60 (d, J=8.7 Hz, 2H), 7.69 (d, J=4.2 Hz, 1H), 7.99 (m, 1H) |
| O-9 | 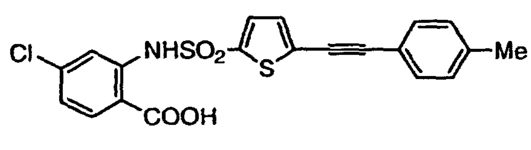 | 2.34 (s, 3H), 6.99 8dd, J=2.1, 8.7Hz, 1H), 7.25 (d, J=8.1Hz, 2H), 7.32 (d, J=3.9Hz, 1H), 7.42-7.50 (m, 4H), 7.60 (m, 1H), 7.66 (d, J=8.1Hz, 2H), 12.0 (br, 1H) |
| O-10 | 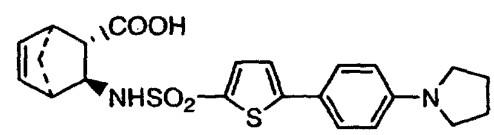 | 1.26 (dd, J=2.1, 9.0 Hz, 1H), 1.49 (d, J=9.0 Hz, 1H), 1.90-2.04 (m, 5H), 2.87 (br s, 2H), 3.28 (t, J=6.3 Hz, 4H), 4.02 (m, 1H), 6.30 (dd, J=3.0, 5.4 Hz, 1H), 6.35 (dd, J=3.3, 5.4 Hz, 1H), 6.59 (d, J=8.7 Hz, 2H), 7.28 (d, J=4.2 Hz, 1H), 7.45 (d, J=3.9 Hz, 1H), 7.51(d, J=8.7 Hz, 2H), 7.68 (d, J=6.0 Hz, 1H) |

Test example

The following tests were done in regard of the present invention compound.

Example 1. Assay method of aggrecanase inhibition activity

(1) Cartilage culture test

Cartilage disks (2mm in diameter) were prepared from the cartilage of juvenile rabbits (3 weeks). Cartilage disks were transferred and cultured individually in the wells of a 48-well tissue culture plate containing DMEM/10% FCS for over 3 days at 37°C. In the night before the test, the medium of DMEM was changed to that not containing FCS. Next morning, IL-1 (4ng/ml) and a test compound were added to the plate. It is provided that some wells of a plate contain only IL-1 (not containing test compound) and that some wells are vehicle (not containing IL-1 and test compound). After 4 days from the IL-1 addition, the amount of released glycosaminoglycan (GAG) was measured after collecting culture supernatant and cartilage disks.

Quantitation of GAG release was as follows; the weight of the cartilage disk was measured, then 200μl of a papain digestion buffer solution (1mM EDTA, 2mM DTT, 20mM Tris, (pH 7.0), Papain (300μg/ml)) was added to each well and the plate containing the digestive solution was incubated for 2 h at 65°C. 50μl of 100mM Iodoacetatic acid (pH 7.0) was added for stopping the digestion. The extracts of cartilage are centrifuged at 12000rpm for 5min. 20 μl of centrifuged supernatant or culture supernatant was transferred in the wells of a 96-well assay plate. The amount of released GAG in the transferred solution was quantitated by using a proteoglycan & glycosaminoglycan assay kit (Blyscan, Funakoshi Co., Ltd, Tokyo, Japan).

The following tables 37 and 38 show the above test data of sulfonamide derivatives.

(2) Aggrecanase inhibitory activity test

To 50μl of the above 4 days cultured supernatant (not including IL-1 and a test compound, including IL-1 only, including both IL-1 and a test compound) was added a 5 volume solution of 95% EtOH containing 1.3% (w/v) potassium acetate. After the mixed solution was stood at -20°C for 30min, the solution was centrifuged to obtain a precipitation. The precipitation was solubilized in 100μl of 25mM Tris-hydrochloric acid buffer solution (pH7.6) containing 5mM calcium chloride·0.15M sodium chloride·0.0025% Brij35. After addition of 5μl of 250mM ethylenediaminetetraacetic acid, and 2.5μl each of 1M sodium acetate (pH6.0) and 1M Tris-hydrochloric acid (pH7.9) to the solution, 2.5μl of 0.02 units/μl Chondroitinase ABC and 1.0 μl of 0.1 units/μl Keratanase (Both are Seikagaku Kogyo, Japan) were added and the mixture was incubated at 37°C for 16 h to remove GAG. A 5 volume solution of 95%EtOH containing 1.3% (w/v) potassium acetate was added again and the mixture was stirred, cooled, and centrifuged like conducted above to obtain a precipitation. The precipitation was solubilized in 20μl of NuPAGE LDS Sample Buffer (Invitrogen) containing 5% 2-mercaptoehtanol and boiled for 5min. The resultant was used as a sample for western blot analysis. The Western blot analysis was carried out in an ordinary procedure. SDS-PAGE electrophoresis used 3-8% Tris-acetate gradient gel of Invitrogen and following electrophoresis resolved proteins were transferred to PVDF membrane. Immunodetection was performed by utilizing an anti ARGSV antibody as a primary antibody and a horseradish peroxidase-conjugated anti mouse IgG antibody (Amersham) as a secondary antibody. A color identification test was carried out by utilizing ECLplus (Amersham). An operation after the detection using the secondary antibody was performed according to a protocol attached to ECLplus detection kit. The anti ARGSV antibody that reacts to the terminus ARGSV newly-formed by cleavage of the aggrecan by aggrecanase was able to be prepared, for example, by the Sandy's method (J. D. Sandy. et al. J.Biol.Chem.270 (1995) 2550-2556).

Figure 1 shows the results of a test compound (XXI), wherein the GAG value is 87. In the culture supernatant containing only IL-1, the 250kDa aggrecanase-generated fragment of aggrecan which was recognized by anti N-terminus neoepitope antibody was observed, which was not detected in the supernatant not containing IL-1 and a test compound (XXI). On the other hand, in the culture supernatant containing both IL-1 and a test compound (XXI), the 250kDa aggrecanase-generated fragment was not detected. It is considered that test compound inhibits an aggrecanase activity. Figure 2 shows western blot analysis of other test compounds as an example.

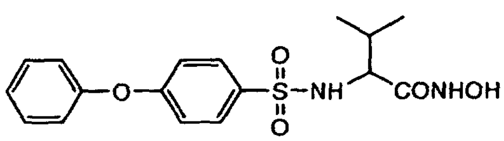

(XXI)

Test example 2 MMP-13

(1) Isolation and Purification

In regard to MMP-13, mRNA was prepared from carcinoma cell SW1353 derived from human cartilage stimulate by IL-1, TNF and catalytic domain ($^{104}$Tyr∼$^{267}$Gly) was amplified with RT-PCR. This was cloned in Escherichia. coli expression vector pTrc99AHE inserted with His-tag sequence and enterokinase digestion-site, induced and expressed by IPTG (Isopropyl-β-D-thiogalactopyranoside) and expressed in a insoluble fraction. Isolation of MMP-13 from an insoluble fraction was carried out by dissolving in modifier (6M urea) by a usual method and purification with metal chelate chromatography (Ni Chelateing Sepharose). And then removing modifier (6M urea) with dialyze and refolding of the enzyme spontaneously gave activated MMP-13.

(2) Assay for inhibitory activities on MMP-13

The enzymatic activity on MMP was analyzed by the method described in "C. Graham Knight, Frances Willenbrock and Gillian Murphy: A novel coumarin-labelled peptide for sensitive continuous assays of the matrix metalloproteinases: FEBS LETT., 296, (1992), 263-266". The substrate : MOCAc-Pro-Leu-Gly-Leu-$A_2$Pr(DNP)-Ala-Arg-$NH_2$ was purchased from Peptide Institute, Inc., Osaka, Japan. The measurement of the inhibitory activities ($IC_{50}$) was carried out by the following four methods;

(A) Reaction with substrate, enzyme (MMPs) and inhibitor
(B) Reaction with substrate and inhibitor, without enzyme
(C) Reaction with substrate and enzyme (MMPs), without inhibitor
(D) Reaction with substrate only

$IC_{50}$ values were calculated by using the following formula and each fluorescence values of above four methods (A to D).

$$\% \text{ inhibition} = \{1-(A-B)/(C-D)\} \times 100$$

$IC_{50}$ means the concentration required to inhibit 50% of the enzyme activity.

This test gave the data shown in Table 37 to Table 38 in regard of the following sulfonamide.

Table 37

| Compound No. | GAG | MMP-13 ($IC_{50}\mu M$) | Compound No. | GAG | MMP-13 ($IC_{50}\mu M$) |
|---|---|---|---|---|---|
| A-1 | 50.8 | 0.0120 | D-4 | 27.9 | 0.167 |
| A-2 | 47.5 | 0.0013 | D-5 | 28.0 | 1.01 |
| A-3 | 80.1 | 0.0023 | D-6 | 43.1 | 0.764 |
| A-4 | >100 | 0.0024 | E-1 | 59.1 | 0.400 |
| A-5 | >100 | 0.0020 | E-2 | 78.8 | 0.00877 |
| A-6 | 58.3 | 0.0029 | E-3 | 40.8 | 0.083 |
| A-7 | 50.5 | 0.0111 | E-4 | 83.8 | 0.0209 |
| A-8 | 46.7 | 0.0038 | E-5 | 94.3 | 0.0022 |
| A-9 | 54.9 | 0.0065 | E-6 | 55.3 | 0.0026 |
| A-10 | >100 | 0.0246 | E-7 | 68.3 | 0.0197 |
| A-11 | 53.0 | 0.0114 | E-8 | 74.7 | 0.0105 |
| A-12 | 49.0 | 0.012 | E-9 | 64.2 | 0.0132 |
| A-13 | 40.1 | 0.019 | E-10 | 42.9 | 0.0236 |
| A-14 | 67.4 | 0.0061 | E-11 | >100 | 0.0091 |
| A-15 | >100 | 0.0135 | E-12 | >100 | 0.0165 |

Table 37 (continued)

| Compound No. | GAG | MMP-13 ($IC_{50}\mu M$) | Compound No. | GAG | MMP-13 ($IC_{50}\mu M$) |
|---|---|---|---|---|---|
| A-16 | 86.2 | 0.0154 | E-13 | >100 | 0.0254 |
| A-17 | >100 | 0.0065 | E-14 | >100 | 0.0135 |
| A-18 | >100 | 0.0052 | E-15 | >100 | 0.0195 |
| A-19 | 41.0 | 0.0092 | E-16 | >100 | 0.0142 |
| A-20 | 93.2 | 0.0183 | E-17 | 93.4 | 0.0067 |
| A-24 | 27.4 | 0.0482 | E-18 | 70.4 | 0.0204 |
| A-25 | 53.3 | 0.00033 | E-19 | >100 | 0.0122 |
| A-26 | 26.3 | 0.00045 | E-20 | >100 | 0.0141 |
| A-27 | 39.7 | 0.001 | E-21 | 69.9 | 0.0020 |
| B-1 | >100 | 0.0199 | E-22 | 35.1 | 0.0139 |
| B-2 | 14.4 | 0.763 | E-23 | 22.1 | 1.06 |
| B-3 | 59.9 | 0.0443 | E-24 | 40.5 | 0.024 |
| B-5 | 37.4 | 0.0169 | E-25 | 24.6 | 0.39 |
| C-1 | 65.3 | 0.010 | E-26 | 31.0 | 0.21 |
| C-2 | 48.5 | 0.027 | E-27 | 55.7 | 0.036 |
| C-3 | >100 | 0.0113 | E-28 | 22.6 | 0.012 |
| C-4 | 71.7 | 0.0057 | E-29 | 23.9 | 0.00992 |
| C-5 | >100 | 0.0183 | E-30 | 71.9 | 0.0026 |
| C-6 | >100 | 0.0180 | F-1 | 47.1 | 0.018 |
| C-7 | 69.4 | 0.0196 | F-2 | 15.0 | 0.530 |
| C-8 | 42.0 | 0.049 | F-3 | 45.3 | 0.007 |
| C-9 | 55.8 | >10 | F-4 | 75.7 | 0.0094 |
| C-10 | 28.5 | 0.0174 | F-5 | >100 | 0.0183 |
| D-1 | 61.7 | 0.028 | F-6 | 41.9 | 0.0376 |
| D-2 | 21.8 | 0.141 | F-7 | 64.8 | 0.043 |
| D-3 | 13.0 | 0.1 | F-9 | 17.1 | 0.0036 |

Table 38

| Compound No.. | GAG | MMP-13 ($IC_{50}\mu M$) | Compound No. | GAG | MMP-13 ($IC_{50}\mu M$) |
|---|---|---|---|---|---|
| F-10 | 13.6 | 0.21 | H-13 | 55.7 | 0.0604 |
| F-11 | 28.0 | 0.00047 | H-14 | 52.2 | 0.153 |
| F-12 | 64.2 | 0.0318 | I-1 | 52.0 | 0.036 |
| F-13 | 18.5 | 0.033 | I-2 | 37.0 | 0.01 |
| F-14 | 10.8 | 0.0104 | I-3 | 61.8 | 0.029 |
| F-15 | 32.2 | 0.023 | I-4 | 41.6 | 0.0082 |
| F-16 | 15.8 | 0.25 | I-5 | 30.6 | 0.0179 |
| F-17 | 64.8 | 0.0037 | J-1 | 26.1 | 0.0485 |
| F-18 | 67.0 | 0.032 | J-3 | 15.1 | >10 |

Table 38 (continued)

| Compound No.. | GAG | MMP-13 ($IC_{50}\mu M$) | Compound No. | GAG | MMP-13 ($IC_{50}\mu M$) |
|---|---|---|---|---|---|
| F-19 | 48.3 | 0.63 | J-6 | 12.4 | 0.454 |
| F-20 | 41.3 | 0.811 | K-1 | 56.9 | 0.0202 |
| F-21 | 31.1 | 1.26 | K-2 | 25.2 | 0.0116 |
| F-22 | 11.6 | 0.854 | K-3 | 32.6 | 0.095 |
| F-24 | 40.1 | 0.0831 | K-4 | 17.8 | 0.0938 |
| F-25 | 48.1 | 0.106 | K-5 | 63.1 | 0.00526 |
| F-26 | 44.9 | 0.0101 | K-6 | 48.0 | 0.225 |
| G-1 | 50.2 | 0.046 | L-1 | 50.4 | 0.031 |
| G-2 | 50.2 | 0.015 | L-2 | 91.0 | 0.0147 |
| G-3 | 10.1 | >10 | L-3 | 22.9 | 0.109 |
| G-4 | 28.6 | >10 | L-4 | 10.8 | 0.281 |
| G-5 | 39.3 | >10 | L-5 | 34.0 | 0.134 |
| G-6 | 43.0 | 0.893 | M-2 | 11.3 | >10 |
| G-9 | 75.0 | 0.0366 | M-4 | 33.4 | 8.94 |
| G-10 | 10.2 | 0.0751 | N-1 | 47.6 | 1.1 |
| G-11 | 30.3 | 0.12 | N-3 | 16.2 | 0.00771 |
| G-12 | 37.0 | 0.147 | N-4 | 27.9 | 0.07 |
| G-13 | 28.5 | 0.831 | N-5 | 31.0 | 0.0319 |
| G-14 | 30.9 | 1.07 | N-6 | 47.7 | 0.498 |
| G-15 | 37.1 | 1.09 | N-7 | 29.8 | 0.114 |
| H-1 | 44.5 | 0.044 | N-8 | 43.0 | 0.182 |
| H-2 | 14.1 | >10 | O-1 | 36.7 | >10 |
| H-3 | 44.7 | 6 | O-2 | 56.0 | 0.34 |
| H-5 | 68.2 | 0.0328 | O-3 | 47.2 | 0.353 |
| H-6 | 86.4 | 0.0113 | O-4 | 42.7 | 0.0114 |
| H-7 | 73.2 | >10 | O-5 | 16.7 | >10 |
| H-8 | 48.3 | 0.151 | O-6 | 19.5 | 3.14 |
| H-9 | 14.3 | >10 | O-7 | 15.8 | 0.883 |
| H-10 | >100 | 1.37 | O-8 | 13.6 | 2.99 |
| H-11 | 21.6 | 0.573 | O-9 | 73.3 | >10 |
| H-12 | 16.7 | 0.15 | O-10 | 80.3 | 0.6 |

Test example 3 The evaluation of the inhibitory activities of the compounds on the disease progression in the guinea-pig osteoarthritis model.

Guinea pig osteoarthritis model was prepared according to the procedure reported by Meacock et. al. (J. Exp. Path. 71: 279-293, 1990), by medial meniscectomy and collateral ligament transection on the right knee of 12 weeks old female Hartley guinea pigs (Charles River Japan). 0.5% methylcellulose or compounds (30 mg/kg) were orally administered once daily from the following day of the surgery for 10 days.

On the next day of final administration, femoral chondyles and tibial plateaus of right knees were removed. The surfaces of the tibial plateau were stained with India ink (Kuretake), then photographed using a digital camera

(Nikon). Whole area and stained area of the medial plateaus were measured by using a computer-aided image analyzing system (WinRoof, MITANI CORPORATION.).

Test example 3 shows that the present invention compounds are effective on the treatment of osteoarthritis. In a similar way, oral administration to 12 weeks old female NZW rabbits (Kitayama Labes) or 12 weeks old female SD rats (Clea Japan) for 6 weeks also shows that the present invention compounds are effective on the treatment of osteoarthritis.

Formulation example

Formulation example 1

| Granules are prepared using the following ingredients. | | |
|---|---|---|
| Ingredients | The compound represented by the formula (I)<br>Lactose<br>Corn starch<br>HPC-L | 10 mg<br>700 mg<br>274 mg<br>16 mg<br>1000 mg |

The compound represented by the formula (I) and lactose are made pass through a 60 mesh sieve. Corn starch is made pass through a 120 mesh sieve. They are mixed by a twin shell blender. An aqueous solution of HPC-L (low mucosity hydroxypropylcellulose) is added to the mixture and the resulting mixture is kneaded, granulated (by the extrusion with pore size 0.5 to 1 mm mesh), and dried. The dried granules thus obtained are sieved by a swing sieve (12/60 mesh) to yield the granules.

Formulation example 2

| Powders for filling capsules are prepared using the following ingredients. | | |
|---|---|---|
| Ingredients | The compound represented by the formula (I)<br>Lactose<br>Corn starch<br>Magnesium stearate | 10 mg<br>79 mg<br>10 mg<br>1 mg |
| | | 100 mg |

The compound represented by the formula (I) and lactose are made pass through a 60 mesh sieve. Corn starch is made pass through a 120 mesh sieve. These ingredients and magnesium stearate are mixed by a twin shell blender. 100 mg of the 10-fold trituration is filled into a No. 5 hard gelatin capsule.

Formulation example 3

| Granules for filling capsules are prepared using the following ingredients. | | |
|---|---|---|
| Ingredients | The compound represented by the formula (I)<br>Lactose<br>Corn starch<br>HPC-L | 15 mg<br>90 mg<br>42 mg<br>3 mg |
| | | 150 mg |

The compound represented by the formula (I) and lactose are made pass through a 60 mesh sieve. Corn

starch is made pass through a 120 mesh sieve. After mixing them, an aqueous solution of HPC-L is added to the mixture and the resulting mixture is kneaded, granulated, and dried. After the dried granules are lubricated, 150 mg of that are filled into a No. 4 hard gelatin capsule.

Formulation example 4

| Tablets are prepared using the following ingredients. | | |
|---|---|---|
| Ingredients | The compound represented by the formula (I)<br>Lactose<br>Microcrystal cellulose<br>CMC-Na<br>Magnesium stearate | 10 mg<br>90 mg<br>30 mg<br>15 mg<br>5 mg |
| | | 150 mg |

The compound represented by the formula (I), lactose, microcrystal cellulose, and CMC-Na (carboxymethylcellulose sodium salt) are made pass through a 60 mesh sieve and then mixed. The resulting mixture is mixed with magnesium stearate to obtain the mixed powder for the tablet formulation. The mixed powder is compressed to yield tablets of 150 mg.

**Industrial Applicability**

The sulfonamide derivatives of the present invention have activities of inhibiting aggrecanase and are useful as a composition for treating osteoarthrosis or rheumatoid arthritis.

## Claims

1. A pharmaceutical composition inhibiting aggrecanase which contains as an active ingredient a compound of the formula (I):

$$R^6—R^5—R^4—SO_2—W \qquad (I)$$

wherein W group is represented by the formula:

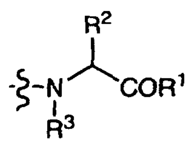 , 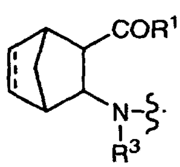 , 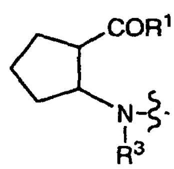 , 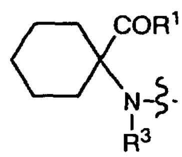 ,

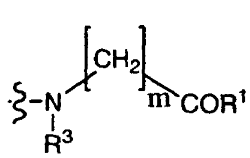 , 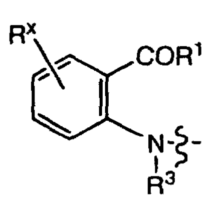 , or 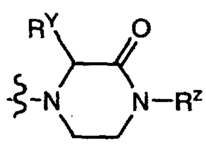

wherein $R^1$ is NHOH, hydroxy, or lower alkyloxy;

$R^2$ is hydrogen atom, optionally substituted lower alkyl, optionally substituted aryl, optionally substituted aralkyl, optionally substituted heteroaryl, or optionally substituted heteroarylalkyl;
$R^3$ is hydrogen atom, optionally substituted lower alkyl, optionally substituted aryl, optionally substituted aralkyl, optionally substituted heteroaryl, or optionally substituted heteroarylalkyl;
$R^x$ is hydrogen atom, lower alkyl, or halogen;
$R^Y$ is hydrogen atom or lower alkyl;
$R^Z$ is hydrogen atom or lower alkyl;
m is an integer from 2 to 6;
a broken line (---) represents the presence or absence of a bond;
$R^4$ is optionally substituted arylene, or optionally substituted heteroarylene;
$R^5$ is a bond, $-(CH_2)_p-$, -CH=CH-, -C≡C-, -CO-, -CO-NH-, -N=N-, $-N(R^A)-$, -NH-CO-NH-, -NH-CO-, -S-, $-SO_2-$, $-SO_2NH-$, $-SO_2-NH-N=CH-$ or a group represented by the formula:

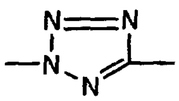 , 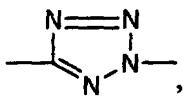 , 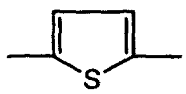 , 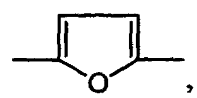 , 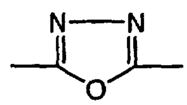

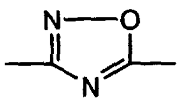 , 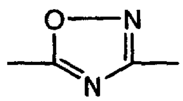 , 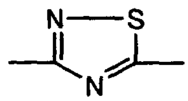 , 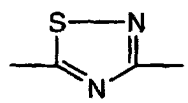 , 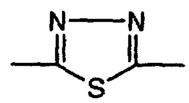

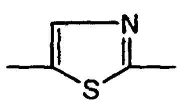 , 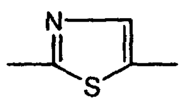 , 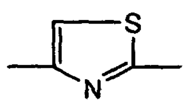 , 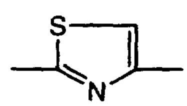

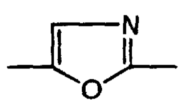 , 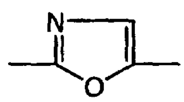 , 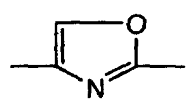 , 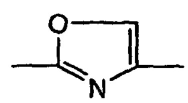 ,

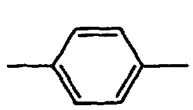 , or 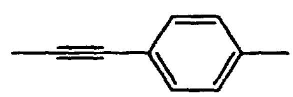

$R^A$ is hydrogen atom or lower alkyl;
p is 1 or 2;
$R^6$ is optionally substituted lower alkyl, optionally substituted cycloalkyl, optionally substituted aryl, optionally substituted aralkyl, optionally substituted heteroaryl, optionally substituted heteroarylalkyl, optionally substituted non-aromatic heterocyclic group; or optionally substituted cycloalkenyl,
its optically active substance, its prodrug, their pharmaceutically acceptable salt, or solvate thereof.

**2.** A pharmaceutical composition inhibiting aggrecanase which contains as an active ingredient a compound as described in claim 1, wherein W group is represented by the formula:

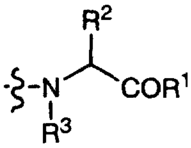 , 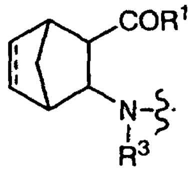 , 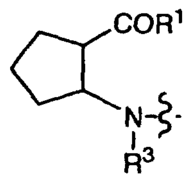 , or 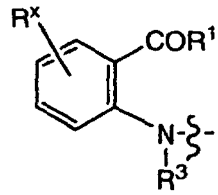

wherein $R^1$, $R^2$, $R^3$ and $R^X$ are as defined in claim 1;
its optically active substance, its prodrug, their pharmaceutically acceptable salt, or solvate thereof.

**3.** A pharmaceutical composition inhibiting aggrecanase which contains as an active ingredient a compound as described in claim 2, wherein W group is represented by the formula:

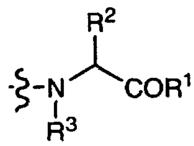

wherein $R^1$, $R^2$, and $R^3$ are as defined in claim 1;
its optically active substance, its prodrug, their pharmaceutically acceptable salt, or solvate thereof.

**4.** A pharmaceutical composition inhibiting aggrecanase which contains as an active ingredient a compound, as described in claim 3, wherein $R^2$ is hydrogen atom, optionally substituted lower alkyl wherein the substituent is hydroxy, carboxy, carbamoyl, or lower alkylthio, aryl optionally substituted with hydroxy, optionally substituted aralkyl wherein the substituent is hydroxy or phenyl, heteroaryl, or optionally substituted heteroarylalkyl wherein the substituent is hydroxy or halogen, its optically active substance, its prodrug, their pharmaceutically acceptable salt, or solvate thereof.

**5.** A pharmaceutical composition inhibiting aggrecanase which contains as an active ingredient a compound as described in claim 4, wherein $R^2$ is hydrogen atom, methyl, isopropyl, isobutyl, sec-butyl, carbamoylmethyl, carbamoylethyl, 2-methylthioethyl, hydroxymethyl, carboxymethyl, carboxyethyl, phenyl, 4-hydroxyphenyl, benzyl, 4-hydroxybenzyl, 4-biphenylmethyl, thienyl, indol-3-ylmethyl, (4-hydroxy-indol-3-yl)methyl, (5-hydroxy-indol-3-yl)methyl, (6-hydroxy-indol-3-yl)methyl, (7-hydroxy-indol-3-yl)methyl or (5-fluoro-indol-3-yl)methyl,
its optically active substance, its prodrug, their pharmaceutically acceptable salt, or solvate thereof.

**6.** A pharmaceutical composition inhibiting aggrecanase which contains as an active ingredient a compound, its optically active substance, its prodrug, their pharmaceutically acceptable salt, or solvate thereof as described in any one of claims 1 to 5, wherein $R^1$ is hydroxy.

**7.** A pharmaceutical composition inhibiting aggrecanase which contains as an active ingredient a compound, its optically active substance, its prodrug, their pharmaceutically acceptable salt, or solvate thereof as described in any one of claims 1 to 6, wherein $R^3$ is hydrogen atom.

**8.** A pharmaceutical composition inhibiting aggrecanase which contains as an active ingredient a compound, its optically active substance, its prodrug, their pharmaceutically acceptable salt, or solvate thereof as described in any one of claims 1 to 7, wherein $R^4$ is 1,4-phenylene or 2,5-thiophen-diyl.

**9.** A pharmaceutical composition inhibiting aggrecanase which contains as an active ingredient a compound, its optically active substance, its prodrug, their pharmaceutically acceptable salt, or solvate thereof as described in any one of claims 1 to 8, wherein $R^5$ is a bond, -C≡C-, or a group represented by the formula:

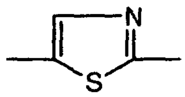, 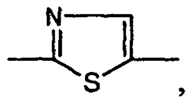, 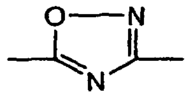, 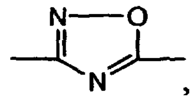, 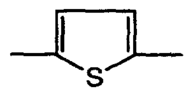

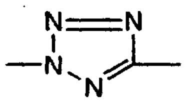, 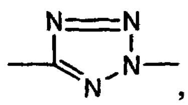, 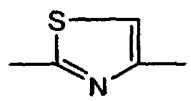, 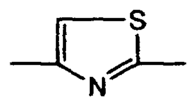, or 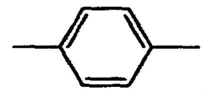.

**10.** A pharmaceutical composition inhibiting aggrecanase which contains as an active ingredient a compound as described in claim 2, wherein W is a group represented by the formula:

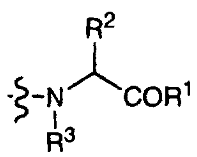, 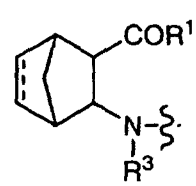, 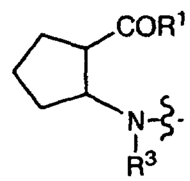, or 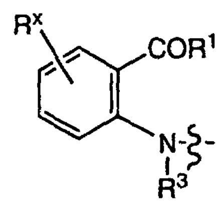

wherein $R^1$ is hydroxy;

$R^2$ is hydrogen atom, methyl, isopropyl, isobutyl, sec-butyl, carbamoylmethyl, carbamoylethyl, 2-methylthioethyl, hydroxymethyl, carboxymethyl, carboxyethyl, phenyl, 4-hydroxyphenyl, benzyl, 4-hydroxybenzyl, 4-biphenylmethyl, thienyl, indol-3-ylmethyl, (4-hydroxy-indol-3-yl)methyl, (5-hydroxy-indol-3-yl)methyl, (6-hydroxy-indol-3-yl)methyl, (7-hydroxy-indol-3-yl)methyl, or (5-fluoro-indol-3-yl)methyl;
$R^3$ is hydrogen atom;
$R^x$ is hydrogen atom, lower alkyl, or halogen;
a broken line (---) represents the presence or absence of a bond;
$R^4$ is 1,4-phenylene or 2,5-thiophen-diyl;
$R^5$ is a bond, -C≡C-, or a group represented by the formula:

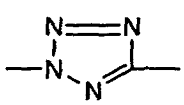, 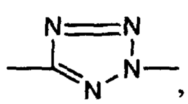, 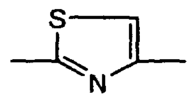, 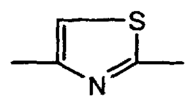, or 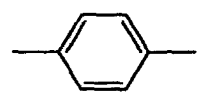

$R^6$ is optionally substituted phenyl, naphtyl, isoxazole, pyrrole, pyrrolidine or cyclohexenyl,
its optically active substance, its prodrug, their pharmaceutically acceptable salt, or solvate thereof.

**11.** A pharmaceutical composition inhibiting aggrecanase which contains as an active ingredient a compound as described in claim 10,
wherein $R^4$ is 2,5-thiophen-diyl;

$R^5$ is a bond or -C≡C-;
$R^6$ is optionally substituted phenyl, naphtyl, or cyclohexenyl,
its optically active substance, its prodrug, their pharmaceutically acceptable salt, or solvate thereof.

**12.** A pharmaceutical composition inhibiting aggrecanase which contains as an active ingredient a compound as described in claim 3,

$R^1$ is hydroxy;
$R^2$ is hydrogen atom, methyl, isopropyl, isobutyl, sec-butyl, carbamoylmethyl, carbamoylethyl, 2-methylthioethyl, hydroxymethyl, carboxymethyl, carboxyethyl, phenyl, 4-hydroxyphenyl, benzyl, 4-hydroxybenzyl, 4-biphenylmethyl, thienyl, indol-3-ylmethyl, (4-hydroxy-indol-3-yl)methyl, (5-hydroxy-indol-3-yl)methyl, (6-hydroxy-indol-3-yl)methyl, (7-hydroxy-indol-3-yl)methyl, or (5-fluoro-indol-3-yl)methyl;
$R^3$ is hydrogen atom;
$R^4$ is 1,4-phenylene or 2,5-thiophen-diyl;
$R^5$ is a bond, -C≡C-, or a group represented by the formula:

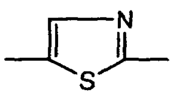 , 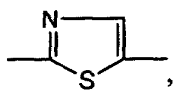 , 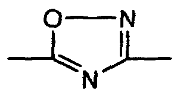 , 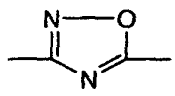 , or 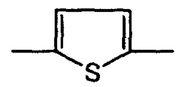

$R^6$ is optionally substituted phenyl, optionally substituted naphtyl, or optionally substituted heteroaryl,
its optically active substance, its prodrug, their pharmaceutically acceptable salt, or solvate thereof.

**13.** A pharmaceutical composition inhibiting aggrecanase which contains as an active ingredient a compound as described in claim 12, wherein

$R^5$ is -C≡C-, or a group represented by the formula:

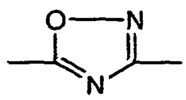 or 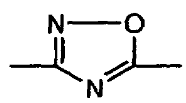

$R^6$ is naphtyl or optionally substituted phenyl,
its optically active substance, its prodrug, their pharmaceutically acceptable salt, or solvate thereof.

**14.** A pharmaceutical composition inhibiting aggrecanase as claimed in any one of claims 1 to 13, wherein the composition has an activity of inhibiting matrix metalloproteinase-13.

**15.** A composition for treating or preventing osteoarthritis as claimed in any one of claims 1 to 14.

**16.** A composition for treating or preventing rheumatoid arthritis as claimed in any one of claims 1 to 14.

**17.** Use of a compound as described in any one of claims 1 to 13 for the preparation of a composition for treating osteoarthrosis.

**18.** Use of a compound as described in any one of claims 1 to 13 for the preparation of a composition for treating rheumatoid arthritis.

**19.** A method of treatment of a mammal, including a human, to alleviate the pathological effects of osteoarthrosis, which comprises administering to said mammal a therapeutically effective amount of a compound as described in any one of claims 1 to 13.

**20.** A method of treatment of a mammal, including a human, to alleviate the pathological effects of rheumatoid arthritis, which comprises administering to said mammal a therapeutically effective amount of a compound as described in any one of claims 1 to 13.

# Figure 1

## Example of Aggrecanase inhibitory activity with anti-neoepitope antibody

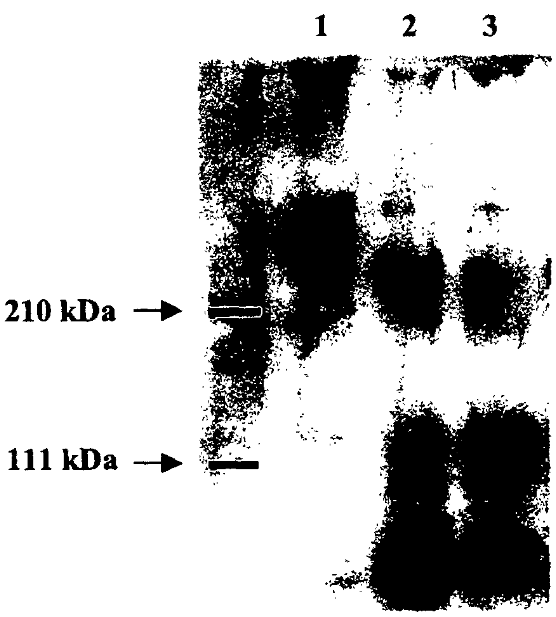


lane 1 : Vehicle (not containing IL-1, compound XXI)
lane 2 : Addition of IL-1only
lane 3 : Addition of IL-1 and compound XXI

**Figure 2**

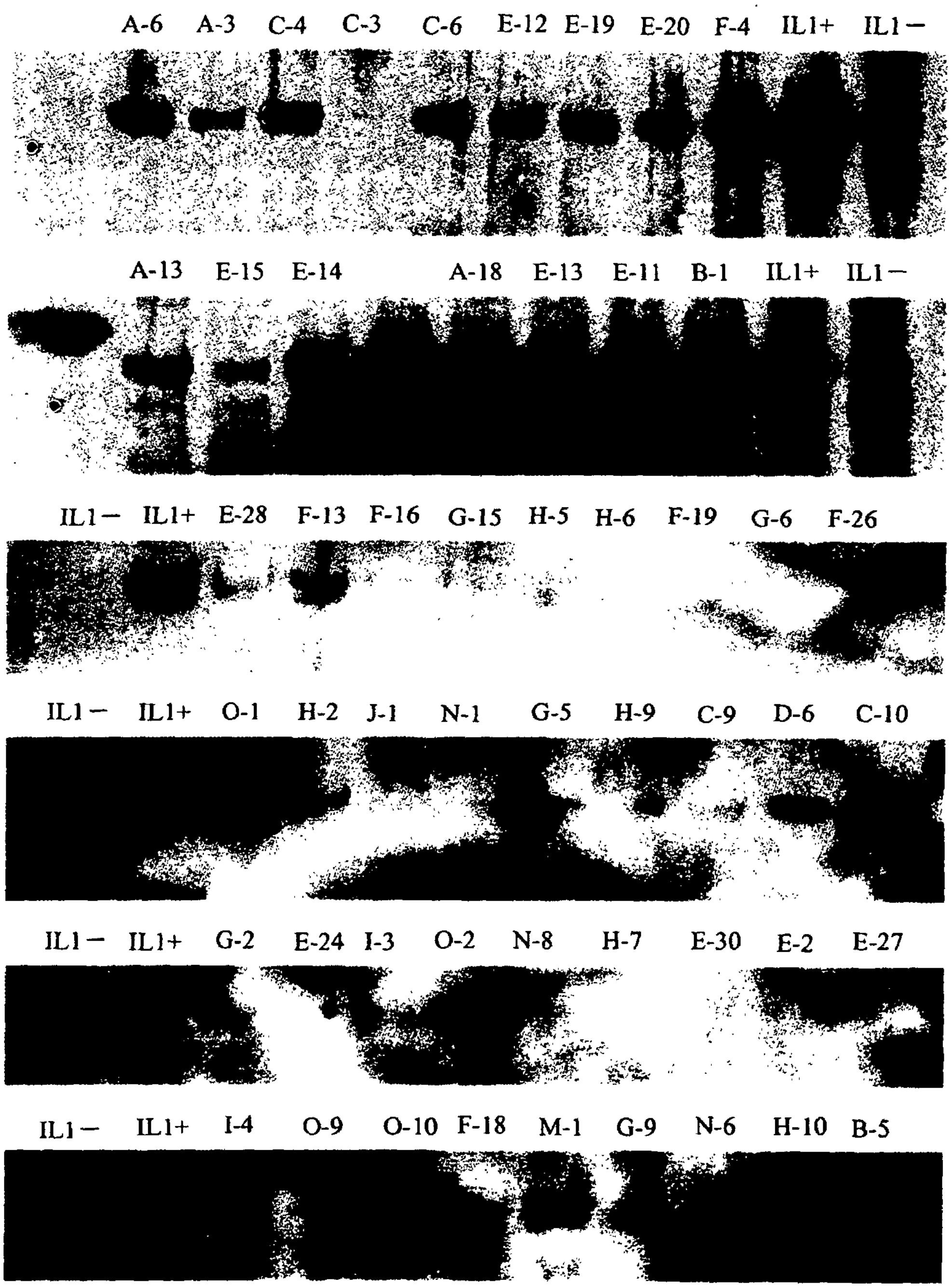
A-6 A-3 C-4 C-3 C-6 E-12 E-19 E-20 F-4 IL1+ IL1−
A-13 E-15 E-14 A-18 E-13 E-11 B-1 IL1+ IL1−
IL1− IL1+ E-28 F-13 F-16 G-15 H-5 H-6 F-19 G-6 F-26
IL1− IL1+ O-1 H-2 J-1 N-1 G-5 H-9 C-9 D-6 C-10
IL1− IL1+ G-2 E-24 I-3 O-2 N-8 H-7 E-30 E-2 E-27
IL1− IL1+ I-4 O-9 O-10 F-18 M-1 G-9 N-6 H-10 B-5

## INTERNATIONAL SEARCH REPORT

International application No.
PCT/JP03/03673

A. CLASSIFICATION OF SUBJECT MATTER
Int.Cl[7] A61K31/198, 31/381, 31/4025, 31/405, 31/421, 31/4245, 31/426, 31/427, A61P19/02, 29/00, 43/00 // C07D209/20, 263/32, 271/06, 277/26, 333/24, 333/34, 409/12, 413/04, 417/04
According to International Patent Classification (IPC) or to both national classification and IPC

B. FIELDS SEARCHED
Minimum documentation searched (classification system followed by classification symbols)
Int.Cl[7] A61K31/198, 31/381, 31/4025, 31/405, 31/421, 31/4245, 31/426, 31/427, A61P19/02, 29/00, 43/00 C07D209/20, 263/32, 271/06, 277/26, 333/24, 333/34, 409/12, 413/04, 417/04

Documentation searched other than minimum documentation to the extent that such documents are included in the fields searched

Electronic data base consulted during the international search (name of data base and, where practicable, search terms used)
CAPLUS(STN), REGISTRY(STN), MEDLINE(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| X | WO 01/27084 A1 (PROCTER & GAMBLE CO.), 19 April, 2001 (19.04.01), Page 1; examples & BR 2000014759 A & EP 1224171 A1 & NO 2002001748 A | 1-18 |
| X | WO 00/51975 A1 (PROCTER & GAMBLE CO.), 08 September, 2000 (08.09.00), Page 1; examples & US 6197770 B1 & NZ 513831 A & EP 1165501 A1 & BR 2000008716 A & JP 2002-538136 A & NO 2001004242 A | 1-18 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 May, 2003 (23.05.03) | 10 June, 2003 (10.06.03) |
| Name and mailing address of the ISA/ Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP03/03673

C (Continuation). DOCUMENTS CONSIDERED TO BE RELEVANT

| Category* | Citation of document, with indication, where appropriate, of the relevant passages | Relevant to claim No. |
|---|---|---|
| X | WO 00/51993 A2 (PROCTER & GAMBLE CO.), 08 September, 2000 (08.09.00), Page 1; examples<br>& NZ 513830 A & EP 1165530 A2<br>& BR 2000009244 A & JP 2002-538146 A<br>& NO 2001004243 A | 1-18 |
| X<br><br>Y | EP 1069110 A1 (SANKYO CO., LTD.), 17 January, 2001 (17.01.01), Page 1; examples<br>& WO 99/51572 A1 & CA 2327290 A<br>& AU 9929615 A & JP 2000-319250 A<br>& BR 9909398 A & NO 2000004949 A | 1-3,6-10, 14-18<br>4,5,11-13 |
| X<br><br>Y | WO 96/00214 A1 (CIBA-GEIGY CORP.), 04 January, 1996 (04.01.96), Page 12; formula I<br>& US 5455258 A & US 5552419 A<br>& US 5646167 A & CA 2192092 A<br>& US 5506242 A & AU 9525369 A<br>& AU 692553 B & EP 766672 A1<br>& EP 766672 B1 & HU 76548 A<br>& JP 11-505502 A & NZ 285846 A<br>& ES 2151599 T3 & IL 114171 A<br>& US 5672615 A & FI 9605156 A<br>& NO 9605568 A & US 5817822 A | 1-4,8-10, 14-18<br>5-7,11-13 |
| X<br><br>Y | EP 935963 A2 (PFIZER PRODUCTS INC.), 18 August, 1999 (18.08.99), Page 2; example 2<br>& EP 935963 A3 & CA 2251197 A<br>& AU 9889481 A & NZ 332478 A<br>& JP 11-199512 A | 1-5,8,9, 14-18<br>6,7,10-13 |
| X<br><br>Y | JP 2001-163786 A (SANKYO CO., LTD.), 19 June, 2001 (19.06.01), Pages 7 to 8; formula I<br>(Family: none) | 1-3,6-10,12, 14-18<br>4,5,11,13 |
| X<br><br>Y | JP 2001-163885 A (SANKYO CO., LTD.), 19 June, 2001 (19.06.01), Pages 5 to 6; formula I<br>& WO 01/23363 A1 | 1-3,6-10,12, 14-18<br>4,5,11,13 |
| X<br><br>Y | WO 99/42443 A1 (NOVARTIS A.-G.), 26 August, 1999 (26.08.99), Page 21; examples<br>& CA 2318145 A & AU 9929235 A<br>& AU 747911 B & EP 1053226 A1<br>& BR 9909322 A & JP 2002-503720 A<br>& NZ 505968 A & NO 2000003565 A<br>& US 6410580 B1 | 1-3,6-9,11, 14-18<br>4,5,10,12,13 |

Form PCT/ISA/210 (continuation of second sheet) (July 1998)

**INTERNATIONAL SEARCH REPORT**

International application No.
PCT/JP03/03673

**Box I Observations where certain claims were found unsearchable (Continuation of item 2 of first sheet)**

This international search report has not been established in respect of certain claims under Article 17(2)(a) for the following reasons:

1. [X] Claims Nos.: 19, 20
because they relate to subject matter not required to be searched by this Authority, namely:
Claims 19 and 20 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)